# EUROPEAN PATENT SPECIFICATION

(11) **EP 2 125 744 B1**
(45) Date of publication and mention of the grant of the patent: **13.04.2011**
(21) Application number: 07856889.6
(22) Date of filing: 19.12.2007
(51) Int. Cl.: C07D 217/22, C07D 217/24, C07D 401/12, A61K 31/472, A61P 9/00, A61P 11/06

(54) **CYCLOALKYLAMINE SUBSTITUTED ISOQUINOLONE AND ISOQUINOLINONE DERIVATIVES**
CYCLOALKYLAMINSUBSTITUIERTE ISOCHINOLON- UND ISOCHINOLINONDERIVATE
DÉRIVÉS D'ISOQUINOLONE ET D'ISOQUINOLINONE SUBSTITUÉS PAR UNE CYCLOALKYLAMINE

(30) Priority: 27.12.2006 EP 06026895
(43) Date of publication of application: 02.12.2009
(73) Proprietor: Sanofi-Aventis, 75013 Paris (FR)
(72) Inventor: PLETTENBURG, Oliver, 65926 Frankfurt am Main (DE); HOFMEISTER, Armin, 65926 Frankfurt am Main (DE); GOERLITZER, Jochen, 65926 Frankfurt am Main (DE); LÖHN, Matthias, 65926 Frankfurt am Main (DE)
(74) Representative: Löschner, Thomas
(86) International application number: PCT/EP2007/011166
(87) International publication number: WO 2008/077553

(56) References cited:
- WO-A-2005/030791
- WO-A-2005/054202
- WO-A-2007/000240
- YOSHIDA Y ET AL: "Studies on anti-helicobacter pylori agents. Part 1: Benzyloxyisoquinoline derivatives" BIOORGANIC & MEDICINAL CHEMISTRY, ELSEVIER SCIENCE LTD, GB, vol. 7, no. 11, 1999, pages 2647-2666, XP002314443 ISSN: 0968-0896

## Description

The present invention relates to novel isoquinolone and isoquinolinone derivatives as described in the claims, their preparation and their use in the treatment and/or prevention of diseases related to the inhibition of Rho-kinase and/or of Rho-kinase mediated phosphorylation of myosin light chain phosphatase.

Activation of a small GTPase RhoA upon agonist stimulation results in conversion of RhoA from the inactive GDP-bound form to the active GTP-bound form with a subsequent binding to and activation of Rho-kinase. Two isoforms, Rho-kinase 1 and Rho-kinase 2, are known. Rho-kinase 2 is expressed in vascular smooth muscle cells and endothelial cells. Activation of Rho-kinase 2 by the active GTP-bound RhoA leads to calcium sensitization of smooth muscle cells through phosphorylation-mediated inhibition of the myosin light chain phosphatase activity and thereby up-regulation of the activity of myosin regulatory light chain (Uehata et al., Nature 1997, 389, 990-994).

It is known that Rho-kinase is involved in vasoconstriction, including the development of myogenic tone and smooth muscle hypercontractility (Gokina et al. J. Appl. Physiol. 2005, 98, 1940-8), bronchial smooth muscle contraction (Yoshii et al. Am. J. Resp. Cell Mol. Biol. 20, 1190-1200), asthma (Setoguchi et al. Br J Pharmacol. 2001, 132,111-8; Nakahara, et al. Eur J 2000,389,103) and chronic obstructive pulmonary disease (COPD, Maruoka, Nippon Rinsho, 1999 , 57, 1982-7), hypertension, pulmonary hypertension (Fukumoto et al. Heart, 91, 391-2, 2005, Mukai et al. Nature 1997, 389, 990-4) and ocular hypertension and regulation of intraoccular pressure (Honjo et al. Invest. Ophthalmol. Visual Sci. 2001, 42, 137-144), endothelial dysfunction (Steioffet al. Eur. J. Pharmacol. 2005, 512, 247-249), angina (Masumoto et al. Circ 2002, 105, 1545-47, Shimokawa et al. JCP, 2002, 40, 751-761), nephropathy, including hypertension-induced, non-hypertension-induced, and diabetic nephropathies, renal failure and peripheral arterial occlusive disease (PAOD) (Wakino et al. Drug News Perspect. 2005, 18, 639-43), myocardial infarction (Demiryurek et al. Eur J Pharmacol. 2005, 527, 129-40, Hattori et al. Circulation, 2004, 109,2234-9), cardiac hypertrophy and failure (Yamakawa, et al. Hypertension 2000, 35, 313-318, Liao et al. Am J Physiol Cell Physiol. 2006, 290, C661-8, Kishi et al. Circ 2005, 111, 2741-2747), coronary heart disease, artherosclerosis, restenosis (Pacaud et al. Arch. Mal. Coeur 2005, 98, 249-254, Retzer, et al. FEBS Lett 2000, 466, 70; Negoro, et al. Biochem Biophys Res Commun 1999, 262, 211), diabetes, diabetic complications, glucose utilization and metabolic syndrome (Sandu, et al.Diabetes 2000, 49, 2178, Maeda et al. Cell Metab. 2005, 2, 119-29), sexual dysfunction, e.g., penile erectile dysfunction (Chitaley et al. Nature Medicine 2001, 7, 119-122), retinopathy, inflammation, immune diseases, AIDS, osteoporosis, endocrine dysfunctions, e.g. hyperaldosteronism, central nervous system disorders such as neuronal degeneration and spinal cord injury (Hara, et al. JNeurosurg 2000, 93, 94), cerebral ischemia (Uehata, et al. Nature 1997, 389, 990; Satoh et al. Life Sci. 2001, 69, 1441-53; Hitomi, et al. Life Sci 2000, 67,1929; Yamamoto, et al. J Cardiovasc Pharmacol. 2000, 35, 203-11), cerebral vasospasm (Sato, et al. Circ Res 2000, 87, 195; Kim, et al. Neurosurgery 2000, 46, 440), pain, e.g. neuropathic pain (Tatsumi, et al. Neuroscience 2005, 131,491, Inoue, et al. Nature medicine 2004, 10, 712), infection of digestive tracts with bacteria (WO 98/06433), cancer development and progression, neoplasia where inhibition of Rho kinase has been shown to inhibit tumor cell growth and metastasis (Itoh, et al. Nature Medicine 1999, 5, 221; Somlyo, et al. Res Commun 2000, 269, 652), angiogenesis (Uchida, et al. Biochem Biophys Res 2000, 269, 633-40 ; Gingras, et al. Biochem J 2000, 348, 273), vascular smooth muscle cell proliferation and motility (Tammy et al. Circ. Res. 1999, 84, 1186-1193; Tangkijvanich et al. Atherosclerosis 2001, 155, 321-327), endothelial cell proliferation, endothelial cell retraction and motility (Oikawa et al. Biochem. Biophys. Res. Commun. 2000, 269, 633-640), stress fiber formation (Kimura et al. Science 1997, 275, 1308-1311; Yamashiro et al. J. Cell Biol. 2000, 150, 797-806), thrombotic disorders (Kikkawa, et al. FEBS Lett. 2000, 466, 70-74; Bauer et al. Blood 1999, 94, 1665-1672; Klages, et al. J Cell Biol 1999, 144, 745; Retzer, et al. Cell Signal 2000, 12, 645) and leukocyte aggregation (Kawaguchi, et al. Eur J Pharmacol. 2000, 403, 203-8; Sanchez-Madrid, et al. J Immunol. 2003, 171, 1023-34, Sanchez-Madrid, et al. J Immunol. 2002, 168, 400-10), and bone resorption (Chellaiah, et al. J Biol Chem. 2003, 278, 29086-97). Na/H exchange transport system activation (Kawaguchi, et al. Eur J Pharmacol. 2000, 403, 203-8), Alzheimer's disease (Zhou et al. Science 2003, 302, 1215-1217), adducin activation (Fukata et al. J. Biol. Chem., 1998, 273, 5542-5548), and in SREB (Sterol response binding element) signalling and its effects on lipid metabolism (Lin et al. Circ. Res., 92, 1296-304, 2003).

Therefore, a compound having inhibitory effect on Rho-kinase and/or on Rho-kinase mediated phosphorylation of myosin light chain phosphatase is useful for the treatment and/or prevention of cardiovascular and non-cardiovascular diseases involving Rho-kinase as the primary or secondary disease cause, like hypertension, pulmonary hypertension, ocular hypertension, retinopathy, and glaucoma, peripheral circulatory disorder, peripheral arterial occlusive disease (PAOD), coronary heart disease, angina pectoris, heart hypertrophy, heart failure, ischemic diseases, ischemic organ failure (end organ damage), fibroid lung, fibroid liver, liver failure, nephropathy, including hypertension-induced, non-hypertension-induced, and diabetic nephropathies, renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain, neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, AIDS, osteopathy such as osteoporosis, infection of digestive tracts with bacteria, sepsis, cancer development and progression, e.g. cancers of the breast, colon, prostate, ovaries, brain and lung and their metastases.

WO 01/64238 describes isoquinoline-5-sulfonamide derivatives optionally substituted by a -(CH₂)₁₋₆-O-(CH₂)₀₋₆-, a -(CH₂)₀₋₆-S-(CH₂)₀₋₆- or a -(CH₂)₀₋₆-linked heterocyclic group useful as neuroprotective agents.

WO 2004/106325 (Schering AG) describes prodrugs of the Rho-kinase inhibitor fasudil carrying an ether or ester group in the 1-position of the isoquinoline ring.

WO 2001/039726 generically describes -O-(C₀-C₁₀)alkyl-heteroaryl substituted cyclohexyl derivatives useful for the treatment of microbial infections.

JP 10087629 A describes isoquinoline derivatives useful for the treatment of diseases caused by Heliobacter pylori such as for example gastritis cancer or ulcer. The isoquinoline derivatives may be substituted by OH in the 1-position and are preferably 5-substituted by X-[(C₁-C₆)alkylene)]₀₋₁-Y wherein X may be oxygen and Y may be an aryl or a heterocyclic group.

Hagihara et al. (Bioorg. Med. Chem. 1999, 7, 2647-2666) disclose 6-benzyloxy-isoquinoline for the treatment of infections caused by Heliobacter pylori.

US 5,480,883 generically discloses as EGF and/or PDGF receptor inhibitors useful for inhibiting cell proliferation compounds of the formula "Ar I - X - Ar II" wherein X may be (CHR₁)ₘ-Z-(CHR₁)ₙ, e.g. Z-CH₂, wherein Z may be O, R₁ is hydrogen or alkyl, Ar I may be among others an optionally substituted isoquinolone and Ar II may be among others an optionally substituted C₃₋₇ monocyclic saturated heterocyclic system.

WO 2005/030791 (Merck & Co.) generically describes as potassium channel inhibitors for the treatment of cardiac arrhythmias, stroke, congestive heart failure etc. isoquinolone derivatives which are optionally substituted in 6-position by a group (CR^{e}R^{f})ₚOR⁴³ wherein p may be zero, and R⁴³ is e.g. a (C₃-C₁₀)cycloalkyl residue optionally substituted by NR⁵¹R⁵², wherein R⁵¹ and R⁵² may be hydrogen, (C₁-C₆)alkyl etc.; or R⁴³ is a group R⁸¹ defined as a 4-6 membered unsaturated or saturated monocyclic heterocylic ring with 1, 2, 3 or 4 heteroatoms; and are substituted by a directly bound optionally substituted aryl or heteroaryl ring in the 4-position.

WO 2005/030130 (Merck & Co.) generically describes as potassium channel inhibitors for the treatment of cardiac arrhythmias, stroke, congestive heart failure etc. isoquinoline derivatives which may be substituted by hydroxyl in the 1-position and are optionally substituted in 6-position by a group (CR^{e}R^{f})ₚOR⁴³ wherein p may be zero, and R⁴³ is e.g. a (C₃-C₁₀)cycloalkyl residue optionally substituted by NR⁵¹R⁵², wherein R⁵¹ and R⁵² may be hydrogen, (C₁-C₆)alkyl etc.; or R⁴³ is a group R⁸¹ defined as a 4-6 membered unsaturated or saturated monocyclic heterocylic ring with 1, 2, 3 or 4 heteroatoms; and are substituted by a directly bound optionally substituted aryl or heteroaryl ring in the 4-position.

WO 03/053330 (Ube) generically describes isoquinolone derivatives of the formula 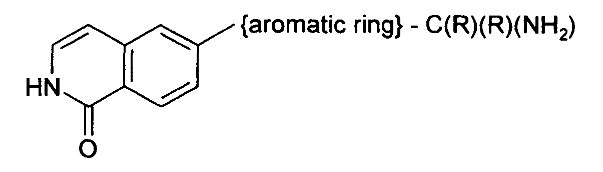 as Rho-kinase inhibitors.

WO 2008/020081 A1 describes isoquinolinamine and isoquinolone derivatives as Rho-kinase inhibitors which are inter alia substituted in the six position by a group -X-(C₄-C₇)cycloalkyl-(CH2)ₙ-NR4R5 wherein X is O, S or NH, n is 0 or 1, R4 is H or optionally substituted (C₁-C₆)alkyl and R5 is H or (C₁-C₄)alky.

An embodiment of the present invention is a compound of the formula (I) 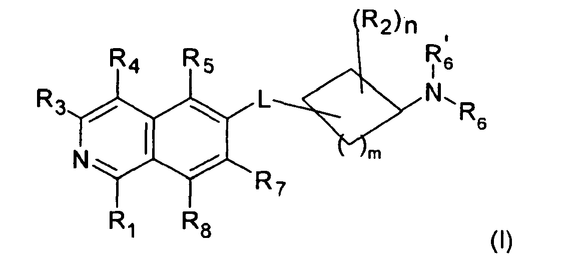 wherein
R₁ is H, OH or NH₂ ;
R₂ is H, halogen or (C₁-C₆)alkyl;
R₃ is
H,
halogen,
(C₁-C₆)alkyl,
(C₁-C₆)alkylene-R',
OH,
O-R"
NH₂,
NHR",
NR"R" or
NH-C(O)-R",
R₄ is
H,
halogen,
hydroxy,
CN,
(C₁-C₆)alkyl,
R',
(C₁-C₆)alkylene-R';
R₅ is
H,
halogen,
CN,
N02,
(C₁-C₆)alkyl,
(C₂-C₆)alkenyl,
R',
(C₁-C₆)alkylene-(C₆-C₁₀)aryl,
(C₂-C₆)alkenylene-(C₆-C₁₀)aryl,
(C₁-C₆)alkylene-(C₅-C₁₀)heterocyclyl,
CH(OH)-(C₁-C₆)alkyl,
NH2,
NH-R',
NH-SO₂H,
NH-SO₂-(C₁-C₆)alkyl,
NH-SO₂-R',
NH-C(O)-(C₁-C₆)alkyl,
NH-C(O)-R',
C(O)N[(C₁-C₆)alkyl]₂,
C(O)OH, or
C(O)O-(C₁-C₆)alkyl;
R₆ and R₆' are independently of each other
H,
R',
(C₁-C₈)alkyl,
(C₁-C₆)alkylene-R',
(C₁-C₆)alkylene-O-(C₁-C₆)alkyl,
(C₁-C₆)alkylene-O-R',
(C₁-C₆)alkylene-CH[R']2,
(C₁-C₆)alkylene-C(O)-R',
(C₁-C₆)alkylene-C(O)NH₂,
(C₁-C₆)alkylene-C(O)NH-R',
(C₁-C₆)alkylene-C(O)NH-(C₁-C₆)alkyl,
(C₁-C₆)alkylene-C(O)N[(C₁-C₆)alkyl]₂,
(C₁-C₆)alkylene-C(O)N[R']₂;
(C₁-C₆)alkylene-C(O)O-(C₁-C₆)alkyl,
C(O)O-(C₁-C₆)alkyl,
C(O)OR'
C(O)(C₁-C₆)alkyl,
C(O)R',
C(O)NH-(C₁-C₆)alkyl,
C(O)NHR',
C(O)N[(C₁-C₆)alkyl]R'
C(O)N[(C₁-C₆)alkyl]₂,
C(O)-(C₁-C₆)alkylene-R',
C(O)O(C₁-C₆)alkylene-R',
or R₆ and R₆', together with the N-atom to which they are attached, form a (C₅-C₁₀) heterocyclyl group;
R₇ is
H,
halogen,
CN,
NO₂,
(C₁-C₆)alkyl,
O-(C₁-C₆)alkyl,
(C₂-C₆)alkenyl,
R',
(C₂-C₆)alkenylene-(C₆-C₁₀)aryl,
(C₁-C₆)alkylene-R',
CH(OH)-(C₁-C₆)alkyl,
NH2,
NH-R',
NH-SO₂H,
NH-SO₂-(C₁-C₆)alkyl,
NH-SO₂-R',
SO₂-NH₂,
SO₂-NHR',
NH-C(O)-(C₁-C₆)alkyl,
NH-C(O)-R',
C(O)N[(C₁-C₆)alkyl]₂,
C(O)OH, or
C(O)O-(C₁-C₆)alkyl;
R₈ is H, halogen or (C₁-C₆)alkyl;
n is 1, 2, 3 or 4;
m is 1, 2, 3, 4 or 5, and
L is NH(CH₂)p, N(C₁-C₆)alkyl-(CH₂)p;
p is 0, 1, 2, 3 or 4;
wherein
R' is
(C₃-C₈)cycloalkyl,
(C₅-C₁₀)heterocyclyl,
(C₆-C₁₀)aryl; and
R" is
(C₃-C₈)cycloalkyl,
(C₅-C₁₀)heterocyclyl,
(C₆-C₁₀)aryl;
(C₁-C₆)alkyl,
(C₁-C₆)a)kylene-R',
(C₁-C₆)alkylene-O-(C₁-C₆)alkyl,
(C₁-C₆)alkylene-O-R', or
(C₁-C₆)alkylene-NRₓR_{y}; and
wherein Rₓ and Ry are independently of each other
(C₁-C₆)alkyl,
(C₅-C₁₀)heterocyclyl,
(C₆-C₁₀)aryl,
(C₁-C₄)alkylene-(C₅-C₁₀)heterocyclyl,
(C₁-C₄)alkylene-(C₆-C₁₀)aryl,
(C₁-C₄)alkylene-NH(C₁-C₆)alkyl,
(C₁-C₄)alkylene-N[(C₁-C₆)alkyl]₂,
(C₁-C₄)alkylene-N[(C₆-C₁₀)aryl]₂, or
(C₁-C₄)alkylene-N[(C₅-C₁₀)heterocyclyl]₂;
wherein in residues R₄, R₅, R₆, R₆', R₇ and R₈ alkyl, alkylene or cycloalkyl can optionally be substituted one or more times by OH, OCH₃, COOH, COOCH₃, NH₂, NHCH₃, N(CH₃)₂, CONH₂, CONHCH₃ or CON(CH₃)₂ ;
wherein in residues R₂ to R₈ alkyl or alkylene can optionally be substituted one or more times by halogen;
wherein in residues R₃ to R₈ (C₆-C₁₀)aryl and (C₅-C₁₀)heterocyclyl are unsubstituted or substituted one or more times by suitable groups independently selected from halogen, OH, NO₂, N₃, CN, C(O)-(C₁-C₆)alkyl, C(O)-(C₆-C₁₀)aryl, COOH, COO(C₁-C₆)alkyl, CONH₂, CONH(C₁-C₆)alkyl, CON[(C₁-C₆)alkyl]₂, (C₃-C₈)cycloalkyl, (C₁-C₆)alkyl, (C₁-C₆)alkylene-OH, (C₁-C₆)alkylene-NH₂, (C₁-C₆)alkylene-NH(C₁-C₆)alkyl, (C₁-C₆)alkylene-N[(C₁-C₆)alkyl]₂, (C₂-C₆)alkenyl, (C₂-C₆)alkynyl, O-(C₁-C₆)alkyl, O-C(O)-(C₁-C₆)alkyl, PO₃H₂, SO₃H, SO₂-NH₂, SO₂NH(C₁-C₆)alkyl, SO₂N[(C₁-C₆)alkyl]₂, S-(C₁-C₆)alkyl, SO-(C₁-C₆)alkyl, SO₂-(C₁-C₆)alkyl,
SO₂-N=CH-N[(C₁-C₆)alkyl]₂,
C(NH)(NH₂), NH₂, NH-(C₁-C₆)alkyl, N[(C₁-C₆)alkyl]₂, NH-C(O)-(C₁-C₆)alkyl, NH-C(O)O-(C₁-C₆)alkyl,
NH-SO₂-(C₁-C₆)alkyl, NH-SO₂-(C₆-C₁₀)aryl, NH-SO₂-(C₅-C₁₀)heterocyclyl, N(C₁-C₆)alkyl-C(O)-(C₁-C₆)alkyl, N(C₁-C₆)alkyl-C(O)O-(C₁-C₆)alkyl,
N(C₁-C₆)alkyl-C(O)-NH-(C₁-C₆)alkyl],
(C₆-C₁₀)aryl, (C₁-C₆)alkylene-(C₆-C₁₀)aryl, O-(C₆-C₁₀)aryl,
O-(C₁-C₆)alkylene-(C₆-C₁₀)aryl, (C₅-C₁₀)heterocyclyl,
(C₁-C₆)alkylene-(C₅-C₁₀)heterocyclyl, or O-(C₁-C₆)alkylene-(C₅-C₁₀)heterocyclyl, wherein the (C₆-C₁₀)aryl or (C₅-C₁₀)heterocyclyl may be substituted one to three times by a group independently selected from halogen, OH, NO₂, CN, O-(C₁-C₆)alkyl, (C₁-C₆)alkyl, NH₂, NH(C₁-C₆)alkyl, N[(C₁-C₆)alkyl]₂, SO₂CH₃, COOH, C(O)O-(C₁-C₆)alkyl, CONH₂, (C₁-C₆)alkylene-O-(C₁-C₆)alkyl, (C₁-C₆)alkylene-O-(C₆-C₁₀)aryl, or O-(C₁-C₆)alkylene-(C₆-C₁₀)aryl;
or wherein (C₆-C₁₀)aryl is vicinally substituted by a O-(C₁-C₄)alkylene-O group whereby a 5-8-membered ring is formed together with the carbon atoms the oxygen atoms are attached to;
and wherein aryl or heterocyclyl substituents of (C₆-C₁₀)aryl and (C₅-C₁₀)heterocyclyl groups may not be further substituted by an aryl or heterocyclyl containing group;
or their stereoisomeric and/or tautomeric forms and/or their pharmaceutically acceptable salts thereof.

In one embodiment of the present invention R₁ is H and the compound is characterized by the formula (II)

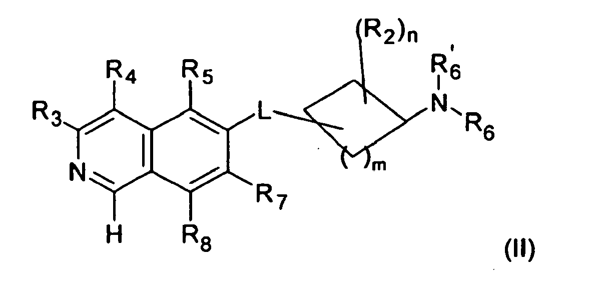

In another embodiment of the present invention R₁ is OH and the compound is characterized by the formula (III)

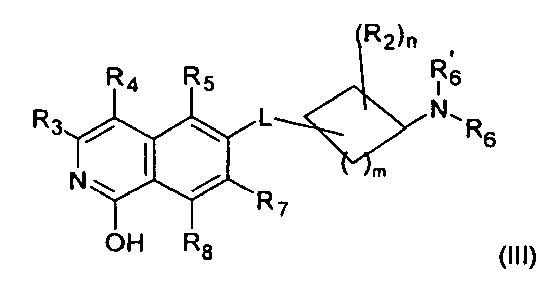

The isoquinoline derivative of formula (I), wherein R₁ is OH, include the corresponding tautomeric 1-isoquinolone derivative which is characterized by the formula (III')

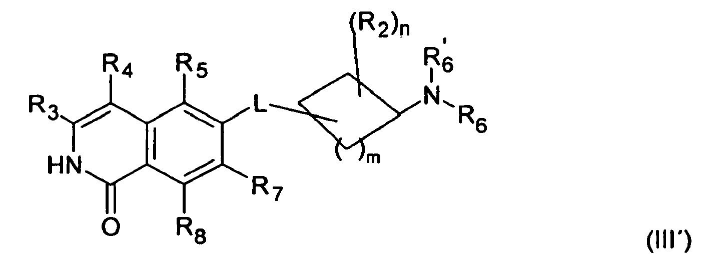

This tautomeric form is also an embodiment of the present invention.

In a further embodiment R₁ is NH₂ and the compound is characterized by the formula (IV)

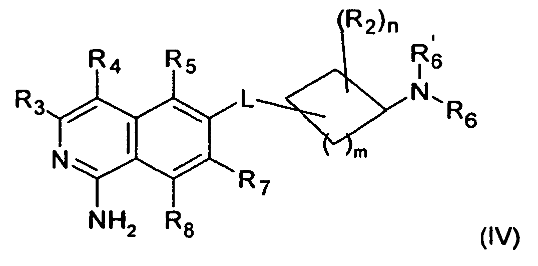

The following embodiments refer to the compounds of formula (I), (II), (III), (III') and (IV).
R₁ is preferably H or OH;
R₃ is preferably H, halogen, (C₁-C₄)alkylene-R', O-R" or NHR". More preferred, R₃ is H or NHR". Most preferred, R₃ is H, NH-(C₅-C₆)heterocyclyl or NH-phenyl, especially preferred are H, NH-(C₅-C₆)heteroaryl containing one or more N atoms or NH-phenyl.
Most especially preferred, R₃ is H.
Examples of R₃ substituents are

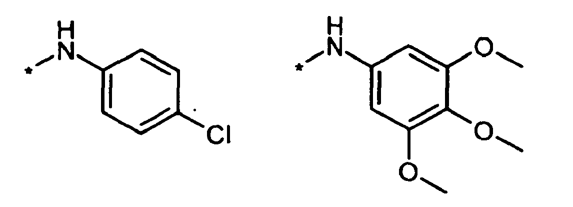

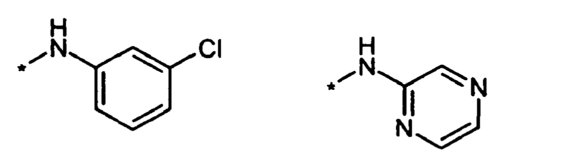

Preferably, R₄ is H, halogen or (C₁-C₆)alkyl. More preferred, R₄ is H, halogen or (C₁-C₄)alkyl. Most preferred, R₄ is H.

Preferably, R₅ is H, halogen, CN, (C₁-C₆)alkyl, (C₂-C₆)alkenyl, R', NH-(C₆-C₁₀)aryl or (C₁-C₆)alkylene-R'. More preferably, R₅ is H, halogen, (C₁-C₆)alkyl, (C₂-C₆)alkenyl, R', NH-(C₆-C₁₀)aryl or (C₁-C₆)alkylene-R'. Most preferably, R₅ is H, halogen, (C₁-C₆)alkyl, (C₂-C₆)alkenyl, (C₆-C₁₀)aryl, NH-(C₆-C₁₀)aryl, (C₁-C₂)alkyl-(C₆-C₁₀)aryl or (C₅-C₁₀)heteroaryl. Especially preferred, R₅ is H, halogen, phenyl, (C₁-C₆)alkyl, (C₂-C₆)alkenyl, (C₆-C₁₀)aryl or (C₅-C₆)heteroaryl. Most especially preferred R₅ is H, halogen, methyl, ethyl, vinyl, phenyl, thienyl or pyridyl.
Examples of R₅ are hydrogen, fluoro, chloro, bromo, iodo, methyl, ethyl, vinyl, phenyl, thienyl or pyridyl, nitrile, nitro, (p-methoxy)-phenyl, N-aniline, benzyl, 2-propenyl, s-butenyl, cyclopropyl, tetrazol, amino, 4-methoxy-aniline or N-acetyl, preferably hydrogen, fluoro, chloro, bromo, iodo, methyl, ethyl, vinyl, phenyl, thienyl or pyridyl More preferred, R₅ is H, halogen, methyl, or ethyl, most preferred R₅ is H.

Preferably, R₆ and R₆' are independently of each other
H, (C₁-C₆)alkyl, R', (C₁-C₄)alkylene-(C₃-C₈)cycloalkyl,
(C₁-C₄)alkylene-(C₅-C₁₀)heterocyclyl, (C₁-C₄)alkylene-(C₆-C₁₀)aryl, (C₁-C₆)alkylene-O-(C₁-C₆)alkyl, (C₁-C₄)alkylene-C(O)-(C₅-C₁₀)heterocyclyl, (C₁-C₄)alkylene-C(O)-(C₆-C₁₀)aryl, (C₁-C₆)alkylene-C(O)N[(C₁-C₆)alkyl]₂, (C₁-C₆)alkylene-C(O)NH-(C₁-C₆)alkyl, (C₁-C₆)alkylene-C(O)O-(C₁-C₆)alkyl, C(O)R' C(O)(C₁-C₆)alkyl, C(O)O-(C₁-C₆)alkyl, C(O)NH-(C₁-C₆)alkyl, C(O)N[(C₁-C₆)alkyl]₂, or C(O)(C₁-C₆)alkylene-R', or
R₆ and R₆', together with the N-atom to which they are attached, form a (C₅-C₁₀)heterocyclyl group.

In a further preferred embodiment, R₆ and R₆' are independently of each other H, (C₁-C₆)alkyl, (C₅-C₁₀)heterocyclyl, (C₃-C₈)cycloalkyl, (C₆-C₁₀)aryl, (C₁-C₄)alkylene-(C₃-C₈)cycloalkyl, (C₁-C₄)alkylene-(C₅-C₁₀)heterocyclyl, (C₁-C₄)alkylene-(C₆-C₁₀)aryl, (C₁-C₆)alkylene-O-(C₁-C₆)alkyl, (C₁-C₆)alkylene-C(O)N[(C₁-C₆)alkyl]₂, (C₁-C₆)alkylene-C(O)NH-(C₁-C₆)alkyl, (C₁-C₆)alkylene-C(O)O-(C₁-C₆)alkyl, C(O)O-(C₁-C₆)alkyl, C(O)(C₁-C₆)alkyl, C(O)(C₃-C₈)cycloalkyl, C(O)NH-(C₁-C₆)alkyl, C(O)N[(C₁-C₆)alkyl]₂, C(O) (C₁-C₆)alkylene-(C₃-C₈)cycloalkyl, C(O)(C₁-C₆)alkylene-(C₅-C₁₀)heterocyclyl, C(O)(C₁-C₆)alkylene-(C₆-C₁₀)aryl, or R₆ and R₆', together with the N-atom to which they are attached form a (C₅-C₁₀)heterocyclyl group.

In a more preferred embodiment, R₆ is H, (C₁-C₆)alkyl, (C₃-C₆)cycloalkyl or (C₁-C₄)alkylene-(C₃-C₆)cycloalkyl, and
R₆' is H, (C₁-C₆)alkyl, (C₃-C₈)cycloalkyl, (C₅-C₁₀)heterocyclyl, (C₆-C₁₀)aryl, (C₁-C₄)alkylene-(C₃-C₈)cycloalkyl, (C₁-C₄)alkylene-(C₅-C₁₀)heterocyclyl, (C₁-C₄)alkylene-(C₆-C₁₀)aryl, (C₁-C₆)alkylene-O-(C₁-C₆)alkyl, (C₁-C₆)alkylene-C(O)NH-(C₁-C₆)alkyl, (C₁-C₆)alkylene-C(O)N[(C₁-C₆)alkyl]₂, (C₁-C₆)alkylene-C(O)O-(C₁-C₆)alkyl, C(O)O-(C₁-C₆)alkyl, C(O)(C₁-C₆)alkyl, C(O)(C₃-C₈)cycloalkyl, C(O)NH-(C₁-C₆)alkyl, C(O)N[(C₁-C₆)alkyl]₂, C(O)(C₁-C₆)alkylene-C₃-C₈)cycloalkyl, C(O)(C₁-C₆)alkylene-(C₅-C₁₀)heterocyclyl, C(O)(C₁-C₆)alkylene-(C₆-C₁₀)aryl, or R₆ and R₆', together with the N-atom to which they are attached, form a (C₅-C₁₀)heterocyclyl group.

In a further more preferred embodiment, R₆ is H, (C₁-C₆)alkyl and R₆' is
H, (C₁-C₆)alkyl, (C₃-C₈)cycloalkyl, (C₆-C₁₀)aryl,(C₅-C₁₀)heterocyclyl, (C₁-C₄)alkylene-(C₃-C₈)cycloalkyl, (C₁-C₄)alkylene-(C₅-C₁₀)heterocyclyl, (C₁-C₆)alkylene-(C₆-C₁₀)aryl, (C₁-C₄)alkylene-O-(C₁-C₄)alkyl, (C₁-C₄)alkylene-C(O)N[(C₁-C₄)alkyl]₂, (C₁-C₆)alkylene-C(O)NH-(C₁-C₆)alkyl, C(O)(C₁-C₆)alkyl, C(O)(C₁-C₆)alkylene-(C₅-C₁₀)heterocyclyl, or
R₆ and R₆', together with the N-atom to which they are attached, form a (C₅-C₁₀)heterocyclyl group.

In a further even more preferred embodiment, R₆ is H, (C₁-C₆)alkyl and R₆' is
H,
(C₁-C₆)alkyl;
(C₃-C₈)cycloalkyl;
(C₁-C₄)alkylene-(C₃-C₈)cycloalkyl;
(C₁-C₄)alkylene-O-(C₁-C₄)alkyl;
(C₁-C₄)alkylene-C(O)N[(C₁-C₄)alkyl]₂;
(C₁-C₄)alkylene-(C₅-C₁₀)heterocyclyl, or
(C₁-C₄)alkylene-(C₆-C₁₀)aryl;
C(O)(C₁-C₄)alkyl;
C(O)(C₁-C₄)alkylene-(C₅-C₁₀)heterocyclyl;
or R₆ and R₆', together with the N-atom to which they are attached, form a (C₅-C₆)heterocyclyl group.

Preferably the formed heterocyclyl group is morpholino, piperidino, pyrrolidino or piperazino. More preferably the heterocyclyl group is morpholino or piperazinyl.

In a most preferred embodiment, R₆ is H, (C₁-C₆)alkyl and R₆' is H, (C₁-C₆)alkyl, (C₃-C₈)cycloalkyl.

In a further most preferred embodiment, R₆ is H and R₆' is H, preferably unsubstituted (C₁-C₆)alkyl, or preferably unsubstituted (C₃-C₈)cycloalkyl. Especially preferred, R₆ and R₆' are H.

As examples for these embodiments, R₆ or R₆' are, independently from each other, hydrogen, methyl, ethyl, propyl, isopropyl, 3-methyl-butyl, 2-methyl-propyl, butyl, pentyl, 3,3,3-trifuoropropyl, 4,4,4-trifluorobutyl or a substituent selected from the group consisting of

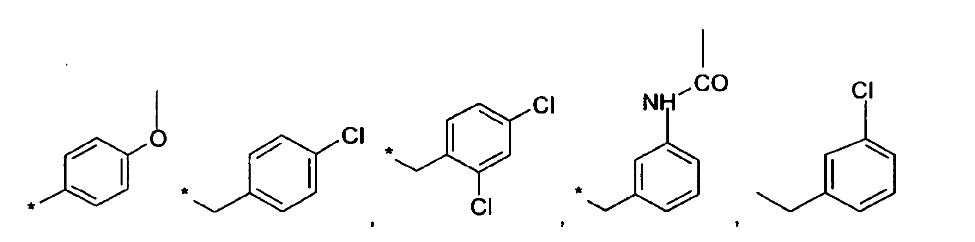

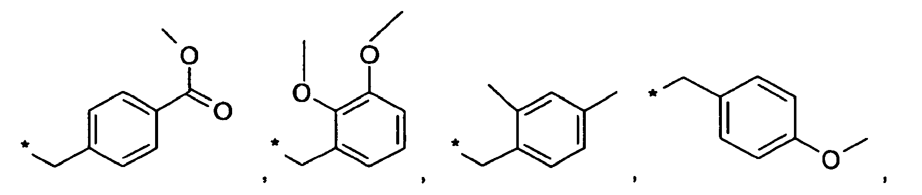

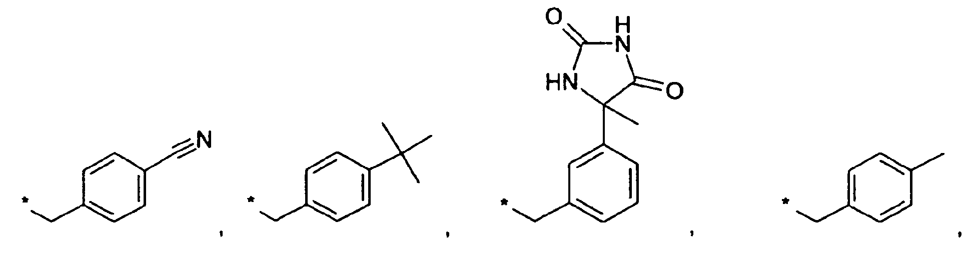

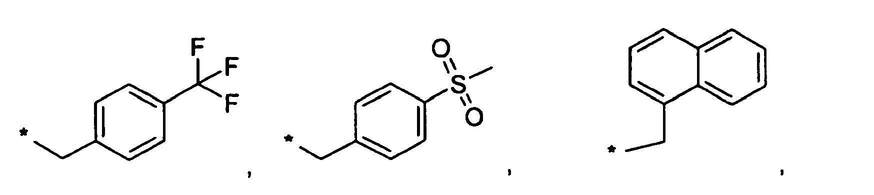

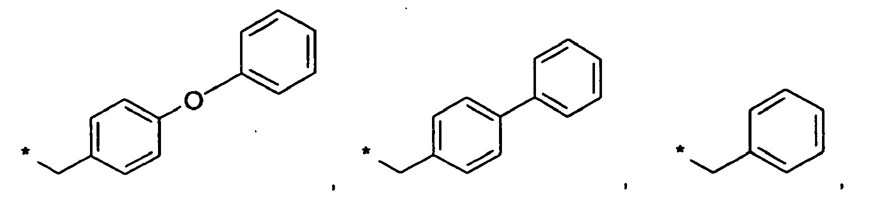

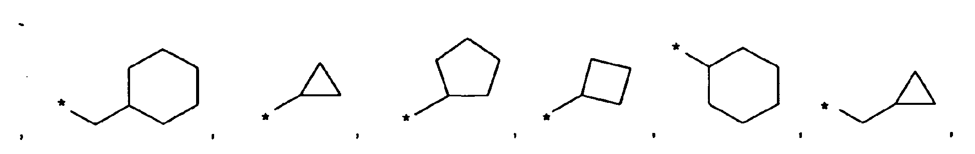

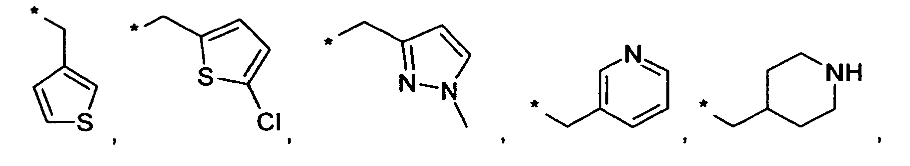

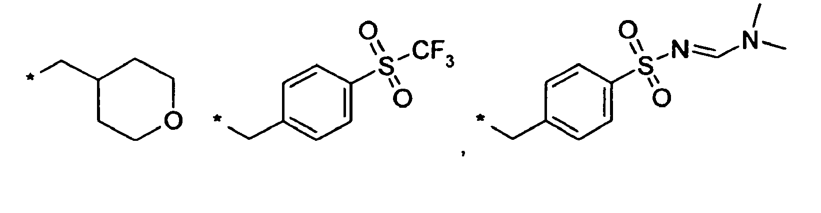

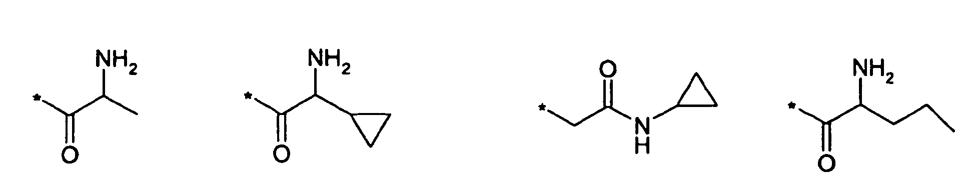

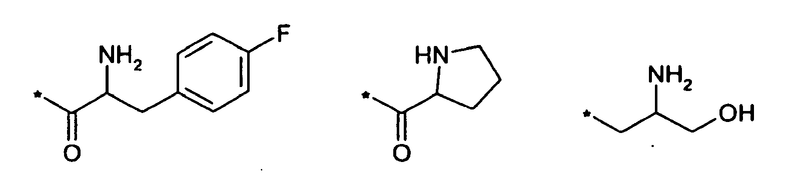

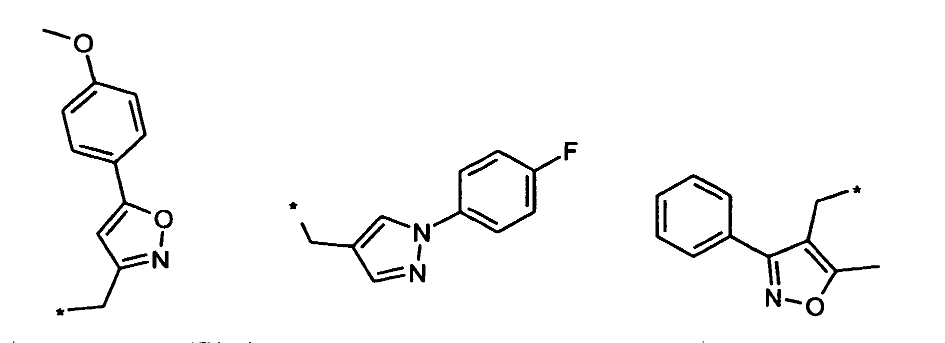

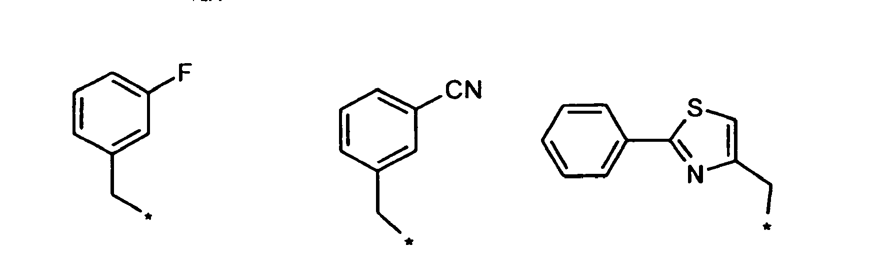

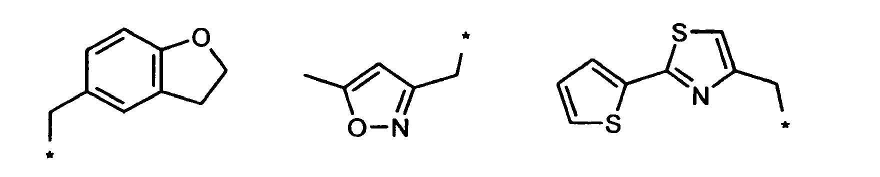

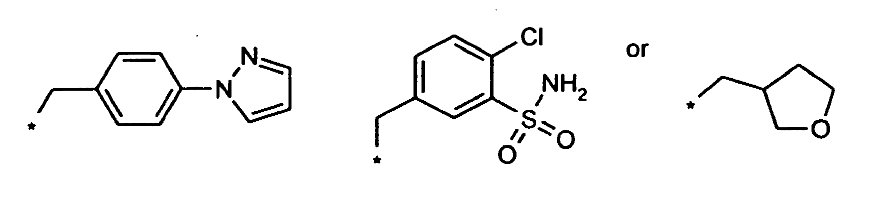

The asterisk (*) denotes where the bond is connected to the N-atom of the amine.

Preferably, R₇ is H, halogen, CN, (C₁-C₆)alkyl, O-(C₁-C₆)alkyl, (C₂-C₆)alkenyl, R' or (C₁-C₆)alkylene-(C₃-C₈)cycloalkyl. More preferred, R₇ is H, halogen, CN, (C₁-C₄)alkyl, O-(C₁-C₄)alkyl, (C₁-C₄)alkenyl, phenyl, cyclopropyl or (C₅-C₆)heteroaryl. Most preferably, R₇ is H, fluoro, chloro, bromo, methyl, ethyl, methoxy, phenyl, nitrile, cyclopropyl, thienyl or vinyl, most especially preferred R₇ is H, fluoro, chloro, methyl or methoxy. More particular preferred R₇ is H.
R₈ is preferably H, halogen or (C₁-C₄)alkyl. More preferred, R₈ is H, Cl, F, methyl or ethyl. Most preferred R₈ is H.

Preferably, R₂ is H, halogen or (C₁-C₄)alkyl. Preferably, R₂ is H or (C₁-C₂)alkyl. More preferred, R₂ is H, methyl or ethyl. Most preferred R₂ is H. R₂ may be bound to any carbon atom of the ring including the position where the linker group L is bound.

Preferably, n is 1, 2 or 3. More preferred, n is 1 or 2. Most preferred n is 1.

Preferably m is 2, 3 or 4. More preferred m is 3. In a further embodiment m is 1, 2, 4 or 5.

The linker group L may be bound to the ring in any position via a ring carbon atom. In a preferred embodiment, m is 3 and L is attached to the 4-position of the amino cyclohexane ring 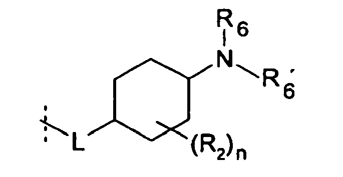 or L is attached to the 3-position of the amino cyclohexane ring 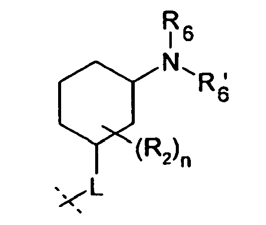 in all their stereochemical forms.

In an especially preferred embodiment, L is attached to the 4-position of the amino cyclohexane ring.

In a further embodiment of L, L is NH(CH₂)p or N(C₁-C₆)alkyl-(CH₂)p. A preferred N(C₁-C₆)alkyl is N(C₁-C₄)alkyl, more preferably methyl or ethyl with methyl being more preferred. Even more preferred L is NH(CH₂)p. A preferred N(C₁-C₆)alkyl is N(C₁-C₄)alkyl, more preferably NCH₃ or NCH₂CH₃ with NCH₃ being more preferred. Most preferred L is NH.

Preferably p is 0, 1, 2, or 3, more preferred 0 or 1, with 0 being most preferred.

More preferably, m is 3 and L is NH and is attached to the 4-position of the amino cyclohexane ring.

In residues R₂ to R₈ an alkyl or alkylene can optionally be substituted one or more times by halogen. Preferably alkyl or alkylene is substituted one to three times by halogen selected from chloro or bromo but may be substituted by fluoro once or more, e.g. being perfluorinated. Preferably halogen is fluor. More preferred an alkyl or alkylene is not halogenated.

In residues R₄, R₅, R_{6,} R₆', R₇ and R₈ alkyl, alkylene or cycloalkyl can optionally be substituted one or more times by a group selected independently from OH, OCH₃, COOH, COOCH₃, NH₂, NHCH₃, N(CH₃)₂, CONH₂, CONHCH₃ or CON(CH₃)₂.
If substituted, the number of substituents is preferably between 1, 2, 3 or 4, more preferably 1 or 2 with 1 being even more preferred. Preferably an alkylene or cycloalkyl is not substituted. More preferably an alkyl, alkylene or cycloalkyl is not substituted. Preferably alkyl, alkylene or cycloalkyl in R₄, R₅, R₇ and R₈ are not substituted. In a further embodiment alkyl, alkylene or cycloalkyl in R₄, R₅, R₆, R₆', R₇ and R₈ are not substituted.

In preferred embodiments of the present invention one or more or all of the groups contained in the compounds of formula (I) can independently of each other have any of the preferred, more preferred or most preferred definitions of the groups specified above or any one or some of the specific denotations which are comprised by the definitions of the groups and specified above, all combinations of preferred definitions, more preferred or most preferred and/or specific denotations being a subject of the present invention. Also with respect to all preferred embodiments the invention includes the compounds of the formula (I) in all stereoisomeric forms and mixtures of stereoisomeric forms in all ratios, and their pharmaceutically acceptable salts.

The term "*-" in the exemplified substituents *vide supra* marks the point where the substituent is attached, which means, for example, for a R₃ substituent 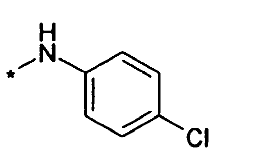 and m is 3 a compound of the formula

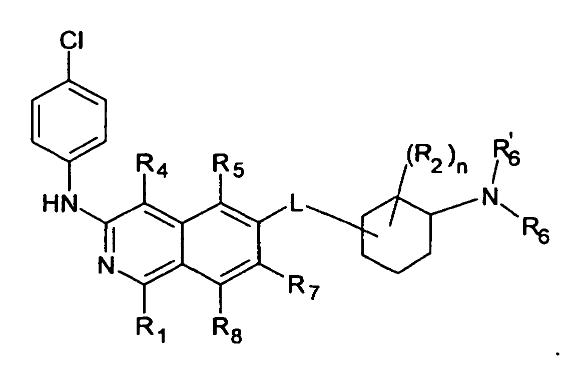

A preferred embodiment is a compound of the formula (I) wherein
R₁ is H or OH
R₂ is hydrogen, halogen, or (C₁-C₆)alkyl;
R₃ is H, halogen, (C₁-C₄)alkylene-R', O-R" or NHR";
R₄ is H, halogen or (C₁-C₆)alkyl;
R₅ is H, (C₁-C₆)alkyl, halogen, CN, (C₂-C₆)alkenyl, (C₆-C₁₀)aryl, NH-(C₆-C₁₀)aryl,
(C₁-C₆)alkylene-(C₆-C₁₀)aryl, (C₅-C₁₀)heterocyclyl or
(C₁-C₆)alkylene-(C₅-C₁₀)heterocyclyl;
R₆ and R₆' are independently of each other H, R', (C₁-C₈)alkyl, (C₁-C₆)alkylene-R', (C₁-C₆)alkylene-O-(C₁-C₆)alkyl, (C₁-C₆)alkylene-O-R', (C₁-C₆)alkylene-CH[R']₂, (C₁-C₆)alkylene-C(O)NH₂, (C₁-C₆)alkylene-C(O)NH-R', (C₁-C₆)alkylene-C(O)N[(C₁-C₄)alkyl]₂, (C₁-C₆)alkylene-C(O)N[R']₂, C(O)O-(C₁-C₆)alkyl, C(O)(C₁-C₆)alkyl, C(O)(C₃-C₈)cycloalkyl, C(O)(C₅-C₁₀)heterocyclyl, C(O)NH-(C₁-C₆)alkyl, C(O)N[(C₁-C₆)alkyl]₂, C(O)-(C₁-C₆)alkylene-C₃-C₈)cycloalkyl,
C(O)(C₁-C₆)alkylene-(C₅-C₁₀)heterocyclyl, C(O)(C₁-C₆)alkylene-(C₆-C₁₀)aryl,
or R₆ and R₆', together with the N-atom to which they are attached, form a (C₅-C₆)heterocyclyl group.
R₇ is H, halogen, CN, (C₁-C₆)alkyl, O-(C₁-C₆)alkyl, (C₂-C₆)alkenyl or R';
R₈ is H, halogen or (C₁-C₆)alkyl;
m is 2, 3 or 4
n is 1, 2 or 3, and
L is NH(CH₂)p or N(C₁-C₆)alkyl-(CH₂)p; and
p is 0, 1 or 2;
and their stereoisomeric and/or tautomeric forms and/or their pharmaceutically acceptable salts.

A further preferred embodiment is a compound of the formula (I) wherein
R₁ is H or OH;
R₂ is H or (C₁-C₄)alkyl;
R₃ is H, halogen or NHR", wherein R" is defined as above;
R₄ is H, halogen or (C₁-C₄)alkyl;
R₅ is H, (C₁-C₆)alkyl, halogen, (C₂-C₄)alkenyl, (C₆-C₁₀)aryl, (C₁-C₆)alkylene-(C₆-C₁₀)aryl or (C₅-C₁₀)heterocyclyl;
R₆ and R₆' are independently of each other H, (C₃-C₈)cycloalkyl, (C₁-C₈)alkyl, (C₁-C₆)alkylene-O-(C₁-C₆)alkyl, (C₁-C₃)alkylene-R'; C(O)(C₁-C₆)alkyl, C(O)(C₃-C₈)cycloalkyl, C(O)(C₅-C₁₀)heterocyclyl, C(O)(C₁-C₆)alkylene-(C₃-C₈)cycloalkyl, C(O)(C₁-C₆)alkylene-(C₅-C₁₀)heterocyclyl or C(O)(C₁-C₆)alkylene-(C₆-C₁₀)aryl;
R₇ is H, halogen, CN, (C₁-C₆)alkyl, O(C₁-C₆)alkyl, (C₂-C₆)alkenyl or R';
R₈ is H, halogen or (C₁-C₆)alkyl;
m is 2, 3 or 4
n is 1, 2 or 3; and
L is NH(CH₂)p,
p is 0 or 1;
or their stereoisomeric and/or tautomeric forms and/or their pharmaceutically acceptable salts.

An especially preferred embodiment is a compound of the formula (I) wherein
R₁ is H or OH;
R₂ is H, (C₁-C₄)alkyl;
R₃ is H, NH-(C₅-C₆)heteroaryl or NH-phenyl;
R₄ is H, halogen or (C₁-C₄)alkyl;
R₅ is H, (C₁-C₄)alkyl, halogen, (C₁-C₄)alkenyl, (C₆-C₁₀)aryl, (C₁-C₂)alkyl-(C₆-C₁₀)aryl or (C₅-C₆)heteroaryl;
R₆ is H, (C₃-C₆)cycloalkyl or (C₁-C₄)alkyl;
R₆' is H, (C₃-C₈)cycloalkyl, (C₁-C₈)alkyl, (C₁-C₃)alkylene-R', C(O)O-(C₁-C₆)alkyl, C(O)(C₁-C₆)alkyl, C(O)(C₃-C₆)cycloalkyl, C(O)(C₅-C₆)heterocyclyl, C(O)(C₁-C₃)alkylene-(C₃-C₆)cycloalkyl, C(O)(C₁-C₃)alkylene-(C₅-C₆)heterocyclyl, or C(O)(C₁-C₃)alkylene-phenyl;
R₇ is H, halogen, CN, (C₁-C₄)alkyl, O(C₁-C₄)alkyl, (C₁-C₄)alkenyl, phenyl, cyclopropyl, (C₅-C₆)heteroaryl;
R₈ is H, halogen or (C₁-C₄)alkyl;
m is 3
n is 1; and
L is NH;
or their stereoisomeric and/or tautomeric forms and/or their pharmaceutically acceptable salts.

In an embodiment the invention relates to a compound of formula (I) independently selected from the group of
- 26.: [4-(Isoquinolin-6-ylamino)-cyclohexyl]-carbamic acid tert-butyl ester,
- 35.: N-Isoquinolin-6-yl-cyclohexane-1,4-diamine,
- 36.: 6-trans-(4-Amino-cyclohexyl-amino)-2H-isoquinolin-1-one,
- 37.: 6-cis-(4-Amino-cyclohexyl-amino)-2H-isoquinolin-1-one,
- 38.: 6-cis-(4-Amino-cyclohexyl-amino)-7-chloro-2H-isoquinolin-1-one,
- 39.: 6-trans-(4-Amino-cyclohexyl-amino)-7-chloro-2H-isoquinolin-1-one,
- 40.: 6-cis-(2-Amino-cyclohexyl-amino)-2H-isoquinolin-1-one,
- 43.: 6-trans-(2-Amino-cyclohexylamino)-2H-isoquinolin-1-one,
- 44.: 6-cis-(4-Diethyl-amino-cyclohexylamino)-2H-iso-quinolin-1-one,
- 45.: 6-cis-(4-Ethyl-amino-cyclohexylamino)-2H-isoquinolin-1-one,
- 46.: 6-cis-(4-Dipropyl-amino-cyclohexyl-amino)-2H-iso-quinolin-1-one,
- 47.: 6-cis-(4-Propyl-amino-cyclohexyl-amino)-2H-iso-quinolin-1-one,
- 48.: 6-cis-(4-Benzyl-amino-cyclohexylamino)-2H-iso-quinolin-1-one,
- 49.: 6-cis-(4-Isopropyl-amino-cyclohexylamino)-2H-iso-quinolin-1-one,
- 50.: 6-cis-[4-(3-Chloro-benzylamino)-cyclohexylamino]-2H-isoquinolin-1-one,
- 51.: 6-cis-[4-(4-Chloro-benzylamino)-cyclohexylamino]-2H-isoquinolin-1-one,
- 52.: 6-cis-{4-[(Piperidin-4-ylmethyl)-amino]-cyclohexylamino}-2H-isoquinolin-1-one,
- 53.: 6-cis-(4-Cyclopropylamino-cyclohexylamino)-2H-isoquinolin-1-one,
- 54.: 6-cis-(4-Dicyclopropyl-amino-cyclohexylamino)-2H-isoquinolin-1-one,
- 55.: 7-Chloro-6-cis-(4-isopropylamino-cyclohexylamino)-2H-isoquinolin-1-one,
- 56.: 7-Chloro-6-cis-(4-diethylamino-cyclohexylamino)-2H-isoquinolin-1-one,
- 57.: 7-Chloro-6-trans-(4-isopropylamino-cyclohexylamino)-2H-isoquinolin-1-one,

### Reference examples :

- 61.: cis-4-(7-Chloro-isoquinolin-6-ylsulfanyl)-cyclohexylamine,
- 62.: cis-4-(7-Bromo-isoquinolin-6-ylsulfanyl)-cyclohexylamine, or
- 64.: 6-cis-(4-Amino-cyclohexylsulfanyl)-7-chloro-2H-isoquinolin-1-one,
or their stereoisomeric and/or tautomeric forms and/or their pharmaceutically acceptable salts.

As in any embodiment of the invention, in the preceding embodiments which contain preferred, more preferred, most preferred or exemplary definitions of compounds according to the invention, one or more or all of the groups can have any of its preferred, more preferred, most preferred definitions specified above or any one or some of the specific denotations which are comprised by its definitions and are specified above.

Isoquinoline substitution pattern is numbered according to IUPAC rules:

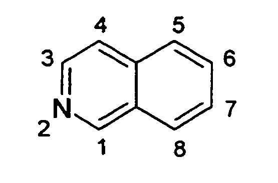

All references to "compound(s) of formula (I)" hereinafter refer to compound(s) of the formula (I), (II) (III), (III') and (IV) as described above, and their pharmaceutically acceptable salts, and/or to their stereoisomeric forms, polymorphs and solvates. Physiologically functional derivatives as described herein are also included.

Pharmaceutically acceptable salts of compounds of the formula (I) mean both their organic and inorganic salts as described in Remington's Pharmaceutical Sciences (17th edition, page 1418 (1985)). Because of the physical and chemical stability and the solubility, preference is given for acidic groups inter alia to sodium, potassium, calcium and ammonium salts; preference is given for basic groups inter alia to salts of maleic acid, fumaric acid, succinic acid, malic acid, tartaric acid, methylsulfonic acid, hydrochloric acid, sulfuric acid, phosphoric acid or of carboxylic acids or sulfonic acids, for example as hydrochlorides, hydrobromides, phosphates, sulfates, methanesulfonates, acetates, lactates, maleates, fumarates, malates, gluconates, and salts of amino acids, of natural bases or carboxylic acids. The preparation of pharmaceutically acceptable salts from compounds of the formula (I) which are capable of salt formation, including their stereoisomeric forms, takes place in a manner known per se. The compounds of the formula (I) form stable alkali metal, alkaline earth metal or optionally substituted ammonium salts with basic reagents such as hydroxides, carbonates, bicarbonates, alcoholates and ammonia or organic bases, for example trimethyl- or triethylamine, ethanolamine, diethanolamine or triethanolamine, trometamol or else basic amino acids, for example lysine, ornithine or arginine. Where the compounds of the formula (I) have basic groups, stable acid addition salts can also be prepared with strong acids. Suitable pharmaceutically acceptable acid addition salts of the compounds of the invention are salts of inorganic acids such as hydrochloric acid, hydrobromic, phosphoric, metaphosphoric, nitric and sulfuric acid, and of organic acids such as, for example, acetic acid, benzenesulfonic, benzoic, citric, ethanesulfonic, fumaric, gluconic, glycolic, isethionic, lactic, lactobionic, maleic, malic, methanesulfonic, succinic, p-toluenesulfonic and tartaric acid.

Salts with a pharmaceutically unacceptable anion such as, for example, trifluoroacetate likewise belong within the framework of the invention as useful intermediates for the preparation or purification of pharmaceutically acceptable salts and/or for use in nontherapeutic, for example in vitro, applications.

The invention relates to compounds of the formula (I) in the form of their stereoisomeric forms, which include racemates, racemic mixtures, pure enantiomers and diastereomers and mixtures thereof.

The compounds of the invention may also exist in various polymorphous forms, for example as amorphous and crystalline polymorphous forms.

If radicals or substituents may occur more than once in the compounds of the formula (I), they may all, independently of one another, have the stated meaning and be identical or different.

The terms (C₁-C₂)alkyl, (C₁-C₄)alkyl, (C₁-C₆)alkyl, (C₁-C₈)alkyl and the corresposponding alkylene substituents are understood as a hydrocarbon residue which can be linear, i.e. straight-chain, or branched and has 1, 2, 3, 4, 5, 6, 7 or 8 carbon atoms, respectively. This also applies if an alkyl group occurs as a substituent on another group, for example in an alkoxy group (O-alkyl), S-alkyl or a -O(C₁-C₆)alkylene-O-, an alkoxycarbonyl group or an arylalkyl group. Examples of alkyl groups are methyl, ethyl, propyl, butyl, pentyl or hexyl, the n-isomers of all these groups, isopropyl, isobutyl, 1-methylbutyl, isopentyl, neopentyl, 2,2-dimethylbutyl, 2-methylpentyl, 3-methylpentyl, isohexyl, sec-butyl, tert-butyl or tert-pentyl. Alkyl or alkylene groups may - if not otherwise stated - be halogenated once or more, e.g. alkyl groups may be fluorinated, e.g. perfluorinated. Examples of halogenated alkyl groups are CF₃ and CH₂CF₃, OCF₃, SCF₃, or -O-(CF₂)₂-O-.

Alkenyl are, for example, vinyl, 1-propenyl, 2-propenyl (= allyl), 2-butenyl, 3-butenyl, 2-methyl-2-butenyl, 3-methyl-2-butenyl, 5-hexenyl or 1,3-pentadienyl.

Alkynyl are, for example, ethynyl, 1-propynyl, 2-propynyl (= propargyl) or 2-butynyl.

Halogen means fluoro, chloro, bromo or iodo.

(C₃-C₈)cycloalkyl groups are cyclic alkyl groups containing 3, 4, 5, 6, 7 or 8 ring carbon atoms like cyclopropyl, cyclobutyl, cyclopentyl, cyclohexyl or cyclooctyl, which can also be substituted and/or contain 1 or 2 double bounds (unsaturated cycloalkyl groups) like, for example, cyclopentenyl or cyclohexenyl can be bonded via any carbon atom.

A (C₆-C₁₀)aryl group means an aromatic ring or a ring system which comprises two aromatic rings which are fused or otherwise linked, for example a phenyl, naphthyl, biphenyl, tetrahydronaphthyl, alpha- or beta-tetralon-, indanyl- or indan-1-on-yl group. A preferred (C₆-C₁₀)aryl group is phenyl.

A (C₅-C₁₀)heterocyclyl group means a mono- or bicyclic ring system in which one or more carbon atom can be replaced by one or more heteroatoms such as, for example, e.g. 1, 2 or 3 nitrogen atoms, 1 or 2 oxygen atoms, 1 or 2 sulfur atoms or combinations of different hetero atoms. The heterocyclyl residues can be bound at any positions, for example on the 1-position, 2-position, 3-position, 4-position, 5-position, 6-position, 7-position or 8-position. (C₅-C₁₀)heterocyclyl groups may be (1) aromatic [= heteroaryl groups] or (2) saturated or (3) mixed aromatic/saturated.

Suitable (C₅-C₁₀)heterocyclyl groups include acridinyl, azocinyl, benzimidazolyl, benzofuryl, benzomorpholinyl, benzothienyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, furanyl, quinazolinyl, quinolinyl, 4H-quinolizinyl, quinoxalinyl, quinuclidinyl, chromanyl, chromenyl, chromen-2-onyl, cinnolinyl, decahydroquinolinyl, 2H,6H-1,5,2-dithiazinyl, dihydrofuro[2,3-b]-tetrahydrofuran, furyl, furazanyl, homomorpholinyl, homopiperazinyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperazinyl, piperidinyl, prolinyl, pteridinyl, purynyl, pyranyl, pyrazinyl, pyroazolidinyl, pyrazolinyl, pyrazolyl, pyridazinyl, pyridonyl, pyridooxazoles, pyridoimidazoles, pyridothiazoles, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, 6H-1,2,5-thiadazinyl, thiazolyl, 1,2,3-thiadiazolyl, 1,2,4-thiadiazolyl, 1,2,5-thiadiazolyl, 1 ,3,4-thiadiazolyl, thienyl, triazolyl, tetrazolyl and xanthenyl. Pyridyl stands both for 2-, 3- and 4-pyridyl. Thienyl stands both for 2- and 3-thienyl. Furyl stands both for 2- and 3-furyl. Also included are the corresponding N-oxides of these compounds, for example, 1-oxy-2-, 3- or 4-pyridyl.

Substitutions in (C₅-C₁₀)heterocyclyl residues can occur on free carbon atoms or on nitrogen atoms.

Preferred examples of (C₅-C₁₀)heterocyclyl residues are pyrazinyl, pyridyl, pyrimidinyl, pyrazolyl, morpholinyl, pyrrolidinyl, piperazinyl, piperidinyl, thienyl, benzofuryl, quinolinyl, tetrazolyl and triazolyl. A preferred (C₅-C₁₀)heterocyclyl is (C₅-C₆)heterocyclyl.

(C₆-C₁₀)aryl and (C₅-C₁₀)heterocyclyl groups are unsubstituted or, if not stated otherwise, substituted one or more times, preferably one to three times, by suitable groups independently selected from halogen, OH, NO₂, N₃, CN, C(O)-(C₁-C₆)alkyl, C(O)-(C₁-C₆)aryl, COOH, COO(C₁-C₆)alkyl, CONH₂, CONH(C₁-C₆)alkyl, CON[(C₁-C₆)alkyl]₂, (C₃-C₈)cycloalkyl, (C₁-C₆)alkyl, (C₁-C₆)alkylene-OH, (C₁-C₆)alkylene-NH₂, (C₁-C₆)alkylene-NH(C₁-C₆)alkyl, (C₁-C₆)alkylene-N[(C₁-C₆)alkyl]₂, (C₂-C₆)alkenyl, (C₂-C₆)alkynyl, O-(C₁-C₆)alkyl, O-C(O)-(C₁-C₆)alkyl, PO₃H₂, SO₃H, SO₂-NH₂, SO₂NH(C₁-C₆)alkyl, SO₂N[(C₁-C₆)alkyl]₂, S-(C₁-C₆)alkyl; SO-(C₁-C₆)alkyl, SO₂-(C₁-C₆)alkyl, SO₂-N=CH-N[(C₁-C₆)alkyl]₂, C(NH)(NH₂), NH₂, NH-(C₁-C₆)alkyl, N[(C₁-C₆)alkyl]₂, NH-C(O)-(C₁-C₆)alkyl, NH-C(O)O-(C₁-C₆)alkyl, NH-SO₂-(C₁-C₆)alkyl, NH-SO₂-(C₆-C₁₀)aryl, NH-SO₂-(C₅-C₁₀)heterocyclyl, N(C₁-C₆)alkyl-C(O)-(C₁-C₆)alkyl, N(C₁-C₆)-alkyl-C(O)O-(C₁-C₆)alkyl, N(C₁-C₆)alkyl-C(O)-NH-(C₁-C₆)alkyl], (C₆-C₁₀)aryl, (C₁-C₆)alkylene-(C₆-C₁₀)aryl, O-(C₆-C₁₀)aryl, O-(C₁-C₆)alkylene-(C₆-C₁₀)aryl, (C₅-C₁₀)heterocyclyl, (C₁-C₆)alkylene-(C₅-C₁₀)heterocyclyl, O-(C₁-C₆)alkylene-(C₅-C₁₀)heterocyclyl, wherein the (C₆-C₁₀)aryl or (C₅-C₁₀)heterocyclyl may be substituted one to 3 times by a group independently selected from halogen, OH, NO₂, CN, O-(C₁-C₆)alkyl, (C₁-C₆)alkyl, NH₂, NH(C₁-C₆)alkyl, N[(C₁-C₆)alkyl]₂, SO₂CH₃, COOH, C(O)O-(C₁-C₆)alkyl, CONH₂, (C₁-C₆)alkylene-O-(C₁-C₆)alkyl, (C₁-C₆)alkylene-O-(C₆-C₁₀)aryl, O-(C₁-C₆)alkylene-(C₆-C₁₀)aryl; or wherein (C₆-C₁₀)aryl is vicinally substituted by a O-(C₁-C₄)alkylene-O group whereby a 5-8-membered ring is formed together with the carbon atoms the oxygen atoms are attached to. Aryl or heterocyclyl substituents of (C₆-C₁₀)aryl and (C₅-C₁₀)heterocyclyl groups may not be further substituted by an aryl or heterocyclyl containing group.

Preferred substituents for (C₆-C₁₀)aryl groups are (C₁-C₄)alkyl, O-(C₁-C₄)alkyl, O-phenyl, phenyl, C(O)O-(C₁-C₆)alkyl, C(O)OH, C(O)-(C₁-C₄)alkyl, halogen, NO₂, SO₂NH₂, CN, SO₂-(C₁-C₄)alkyl, SO₂-N=CH-N[(C₁-C₆)alkyl]₂, NH-SO₂-(C₁-C₄)alkyl, NH₂, NH-C(O)-(C₁-C₄)alkyl, (C₃-C₈)cycloalkyl, (C₁-C₄)alkyl-OH, C(O)N[(C₁-C₄)alkyl]₂, CONH(C₁-C₆)alkyl, C(O)NH₂, N[(C₁-C₄)alkyl]₂, (C₁-C₄)alkylene-(C₆-C₁₀)aryl, wherein the (C₆-C₁₀)aryl may be further substituted one to three times, preferabyl once, by (C₁-C₄)alkyl, (C₁-C₄)alkylene-O-(C₁-C₆)alkyl, (C₆-C₁₀)aryl O-(C₁-C₆)alkyl-(C₆-C₁₀)aryl, or may be vicinally substituted by a O-(C₁-C₄)alkylene-O group whereby a 5-8-membered ring is formed together with the carbon atoms the oxygen atoms are attached to. More preferred substituents for (C₆-C₁₀)aryl are halogen, CN, phenyl, O-phenyl, NH-C(O)-(C₁-C₄)alkyl especially NH-C(O)-CH₃, C(O)-(C₁-C₄)alkyl especially C(O)-CH₃, C(O)-O(C₁-C₄)alkyl especially C(O)-OCH₃, (C₁-C₄)alkyl especially CH₃ or CF₃, O-(C₁-C₄)alkyl especially O-CH₃, SO₂-NH₂, SO₂-(C₁-C₄)alkyl especially SO₂-CH₃ or SO₂-CF₃; or SO₂-N=CH-N[(C₁-C₄)alkyl]₂ especially SO₂-N=CH-N[(CH₃)₂.

In monosubstituted phenyl groups the substituent can be located in the 2-position, the 3-position or the 4-position, with the 3-position and the 4-position being preferred. If a phenyl group carries two substituents, they can be located in 2,3-position, 2,4-position, 2,5-position, 2,6-position, 3,4-position or 3,5-position. In phenyl groups carrying three substituents the substituents can be located in 2,3,4-position, 2,3,5-position, 2,3,6-position, 2,4,5-position, 2,4,6-position, or 3,4,5-position.

The above statements relating to phenyl groups correspondingly apply to divalent groups derived from phenyl groups, i.e. phenylene which can be unsubstituted or substituted 1,2-phenylene, 1,3-phenylene or 1,4-phenylene. The above statements also correspondingly apply to the aryl subgroup in arylalkylene groups. Examples of arylalkylene groups which can also be unsubstituted or substituted in the aryl subgroup as well as in the alkylene subgroup, are benzyl, 1-phenylethylene, 2-phenylethylene, 3-phenylpropylene, 4-phenylbutylene, 1-methyl-3-phenyl-propylene.

Preferred substituents for (C₅-C₁₀)heterocyclyl groups are (C₁-C₄)alkyl, O-(C₁-C₄)alkyl, (C₁-C₄)alkylene-phenyl, halogen, (C₁-C₄)alkylene-O-(C₁-C₄)alkyl, (C₅-C₁₀)heterocyclyl, (C₁₋C₄)alkylene-N[(C₁-C₄)alkyl]₂, or (C₆-C₁₀)aryl, wherein the (C₆-C₁₀)aryl may be further substituted by halogen, (C₁-C₄)alkyl, O(C₁-C₄)alkyl, (C₁-C₄)alkylene-O-(C₁-C₆)alkyl, O-(C₁-C₆)alkyl-(C₆-C₁₀)aryl, or may be vicinally substituted by a O-(C₁-C₄)alkylene-O group whereby a 5-8-membered ring is formed together with the carbon atoms the oxygen atoms are attached to. More preferred substituents for (C₅-C₁₀)heterocyclyl groups are (C₁-C₄)alkyl, O(C₁-C₄)alkyl, halogen or phenyl, wherein the phenyl may be further substituted one to three times, preferably once, by halogen, (C₁-C₄)alkyl or O-(C₁-C₄)alkyl.

The general and preferred substituents of (C₆-C₁₀)aryl and (C₅-C₁₀)heterocyclyl groups may be combined with the general and preferred definitions of R₁, R₂, R₃, R₄, R₅, R₆, R₆', R₇, R₈, n, m and L as described above.

The present invention therefore also relates to the compounds of the formula (I) and/or their pharmaceutically acceptable salts and/or their prodrugs for use as pharmaceuticals (or medicaments), to the use of the compounds of the formula (I) and/or their pharmaceutically acceptable salts and/or their prodrugs for the production of pharmaceuticals for the treatment and/or prevention of diseases associated with Rho-kinase and/or Rho-kinase mediated phosphorylation of myosin light chain phosphatase, i.e. for the treatment and/or prevention of hypertension, pulmonary hypertension, ocular hypertension, retinopathy, and glaucoma, peripheral circulatory disorder, peripheral arterial occlusive disease (PAOD), coronary heart disease, angina pectoris, heart hypertrophy, heart failure, ischemic diseases, ischemic organ failure (end organ damage), fibroid lung, fibroid liver, liver failure, nephropathy, including hypertension-induced, non-hypertension-induced, and diabetic nephropathies, renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain, neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, AIDS, osteopathy such as osteoporosis, infection of digestive tracts with bacteria, sepsis, cancer development and progression, e.g. cancers of the breast, colon, prostate, ovaries, brain and lung and their metastases.

The present invention furthermore relates to pharmaceutical preparations (or pharmaceutical compositions) which contain an effective amount of at least one compound of the formula (I) and/or its pharmaceutically acceptable salts and a pharmaceutically acceptable carrier, i. e. one or more pharmaceutically acceptable carrier substances (or vehicles) and/or additives (or excipients).

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carrier substances and/or additives being used in addition to the compound(s) of the formula (I) and/or its (their) pharmaceutically acceptable salts and/or its (their) prodrugs. For the production of pills, tablets, coated tablets and hard gelatin capsules it is possible to use, for example, lactose, corn starch or derivatives thereof, talc, stearic acid or its salts, etc. Carrier substances for soft gelatin capsules and suppositories are, for example, fats, waxes, semisolid and liquid polyols, natural or hardened oils, etc. Suitable carrier substances for the production of solutions, for example injection solutions, or of emulsions or syrups are, for example, water, saline, alcohols, glycerol, polyols, sucrose, invert sugar, glucose, vegetable oils, etc. Suitable carrier substances for microcapsules, implants or rods are, for example, copolymers of glycolic acid and lactic acid. The pharmaceutical preparations normally contain about 0.5 to about 90 % by weight of the compounds of the formula (I) and/or their pharmaceutically acceptable salts and/or their prodrugs. The amount of the active ingredient of the formula (I) and/or its pharmaceutically acceptable salts and/or its prodrugs in the pharmaceutical preparations normally is from about 0.5 to about 1000 mg, preferably from about 1 to about 500 mg.

In addition to the active ingredients of the formula (I) and/or their pharmaceutically acceptable salts and to carrier substances, the pharmaceutical preparations can contain one or more additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formula (I) and/or their pharmaceutically acceptable salts. In case a pharmaceutical preparation contains two or more compounds of the formula (I) the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the compounds of the formula (I) allows a great deal of control over the biological and physico-chemical properties of the compounds and thus allows the selection of such desired compounds. Furthermore, in addition to at least one compound of the formula (I) and/or its pharmaceutically acceptable salts, the pharmaceutical preparations can also contain one or more other therapeutically or prophylactically active ingredients.

When using the compounds of the formula (I) the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from about 0.01 to about 100 mg/kg, preferably from about 0.1 to about 50 mg/kg, in particular from about 0.1 to about 10 mg/kg, (in each case in mg per kg of body weight). The daily dose can be divided, in particular in the case of the administration of relatively large amounts, into several, for example 2, 3 or 4, part administrations. As usual, depending on individual behavior it may be necessary to deviate upwards or downwards from the daily dose indicated.

Furthermore, the compounds of the formula (I) can be used as synthesis intermediates for the preparation of other compounds, in particular of other pharmaceutical active ingredients, which are obtainable from the compounds of the formula I, for example by introduction of substituents or modification of functional groups.

In general, protective groups that may still be present in the products obtained in the coupling reaction are then removed by standard procedures. For example, tert-butyl protecting groups, in particular a tert-butoxycarbonyl group which is a protection form of an amino group, can be deprotected, i. e. converted into the amino group, by treatment with trifluoroacetic acid. As already explained, after the coupling reaction also functional groups can be generated from suitable precursor groups. In addition, a conversion into a pharmaceutically acceptable salt or a prodrug of a compound of the formulae (I) or (I') can then be carried out by known processes.

In general, a reaction mixture containing a final compound of the formula (I) or (I') or an intermediate is worked up and, if desired, the product is then purified by customary processes known to those skilled in the art. For example, a synthesized compound can be purified using well known methods such as crystallization, chromatography or reverse phase-high performance liquid chromatography (RP-HPLC) or other methods of separation based, for example, on the size, charge or hydrophobicity of the compound. Similarly, well known methods such as amino acid sequence analysis, NMR, IR and mass spectrometry (MS) can be used for characterizing a compound of the invention.

Isoquinolines and isoquinolinones can by synthesized via a variety of methods. The following general schemes illustrate some of the possible ways to access isoquinolones, but do not limit the present invention.

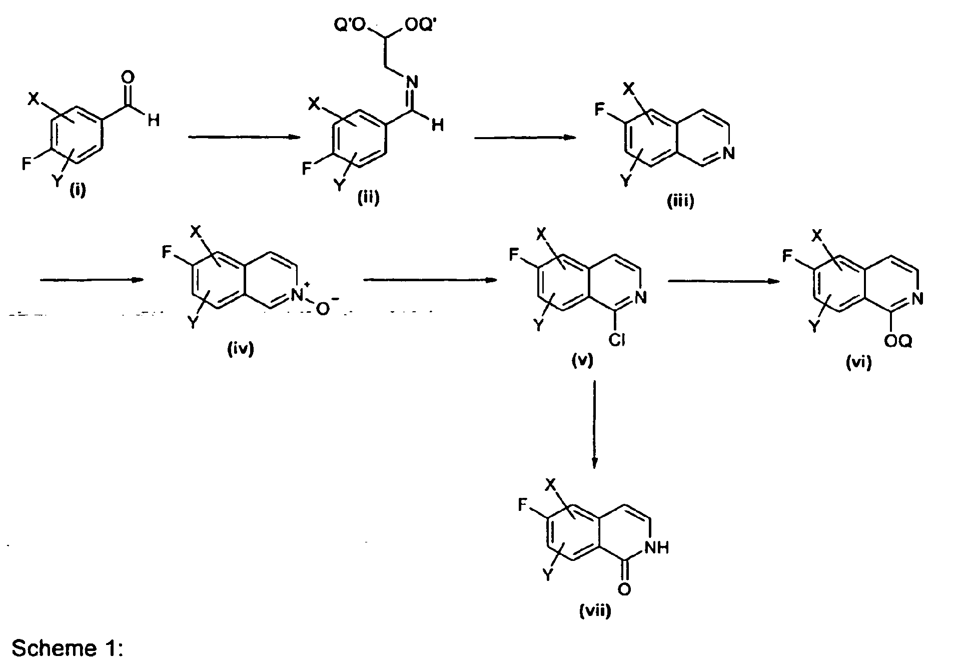

A suitably substituted aldehyde, for example substituted by X or Y being independently from each other hydrogen, alkyl, alkoxy or halide attached in a suitable position, can be reacted with a suitable compound such as for example an actal of aminoacetaldehyde for example in a solvent like THF, chloroform or toluene under acid catalysis by toluene sulfonic acid or another appropriate acid to give imine (ii) wherein Q' can be for instance methyl or ethyl, which in turn can be cyclized by different methods to the isoquinoline (iii). For example this can be done by Lewis acid catalysis by suitable Lewis acids like titanium tetrachloride, ferrous halides, aluminium halides etc. at temperatures ranging from ambient to 100 °C or by reducing the imine to the corresponding amine by action of a suitable reducing agent like sodium borohydride, converting the amine into an amide or sulphonamide by reaction with a suitable acid chloride and subsequent cyclization to the isoquinoline by action of an appropriate lewis acid. The isoquinoline (iii) itself can then be converted to the corresponding N-oxide (iv) by action of a suitable oxidative agent like hydrogen peroxide, m-chloro perbenzoic acid or others at room temperature or elevated temperature. The N-oxide (iv) can then be converted into the 1-chloro-isoquinoline derivative (v) by reacting it with a reagent like phosphorous oxy chloride in or without presence of phosphorous pentachloride. The derivative (v) can then be turned into suitable 1-alkoxy-derivatives by reacting it with various alcohols Q-OH like methanol, ethanol or benzyl alcohol in the presence of a suitable base like sodium hydride and in a suitable solvent like dimethyl formamide, dimethyl acetamide or others. Alternatively (v) can be directly converted into the isoquinolinone derivative (vii) by reacting it with a reagent like ammonium acetate.
Employing suitable bromo derivatives in the described reaction sequences, 6-bromo isoquinolines or bromo isoquinolones can be obtained.

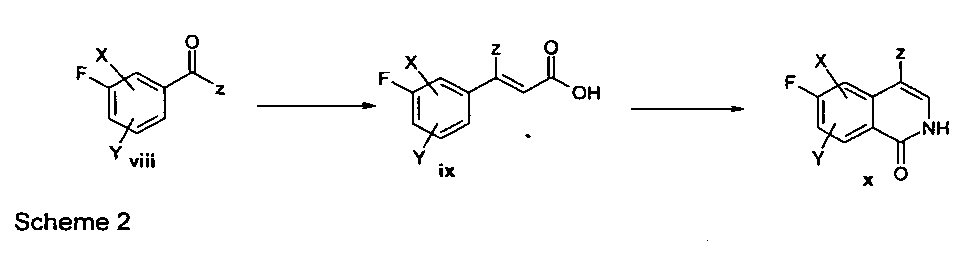

Alternatively isoquinolines can be obtained by reacting suitable 3-formylated or acylated fluorobenzenes (viii), wherein z is for example H or alkyl like methyl or ethyl, with a reagent like triethyl phosphono acetate in the presence of a suitable base like sodium hydride to give the corresponding cinnamic acid ester, which subsequently is cleaved by action of a suitable base like potassium hydroxide, sodium hydroxide or lithium hydroxide in a suitable solvent to deliver acid (ix). (ix) can then be converted in the corresponding acid chloride by well known methods, which can be transferred into the acid azide by reaction with sodium azide in a suitable solvent like ether, chloroform or acetone in or without the presence of water. The corresponding azide then can be converted into isoquinolinone (x) by reacting it in a suitable solvent like diphenylmethane or dipenylether at suitable temperature.

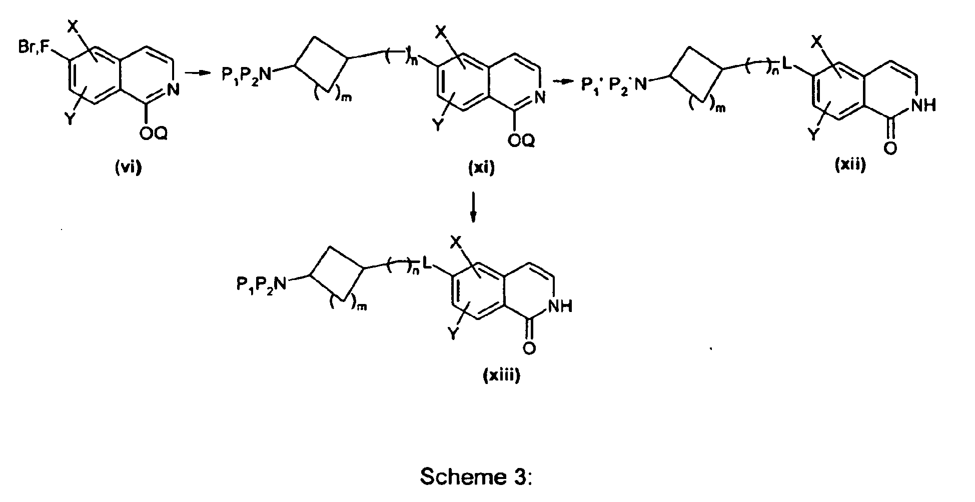

The above obtained 6-fluoro-isoquinolones (or the corresponding isoquinolines iii, alternatively), for example (vi), can be reacted with suitable P₁ / P₂ substituted thiols or amines wherein P₁ / P₂ are independently from each other for example hydrogen, alkyl or a protecting group like for example Boc or phthaloyl in the presence of base such as DBU, cesium carbonate or sodium hydride to give the corresponding alkylthio or alkylamino substituted derivatives (xi). Eventually, this conversion can already be performed at earlier stages of the synthesis (e.g. by reacting a suitable intermediate). It is understood, that this may require in case of unprotected isoquinolones protection on the nitrogen or oxygen of the isoquinolone moiety by suitable methods, like reaction with suitably substituted alkyl or benzyl halides in the presence of base.
In case of amine substitutions, reaction may also be accomplished by reacting a suitable bromo-derivative with the given amine in the presence of a palladium catalyst like palladium acetate, a ligand like e.g. BINAP and a base like cesium carbonate. The products like (xi) obtained via this method can then either be liberated or, if a suitable amino functionality is present, be reacted with suitable aldehydes or ketones in the presence of a reducing agent like sodium triacetoxy borohydride, sodium borohydride or sodium cyanoborohydride in a suitable solvent and in the presence of a water withdrawing agent like molecular sieves or a suitable ortho ester. This amino group may have to be liberated in an initial step like for example acidic removal of Boc-groups. Furthermore an amino group can be acylated by reacting it with a suitable acid chloride in the presence of a base like triethyl amine or Hünig's base or by reacting it with a suitable carboxylic acid in the presence of a base like triethylamine or Hünig's base and a coupling reagent like EDC, PyBOP or TOTU.
In case of use of protected isoquinolones, cleavage of the used protection groups is required to liberate the desired isoquinolone (xii). This liberation, however, can be performed before or after the reductive amination step, depending on the nature of the used aldehyde/ketone and the protection group used.
Isoquinolone derivatives like (xii) can be obtained as free bases or as various salts like for example hydrochlorides, hydrobromides, phosphates, trifluoroacetates, sulfates or fumarates. The salts obtained can be converted into the corresponding free base by either subjecting them to ion exchange chromatography or for example by alkaline aqueous treatment and subsequent extraction with suitable organic solvents like for example methyl tert. butyl ether, chloroform, ethyl acetate or isopropanol / dichloromethane mixtures and subsequent evaporation to dryness.

The general methods for the preparation of isoquinoline derivatives as described above can be readily adapted to the preparation of the compounds of the formula (I). In the following examples the preparation of the compounds of the present invention is outlined in more detail.

Accordingly, the following examples are part of and intended to illustrate but not to limit the present invention:

It is understood that modifications that do not substantially affect the activity of the various embodiments of this invention are included within the invention disclosed herein.

### (4-Bromo-benzyl)-(2,2-dimethoxy-ethyl)-amine (1)

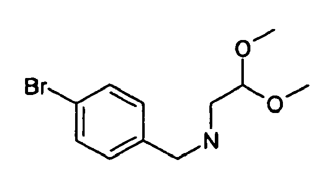

50 g (270.2 mmol) 4-bromobenzaldehyde were dissolved in 200 ml toluene and 28.4 g (270.2 mmol) aminoacetaldehyde dimethylacetal were added. After the addition of 5.1 g (27.0 mmol) p-toluenesulfonic acid monohydrate, the reaction mixture was heated under reflux in a Dean Stark apparatus. After 4 h, the reaction was cooled to room temperature and washed with saturated NaHCO₃-solution (2x) and H₂O. The combined aqueous layers were extracted with toluene and the combined organic layers were dried with MgSO₄ and evaporated. The residue was dissolved in 200 ml ethanol and 5.11 g (135.1 mmol) sodium borohydride were added in small portions. After stirring for 2 h at room temperature and standing overnight, 5.0 ml acetic acid were added and the solvent was removed i. vac. The residue was taken up in dichloromethane and washed (2x) with H₂O. After drying with MgSO₄ and evaporation, 60.5 g of the title compound were obtained (crude product), which were used without further purification. Rₜ = 0.80 min (Method C). Detected mass: 274.1/276.1 (M+H⁺).

### N-(4-Bromo-benzyl)-N-(2,2-dimethoxy-ethyl)-4-methyl-benzenesulfonamide (2)

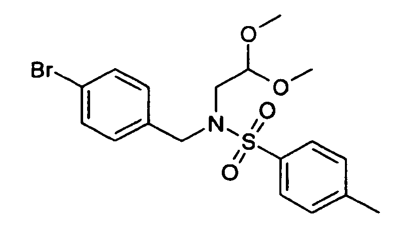

60.5 g (4-bromo-benzyl)-(2,2-dimethoxy-ethyl)-amine (1, crude product) were dissolved in 270 ml dichloromethane/pyridin (8:1). At 0 °C a solution of 76.0 g (386.4 mmol) p-toluenesulfonylchloride in 100 ml dichloromethane were added and the solution was stirred at room temperature. After 3 h, the reaction mixture was washed twice with 2 N HCl and saturated NaHCO₃-solution. The organic layer was dried with MgSO₄ and evaporated. Final silicagel chromatography (heptane/ethylacetate 4:1) gave 59.9 g of the title compound. Rₜ = 1.82 min (Method C). Detected mass: 396.1/398.1 (M-OMe⁻).

### 6-Bromo-isoquinoline (3)

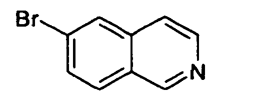

To a mechanically stirred suspension of 95.2 g (699.5 mmol) AlCl₃ in 400 ml dichloromethane a solution of 59.9 g (139.8 mmol) N-(4-Bromo-benzyl)-N-(2,2-dimethoxy-ethyl)-4-methyl-benzenesulfonamide (2) in 400 ml dichloromethane was added and the reaction was stirred at room temperature for 4 h. After standing overnight, the reaction mixture was poured on ice, the organic layer was separated and the aqueous layer was extracted twice with dichloromethane. The combined dichloromethane solutions were washed with 1 N NaOH (2x) and saturated NaHCO₃-solution (2x). After drying with MgSO₄ and evaporation of the solvent, the crude product was purified by silicagel chromatography (heptane/ethyl acetate 1:1) to yield 17.5 g of the title compound. Rₜ = 0.68 min (Method C). Detected mass: 208.1/210.1 (M+H⁺).

### 6-Bromo-isoquinoline-2-oxide (4)

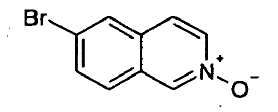

To a solution of 51.0 g (245.1 mmol) 6-bromo-isoquinoline (3) in 800 ml dichloromethane were added under mechanical stirring 90.6 g (367.6 mmol) 3-chloro-benzenecarboperoxoic acid (70%). After stirring for 4 h at room temperature and standing overnight, saturated NaHCO₃-solution was added until two clear layers were obtained. The dichloromethane solution was separated and washed with saturated NaCl-solution. The aqueous layers were extracted with a chloroform/isopropanol (3:1) mixture and the organic layers were combined, washed again with saturated NaCl-solution, dried with MgSO₄ and evaporateed. The obtained crude product (53.0 g) was used without further purification. Rₜ = 0.89 min (Method C). Detected mass: 226.2 (M+H⁺).

### 6-Bromo-1-chloro-isoquinoline (5)

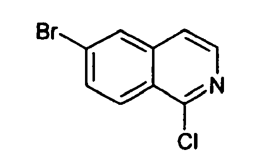

53.0 g (236.5 mmol) 6-bromo-isoquinoline-2-oxide (4) were heated in 400 ml POCl₃ under reflux conditions in two portions. After 4 h, the reaction was cooled to room temperature and poured carefully on ice with mechanical stirring. The aqueous solution was extracted three times with dichloromethane. The combined organic layers were dried with MgSO₄ and evaporated, which gave 42.8 g of the title compound, which was used without further purification. Rₜ = 1.64 min (Method C). Detected mass: 242.1/244.2 (M+H⁺).

### 6-Bromo-2H-isoquinolin-1-one (6)

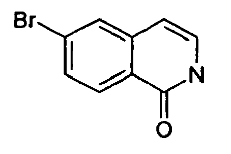

To a solution of 42.8 g (173.5 mmol) 6-bromo-1-chloro-isoquinoline (5) in 700 ml acetic acid were added 133.6 g (1.74 mol) ammonium acetate. After stirring at 100 °C for 3 h, the solution was cooled to room temperature and the solvent was removed i. vac. to a small volume. The residue was poured on H₂O and the suspension was stirred for some minutes. The precipitate was isolated by filtration and dried, to yield 28.2 g of the title compound. Rₜ = 1.30 min (Method B). Detected mass: 224.0 (M+H⁺).

### (4-Bromo-3-chloro-benzyl)-(2,2-dimethoxy-ethyl)-amine (7)

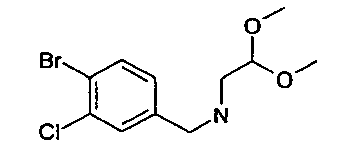

Starting from 4-bromo-3-chloro-benzaldehyde, the title compound was prepared by the methode described for (4-bromo-benzyl)-(2,2-dimethoxy-ethyl)-amine (1). Rₜ = 0.94 min (Method C). Detected mass: 308.3/310.3 (M+H⁺).

### N-(4-Bromo-3-chloro-benzyl)-N-(2,2-dimethoxy-ethyl)-4-methyl-benzene-sulfonamide (8)

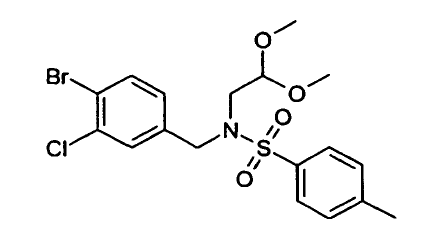

The title compound was prepared by the protocol described for N-(4-bromo-benzyl)-N-(2,2-dimethoxy-ethyl)-4-methyl-benzenesulfonamide (2), starting from (4-bromo-3-chloro-benzyl)-(2,2-dimethoxy-ethyl)-amine (7). Rₜ = 1.93 min (Method C). Detected mass: 430.3/432.3 (M-OMe⁻).

### 6-Bromo-7-chloro-isoquinoline (9)

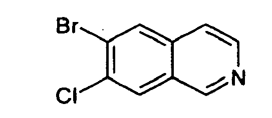

Starting from N-(4-bromo-3-chloro-benzyl)-N-(2,2-dimethoxy-ethyl)-4-methyl-benzenesulfonamide (8), the title compound was synthesized by the protocol described for 6-bromo-isoquinoline (3). Rₜ = 1.02 min (Method C). Detected mass: 242.2/244.2 (M+H⁺).

### 6-Bromo-7-chloro-isoquinoline-2-oxide (10)

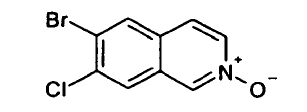

The title compound was prepared by the method described for 6-bromo-isoquinoline 2-oxide (4), starting with 6-bromo-7-chloro-isoquinoline (9). Rₜ = 1.05 min (Method C). Detected mass: 258.1/260.2 (M+H⁺).

### 6-Bromo-1,7-dichloro-isoquinoline (11)

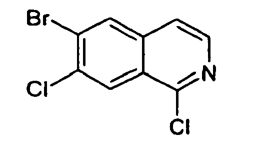

Starting with 6-bromo-7-chloro-isoquinoline-2-oxide (10), the desired 6-bromo-1,7-dichloro-isoquinoline was prepared by the method, described for 6-bromo-1-chloro-isoquinoline (5). Rₜ = 1.85 min (Method C). Detected mass: 276.1/278.2 (M+H⁺).

### 6-Bromo-7-chloro-2H-isoquinolin-1-one (12)

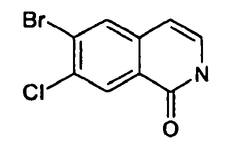

The title compound was prepared by the method, described for 6-bromo-2H-isoquinolin-1-one (6), starting from 6-bromo-1,7-dichloro-isoquinoline (11). Rₜ = 1.26 min (Method C). Detected mass: 258.2/260.2 (M+H⁺).

### 6-Bromo-2-(4-methoxy-benzyl)-2H-isoquinolin-1-one (13)

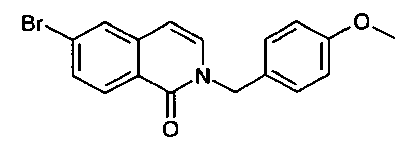

28.18 g (125.8 mmol) 6-bromo-2H-isoquinolin-1-one (6) were dissolved in 200 ml dimethylacetamide and 7.55 g (188.7 mmol) sodium hydride (60%) were added at room temperature. After stirring for 30 minutes, 29.94 g (188.7 mmol) 4-methoxy-benzylchloride were added and stirring was continued at room temperature until complete conversion was detected. The solvent was removed under reduced pressure, the residue taken up in saturated NaHCO₃-solution and extracted three times with dichloromethane. The organic layers were dried with MgSO₄ and evaporated. Final purification was achieved by silicagel chromatography. Rₜ = 1.93 min (Method B). Detected mass: 344.1 (M+H⁺).

### 6-Bromo-7-chloro-2-(4-methoxy-benzyl)-2H-isoquinolin-1-one (14)

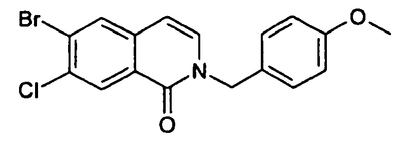

Starting from 6-bromo-7-chloro-2H-isoquinolin-1-one (12), the title compound was prepared by the method described for 6-bromo-2-(4-methoxy-benzyl)-2H-isoquinolin-1-one (13). Rₜ = 2.12 min (Method B). Detected mass: 378.1/380.1 (M+H⁺).

### 1-Benzyloxy-6-bromo-isoquinoline (15)

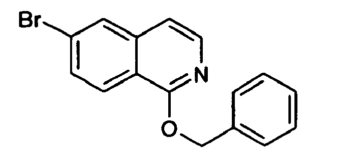

To a solution of 3.93 g (17,5 mmol) 6-bromo-2H-isoquinolin-1-one (6) in 150 ml toluene were added 12.13 g (44.0 mmol) Ag₂CO₃ and 3.60 g (21.1 mmol) benzylbromide. The reaction mixture was refluxed for 1.5 h and then cooled to room temperature. The solution was filtered. The filtrate was washed with H₂O and the aqueous phase extracted with ethyl acetate. The combined organic layers were dried with MgSO₄ and evaporated. Final purification was achieved by preparative HPLC. Rₜ = 2.47 min (Method B). Detected mass: 314.1/316.5 (M+H⁺).

### (4-Fluoro-3-chloro-benzyl)-(2,2-dimethoxy-ethyl)-amine (16)

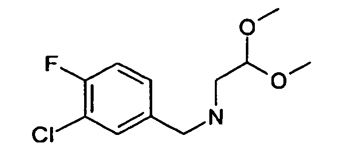

Starting from 4-fluoro-3-chloro-benzaldehyde, the title compound was prepared by the methode described for (4-bromo-benzyl)-(2,2-dimethoxy-ethyl)-amine (1). Rₜ = 0.81 min (Method C). Detected mass: 248.2 (M+H⁺).

### N-(4-Fluoro-3-chloro-benzyl)-N-(2,2-dimethoxy-ethyl)-4-methyl-benzene-sulfonamide (17)

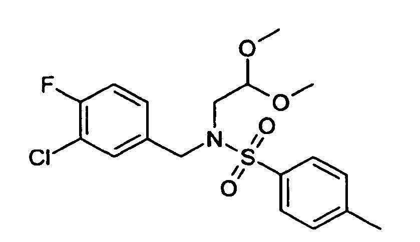

The title compound was prepared by the protocol described for N-(4-bromo-benzyl)-N-(2,2-dimethoxy-ethyl)-4-methyl-benzenesulfonamide (2), starting from (4-fluoro-3-chloro-benzyl)-(2,2-dimethoxy-ethyl)-amine (16). Rₜ = 1.80 min (Method C). Detected mass: 370.2 (M-OMe⁻).

### 6-Fluoro-7-chloro-isoquinoline (18)

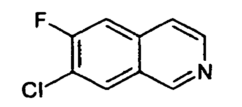

Starting from N-(4-fluoro-3-chloro-benzyl)-N-(2,2-dimethoxy-ethyl)-4-methyl-benzenesulfonamide (17), the title compound was synthesized by the protocol described for 6-bromo-isoquinoline (3). Rₜ = 0.73 min (Method C). Detected mass: 182.1 (M+H⁺).

### 6-Fluoro-7-chloro-isoquinoline-2-oxide (19)

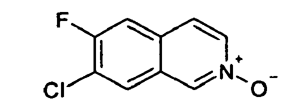

The title compound was prepared by the method, described for 6-bromo-isoquinoline-2-oxide (4), starting with 6-fluoro-7-chloro-isoquinoline (18). Rₜ = 0.87 min (Method C). Detected mass: 198.1 (M+H⁺).

### 6-Fluoro-1,7-dichloro-isoquinoline (20)

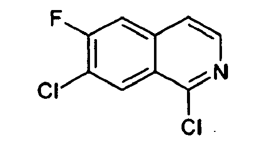

Starting with 6-fluoro-7-chloro-isoquinoline-2-oxide (19), the desired 6-fluoro-1,7-dichloro-isoquinoline was prepared by the method, described for 6-bromo-1-chloro-isoquinoline (5). Rₜ = 2.34 min (Method B). Detected mass: 216.0 (M+H⁺).

### 6-Fluoro-7-chloro-2H-isoquinolin-1-one (21)

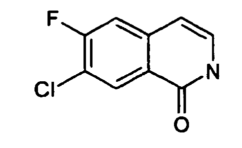

The title compound was prepared by the method, described for 6-bromo-2H-isoquinolin-1-one (6), starting from 6-fluoro-1,7-dichloro-isoquinoline (20). Rₜ = 1.31 min (Method B). Detected mass: 239.1 (M+H⁺+ACN).

### 6-Fluoro-7-chloro-2-(4-methoxy-benzyl)-2H-isoquinolin-1-one (22)

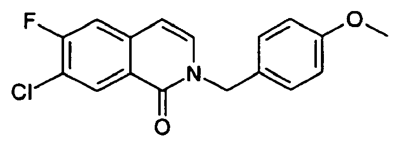

Starting from 6-fluoro-7-chloro-2H-isoquinolin-1-one (21), the title compound was prepared by the method described for 6-bromo-2-(4-methoxy-benzyl)-2H-isoquinolin-1-one (13). Rₜ = 1.94 min (Method B). Detected mass: 318.1 (M+H⁺).

### (4-Fluoro-3-bromo-benzyl)-(2,2-dimethoxy-ethyl)-amine (23)

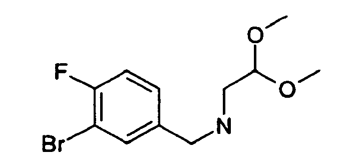

Starting from 4-fluoro-3-bromo-benzaldehyde, the title compound was prepared by the methode described for (4-bromo-benzyl)-(2,2-dimethoxy-ethyl)-amine (1). Rₜ = 1.01 min (Method B). Detected mass: 292.1 (M+H⁺).

### N-(4-Fluoro-3-bromo-benzyl)-N-(2,2-dimethoxy-ethyl)-4-methyl-benzene-sulfonamide (24)

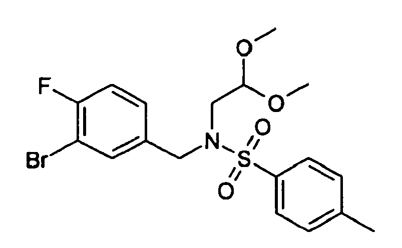

The title compound was prepared by the protocol described for N-(4-bromo-benzyl)-N-(2,2-dimethoxy-ethyl)-4-methyl-benzenesulfonamide (2), starting from (4-fluoro-3-bromo-benzyl)-(2,2-dimethoxy-ethyl)-amine (23). Rₜ = 2.16 min (Method B). Detected mass: 414.1 (M-OMe⁻).

### 6-Fluoro-7-bromo-isoquinoline (25)

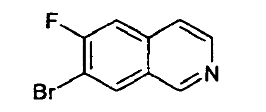

Starting from N-(4-fluoro-3-bromo-benzyl)-N-(2,2-dimethoxy-ethyl)-4-methyl-benzenesulfonamide (24), the title compound was synthesized by the protocol described for 6-bromo-isoquinoline (3). Rₜ = 0.80 min (Method B). Detected mass: 226.0 (M+H⁺).

### General Procedure A for the Hartwig-Buchwald amination reaction:

1.0 eq of the arylbromide, 1.5 eq Cs₂CO₃ and 1.2 eq of the amine were dissolved in toluene. If the amine was taken as a salt another equivalent of Cs₂CO₃ was used; if additionally the arylbromide was used as a salt (HCl- or TFA-salt of the isoquinolines), again, 1 additional equivalent of Cs₂CO₃ was used. The solution was degassed and flushed with argon. Then, 0.03 eq Pd(OAc)₂ and 0.045 eq BINAP were added and the solution was heated at 100 °C until the reaction was complete or no further improvement could be achieved. For product isolation, the solution was cooled to room temperature, filtered and the filtrate was evaporated. The residue was taken up in H₂O and extracted ethyl acetate. The organic layer was separated, dried with MgSO₄ and the solvent was removed i. vac. The crude product was purified by preparative HPLC. The following compounds were synthesized following General Procedure A (Table 1):

**Table 1:**

| Example | Arylbromide | Amine | Product | Chemical Name | Rₜ [min] | Mass [M+H⁺] | LCMS Method |
|---|---|---|---|---|---|---|---|
| 26 | 3 | 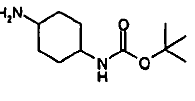 | 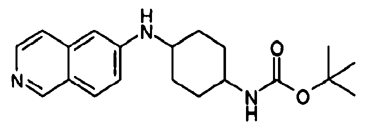 | [4-(Isoquinolin-6-ylamino)-cyclohexyl]-carbamic acid tert-butyl ester | 1.07 | 342.2 | C |
| 27 | 13 | 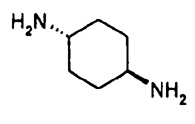 | 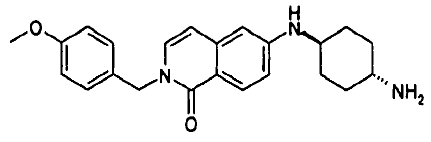 | 6-trans-(4-Aminocyclohexylamino)-2-(4-methoxy-benzyl)-2H-isoquinolin-1-one | 1.01 | 378.3 | C |
| 28 | 13 | 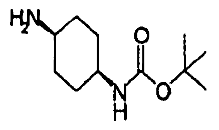 | 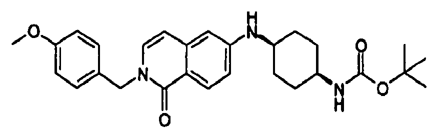 | cis-{4-[2-(4-Methoxybenzyl)-1-oxo-1,2-dihydro-isoquinolin-6-ylamino]-cyclohexyl}-carbamic acid tert-butyl ester | 1.65 | 478.6 | C |
| 29 | 14 | 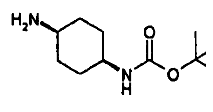 | 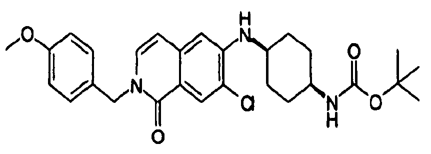 | cis-{4-[7-Chloro-2-(4-methoxy-benzyl)-1-oxo-1,2-dihydro-isoquinolin-6-yl-amino]-cyclohexyl}-carbamic acid tert -butyl ester | 1.86 | 512.4 | C |
| 30 | 14 | 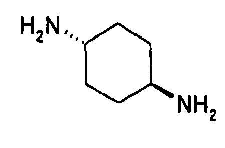 | 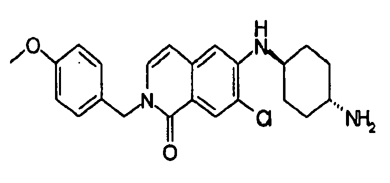 | trans-6-(4-Aminocyclohexylamino)-7-chloro-2-(4-methoxybenzyl)-2H-isoquinol in-1-one | 1.08 | 412.5 | C |
| 31 | 13 | 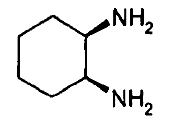 | 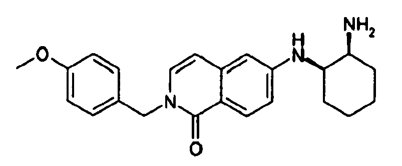 | 6-cis-(2-Amino-cyclohexylamino)-2-(4-methoxy-benzyl)-2H-isoquinolin-1-one | 1.04 | 378.5 | C |
| 32 | 13 | 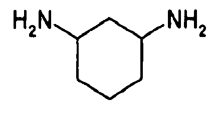 | 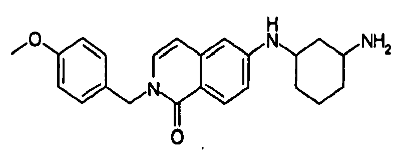 | 6-(3-Amino-cyclohexylamino)-2-(4-methoxy-benzyl)-2H-isoquinolin-1-one | 1.01 | 378.5 | C |
| 33 | 13 | 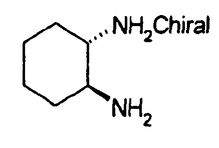 | 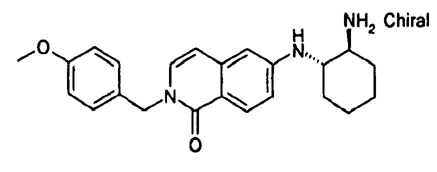 | 6-((1S,2S)-2-Aminocyclohexylamino) -2-(4-methoxy-benzyl)-2H-isoquinolin-1-one | 1.04 | 378.5 | C |
| 34 | 13 | 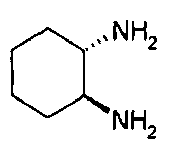 | 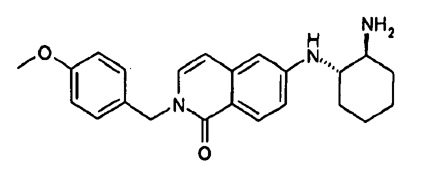 | 6-trans-(2-Aminocyclohexylamino) -2-(4-methoxy-benzyl)-2H-isoquinolin-1-one | 1.01 | 378.5 | C |

### General procedure B for the deprotection of the Boc-group:

The Boc-protected compounds were dissolved in methanol and the same volume of 2 N HCl was added. The solutions were stirred at room temperature until complete conversion was detected. The solvent was removed i. vac. and the residues were dissolved in H₂O. Final lyophilisation gave the desired product as hydrochlorides. The following compounds were synthesized as hydrochlorides following General Procedure B (Table 2):

**Table 2:**

| Example | Prot. comp. | Product | Chemical Name | Rₜ [min] | I Mass [M+H⁺] | LCMS Method |
|---|---|---|---|---|---|---|
| 35 | 26 | 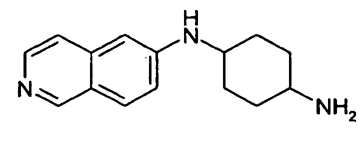 | N-Isoquinolin-6-yl-cyclohexane-1,4-diamine | 0.81 | 242.2 | B |

### General procedure C for the deprotection of PMB-protected compounds:

In the case of PMB- and Boc-protected compounds, the starting materials were dissolved in trifluoro acetic acid and stirred at room temperature for 1 h, followed by 3 h at 140 °C in a microwave. For isolation, the solvent was removed under reduced pressure and the residue was purified by preparative HPLC. The product fractions were combined and evaporated, which gave the desired product as trifluoro acetates, which, in some cases, were dissolved in 2 N HCl and evaporated. After final lyophilisation of the aqueous solutions, the desired products were isolated as hydrochlorides.

The following compounds were synthesized following General Procedure C (Table 3):

| Example | Protected compound | Product | Chemical Name | Rₜ [min] | Mass [M+H⁺] | LC/MS-Method |
|---|---|---|---|---|---|---|
| 36 | 27 | 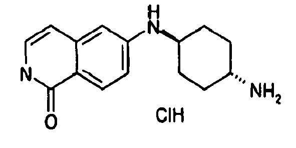 | 6-trans-(4-Amino-cyclohexylamino)-2H-isoquinolin-1-one | 0.85 | 258.2 | B |
| 37 | 28 | 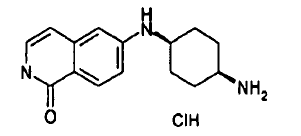 | 6-cis-(4-Amino-cyclohexylamino)-2H-isoquinolin-1-one | 0.90 | 258.2 | B |
| 38 | 29 | 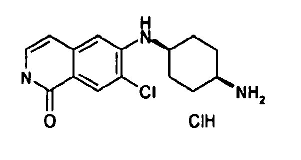 | 6-cis-(4-Amino-cyclohexylamino)-7-chloro-2Hisoquinolin-1-one | 0.88 | 292.1 | B |
| 39 | 30 | 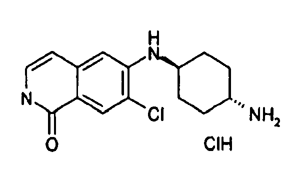 | 6-trans-(4-Amino-cyclohexylamino)-7-chloro-2H-isoquinolin-1-one | 0.86 | 292.2 | B |
| 40 | 31 | 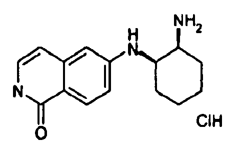 | 6-cis-(2-Amino-cyclohexylamino)-2H-isoquinolin-1-one | 0.87 | 258.2 | B |
| 41 | 32 | 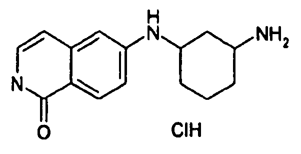 | 6-(3-Amino-cyclohexylamino)-2H-isoquinolin-l-one | 0.80 | 258.2 | B |
| 42 | 33 | 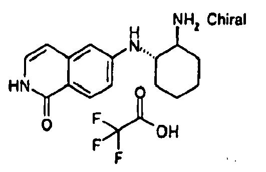 | 6-((1S,2S)-2-Amino-cyclohexylamino)-2H-isoquinolin-1-one | 0.87 | 258.2 | B |
| 43 | 34 | 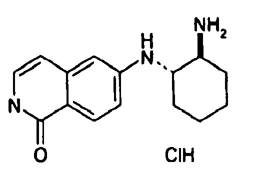 | 6-trans-(2-Amino-cyclohexylamino)-2H-isoquinolin-1-one | 0.83 | 258.2 | B |

### General procedure D for the reductive amination:

The starting material (1.0 eq) was dissolved in dry methanol. After adding freshly dried molecular sieves (4A), triethylamine (2.0 eq), acetic acid (10.0 eq), the carbonyl compounds (3.0 - 6.0 eq) and sodium cyanoborohydride (3.0 eq), the mixture was stirred at room temperature. To achieve complete conversion, in some cases the reaction was heated to 70 °C (bath temperature) or further equivalents of the carbonyl compound and sodium cyanoborohydride were added. For product isolation, the solution was filtered and the filtrate was evaporated. The residue was dissolved in ethyl acetate and washed with 1 N NaOH. The aqueous layer was separated and extracted twice with etyl acetate. The combined organic layers were dried with Na₂SO₄ and the solvent was removed under reduced pressure. The crude products were purified by preparative HPLC. The product fractions were combined and evaporated, which gave the desired product as trifluoro acetates, which were dissolved in 2 N HCl and evaporated. After final lyophilisation of the aqueous solutions, the desired products were isolated as hydrochlorides. The following compounds were synthesized as hydrochlorides following General Procedure D (Table 4):

**Table 4:**

| Example | Starting material | Carbonyl compound | Product | Chemical Name | Rₜ [min] | Mass [M+H⁺] | LC/ MS-Met. | Remark |
|---|---|---|---|---|---|---|---|---|
| 44 | 37 | 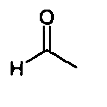 | 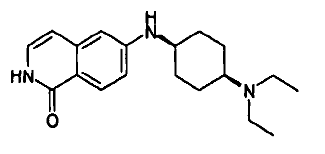 | 6-cis-(4-Diethylaminocyclohexylamino)-2H-isoquinolin-1-one | 0.97 | 314.2 | B | 6.0 eq acetaldehyde were used |
| 45 | 37 | 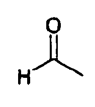 | 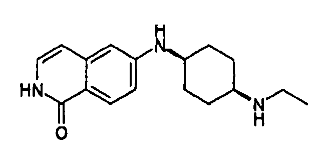 | 6-cis-(4-ethylaminocyclohexylamino )-2H-isoquinolin-1-one | 0.78 | 286.1 | B | 3.0 eq acetaldehyde were used |
| 46 | 37 | 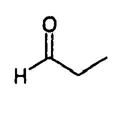 | 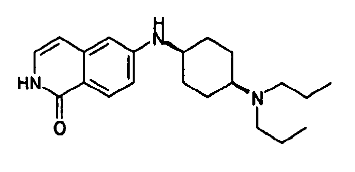 | 6-cis-(4-Dipropyl-aminocyclohexylamino)-2H-isoquinolin-1-one | 1.07 | 342.2 | B | 5.0 eq propionaldehyde were used |
| 47 | 37 | 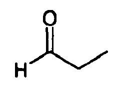 | 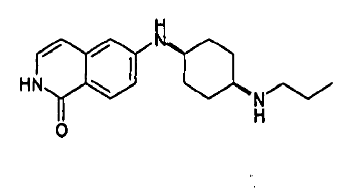 | 6-cis-(4-Propylaminocyclohexylamino)-2H-isoquinolin-1-one | 0.90 | 300.3 | A | 3.0 eq propionaldehyde were used |
| 48 | 37 | 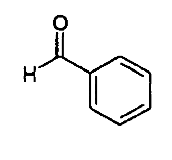 | 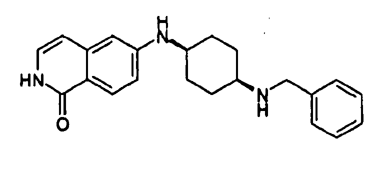 | 6-cis-(4-Benzylaminocyclohexylamino)-2H-isoquinolin-1-one | 1.11 | 348.3 | B | 1.0 eq benzaldehyde was used |
| 49 | 37 | 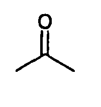 | 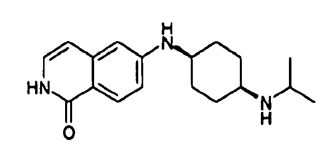 | 6-cis-(4-Isopropylaminocyclohexylamino)-2H-isoquinolin-1-one | 0.88 | 300.3 | B | 1.0 + 5.0 eq acetone were used |
| 50 | 37 | 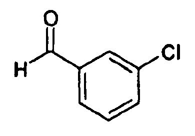 | 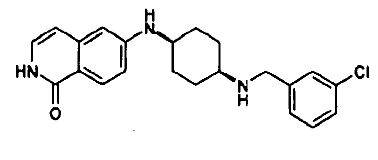 | 6-cis-[4-(3-Chlorobenzylamino)-cyclohexylamino]-2H-isoquinolin-1-one | 1.27 | 382.4 | B | 1.0 + 3.0 eq 3-chlorobenzaldehyde was used |
| 51 | 37 | 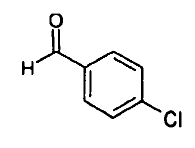 | 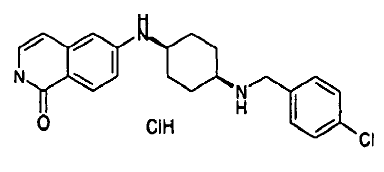 | 6-cis-[4-(4-Chlorobenzylamino)-cyclohexylamino]-2H-isoquinolin-1-one | 1.27 | 382.4 | B | 1.0 + 3.0 eq 4-chlorobenzaldehyde was used |
| 52 | 37 | 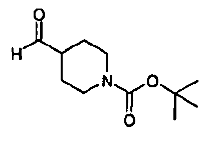 | 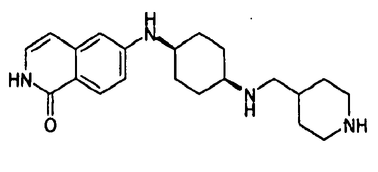 | 6-cis-{4-[(Piperidin-4-ylmethyl)-amino] -cyclohexylamino}-2H-isoquinolin-1-one | 0.72 | 355.3 | B | 1.0 eq aldehyde was used. Boc-deprotection was achieved by stirring in 2 HCl before final lyophilisati on. |
| 53 | 37 | 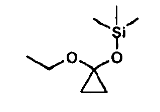 | 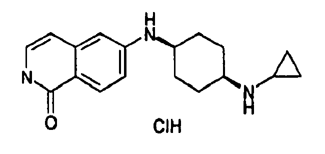 | 6-cis-(4-Cyclopropylamino-cyclohexylamino)-2H-isoquinolin-1-one | 0.97 | 298.2 | B | 1.0+1.0+0. 5 eq "carbonyl compound" was used. |
| 54 | 37 | 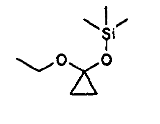 | 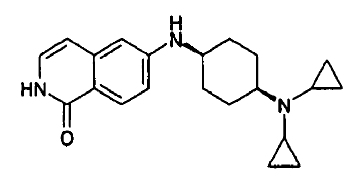 | 6-cis-(4-Dicyclopropyl-aminocyclohexylamino)-2H-isoquinolin-1-one | 0.94 | 338.2 | B | 1.0+1.0+0. 5 eq ^{a}carbonyl compound" was used. Side product from Ex. 53. |
| 55 | 38 | 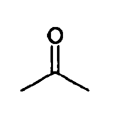 | 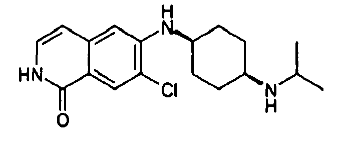 | 7-Chloro-6-cis-(4-isopropylamino-cyclohexylamino)-2H-isoquinolin-1-one | 0.97 | 334.1 | B | 1.25 + 1.25 eq acetone were used. |
| 56 | 38 | 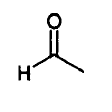 | 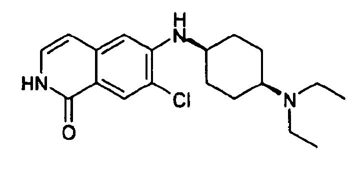 | 7-Chloro-6-cis-(4-diethylaminocyclohexylamino)-2H-isoquinolin-1-one | 1.00 | 348.2 | B | 12.0 eq acetaldehyde were used. |
| 57 | 39 | 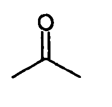 | 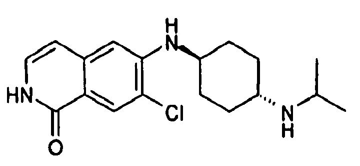 | 7-Chloro-6-trans-(4-isopropylaminocyclohexylamino)-2H-isoquinolin-1-one | 0.98 | 334.2 | B | 2.5 + 1.25 eq acetone were used. |

### trans-2-(4-Hydroxy-cyclohexyl)-isoindole-1,3-dione (58)

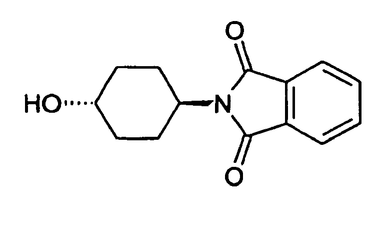

5 g (33 mmol) trans-4-aminocyclohexanol, 4.88 g phthalic anhydride and 7.85 ml tributylamin were stirred at 150°C for 10h. The mixture was dissolved in dichloromethane and washed with 1 N HCl and saturated sodium hydrogen carbonate solution. The organic phase was dried with Na₂SO₄ and evaporated. Purification over silica gel (50% ethyl acetate in heptane) gave 4.8 g of the expected compound.

### Reference example :

### cis-Thioacetic acid 4-(1,3-dioxo-1,3-dihydro-isoindol-2-yl)-cyclohexyl ester (59)

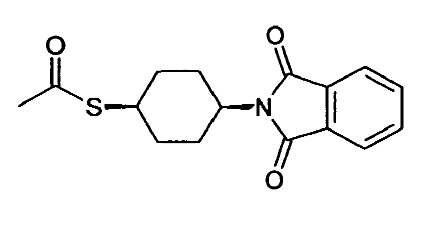

3 g (12.2 mmol) trans-2-(4-Hydroxy-cyclohexyl)-isoindole-1,3-dione (58) and 3.51 g (13.1 mmol) triphenylphosphine were dissolved in 55 ml of dry THF. At 0°C 2 ml (12.2 mmol) diethylazodicarboxylate (15 min) and thereafter 873 µl (12.2 mmol) thioacetic acid were added and stirring was continued over night at RT. After evaporation of all volatiles the residue was purified over silica gel (2% to 10 % ethyl acetate in heptane) to give 2.56 g of the expected compound.

### Reference example:

### cis-2-[4-(7-Chloro-isoquinolin-6-ylsulfanyl)-cyclohexyl]-isoindole-1,3-dione (60)

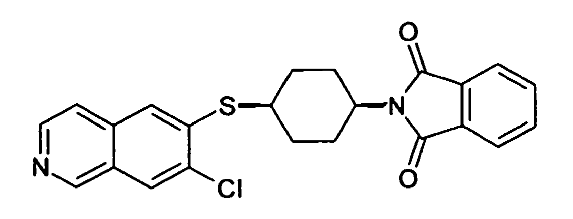

454 mg (1.5 mmol) cis-thioacetic acid 4-(1,3-dioxo-1,3-dihydro-isoindol-2-yl)-cyclohexyl ester (59) and 273 mg (1.5 mmol) 6-fluoro-7-chloro-isoquinoline (18) were dissolved in 5 ml degassed DMF. After addition of 675 µl (3 mmol) DBU the mixture was heated to 78°C for 7 h. The mixture was taken up in ethyl acetate and washed with brine. The organic phases were dried with Na₂SO₄ and evaporated. Purification over silica gel (30% to 50% ethyl acetate in heptane) gave 220 mg of the expected compound.

### Reference example :

### cis-4-(7-Ohloro-isoquinolin-6-ylsulfanyl)-cyclohexylamine (61)

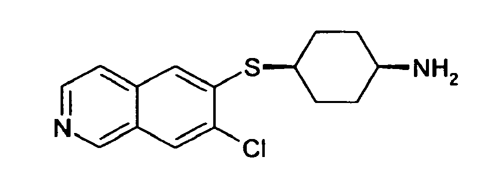

220 mg (0.52 mmol) cis-2-[4-(7-chloro-isoquinolin-6-ylsulfanyl)-cyclohexyl]-isoindole-1,3-dione (60) in 5 ml methanol were treated with 250 µl hydrazine hydrate at RT until complete conversion was achieved. After evaporation, the residue was purified by HPLC. 131 mg of cis-4-(7-chloro-isoquinolin-6-ylsulfanyl)-cyclohexylamine could be obtained as the trifluoroacetate. The obtained trifluoroacetate was dissolved in 2 N HCl. Final lyophilization gave 95 mg of the title compound as hydrochloride. Rₜ = 0.82 min (Method B). Detected mass: 293.1/295.1 (M+H⁺).

### Reference example

### cis-4-(7-Bromo-isoquinolin-6-ylsulfanyl)-cyclohexylamine hydrochloride (62)

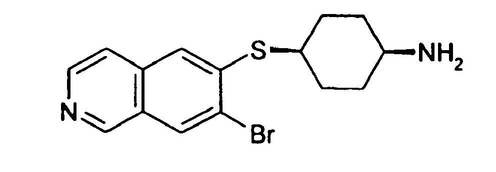

Starting from 6-fluoro-7-bromoisoquinoline (25) the title compound could be obtained as hydrochloride by the same reaction sequence as described for cis-4-(7-chloro-isoquinolin-6-ylsulfanyl)-cyclohexylamine (61) via its phthaloyl protected intermediate. Rₜ = 0.81 min (Method B). Detected mass: 337.1 (M+H⁺).

### Reference example :

### 6-cis-(4-Amino-cyclohexylsulfanyl)-7-chloro-2-(4-methoxy-benzyl)-2H-isoquinolin-1-one (63)

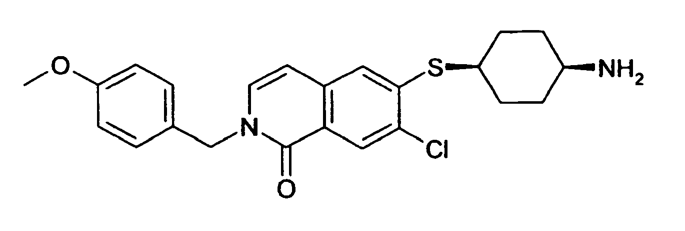

Starting from 7-chloro-6-fluoro-2-(4-methoxy-benzyl)-2H-isoquinolin-1-one (22) the title compound could be obtained as trifluoroacetate by the same reaction sequence as described for cis-4-(7-chloro-isoquinolin-6-ylsulfanyl)-cyclohexylamine (61) via its phthaloyl protected intermediate.

### Reference example:

### 6-cis-(4-Amino-cyclohexylsulfanyl)-7-chloro-2H-isoquinolin-1-one (64)

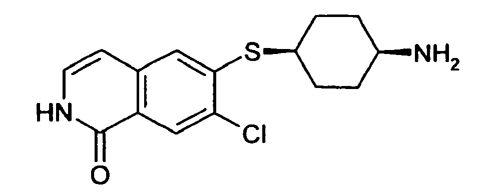

34 mg (0.08 mmol) 6-cis-(4-Amino-cyclohexylsulfanyl)-7-chloro-2-(4-methoxy-benzyl)-2H-isoquinolin-1-one (63) were dissolved in 2 ml TFA and stirred in the microwave at 150°C for 1.5 h. After evaporation the residue was taken up in 1N HCl and extracted with dicloromethan and lyophilized. 11 mg of the desired product could be obtained as hydrochloride. Rₜ = 0.97 min (Method B). Detected mass: 309.1/311.1 (M+H⁺).

### LC/MS-Methods:

| Method A: | |
|---|---|
| Stationary phase: | Col YMC Jsphere 33 x 2 |
| Gradient: | ACN+0.05% TFA : H₂O+0.05% TFA 5:95(0 min) to 95:5(3.4 min) to 95:5(4.4 min) |
| Flow | 1 ml/min |
| | |

| Method B: | |
|---|---|
| Stationary phase: | Col YMC Jsphere 33 x 2 |
| Gradient: | ACN+0.05% TFA : H₂O+0.05% TFA 5:95(0 min) to 95:5(2.5 min) to 95:5(3.0 min) |
| Flow: | 1 ml/min |
| | |

| Method C: | |
|---|---|
| Stationary phase: | Col YMC Jsphere ODS H80 20 x 2 |
| Gradient: | ACN : H₂O+0-05% TFA 4:96(0 min) to 95:5(2.0 min) to 95:5(2.4 min) |
| Flow | 1 ml/min |

### Determination of Rho kinase inhibition

To measure Rho-kinase inhibition, IC₅₀ values were determined according to the following protocol:

Active human recombinant ROCK II (N-terminal His6-tagged recombinant human ROCK-II residues 11-552) was purchased from Upstate Ltd., Dundee, UK. The peptide substrate, Fluorescein-AKRRRLSSLRA-COOH, was obtained from JPT Peptide Technologies, Berlin, Germany. Adenosine-5'-triphosphate (ATP), bovine serum albumine (BSA), dimethylsulphoxide (DMSO), 4-(2-Hydroxyethyl)piperazine-1-ethanesulfonic acid (Hepes), Brij-35 and dithiothreitol (DTT) were purchased from Sigma-Aldrich, Munich, Germany. Tris(hydroxymethyl)-aminomethane (Tris), magnesium chloride, NaOH, 1 M HCl and EDTA were obtained from Merck Biosciences, Darmstadt, Germany. "Complete" protease inhibitor was from Roche Diagnostics, Mannheim, Germany.
Test compounds were diluted to the appropriate concentrations in buffer 1 (25 mM Tris-HCl, pH 7.4, 5 mM MgCl₂, 2 mM DTT, 0.02 % (w/v) BSA and 3 % DMSO). The ROCK II enzyme was diluted to a concentration of 100 ng/ml in buffer 2 (25 mM Tris-HCl, pH 7.4, 5 mM MgCl₂, 2 mM DTT and 0.02 % (w/v) BSA). The peptide substrate and ATP were diluted to concentrations of 3 µM and 120 µM, respectively, in the buffer 2. Two µl of the compound solution were mixed with 2 µl of the diluted enzyme in a 384-well small volume microtiter plate (Greiner, Bio-One, Frickenhausen, Germany), and the kinase reaction was initiated by addition of 2 µl of the solution containing peptide substrate and ATP. After 60 min incubation at 32 °C, the reaction was stopped by addition of 20 µl of a solution containing 100 mM Hepes-NaOH, pH 7.4, 0.015 % (v/v) Brij-35, 45 mM EDTA and 0.227 % chip coating reagent 1 (Caliper Lifescience Inc, Hopkinton, MA). Phosphorylation of the substrate peptide was then detected on a Caliper 3000 instrument essentially as described by Pommereau et al (J. Biomol. Screening 9(5), 409-416, 2004). Separation conditions were as follows: Pressure -1.3 psi, upstream voltage -1562 V, downstream voltage -500 V, sample sip time 200 ms. Positive controls (buffer 1 instead of compound) and negative controls (buffer 1 instead of compound and buffer 2 instead of ROCK II) were run in parallel on each plate.

The following products/compounds were tested in said assay by using the respective form (salt or free base) obtained as in the examples described above and the following activities were measured.

| Compound No. | pIC50 |
|---|---|
| 53 | +++++ |
| 54 | +++++ |
| 52 | +++++ |
| 51 | ++++ |
| 50 | ++++ |
| 48 | ++++ |
| 47 | +++++ |
| 49 | ++++ |
| 45 | +++++ |
| 44 | ++++ |
| 46 | ++++ |
| 40 | +++++ |
| 41 | +++++ |
| 36 | +++++ |
| 61 | +++++ |
| 62 | +++++ |
| 64 | +++++ |

The given activity is denoted as the negative decadal logarithm of the IC₅₀ (pIC₅₀) as follows:

| | | |
|---|---|---|
| +: | pIC50 | ≤ 3.0 |
| ++: | 3.0 ≤ pIC₅₀ | < 4.0 |
| +++ | 4.0 ≤ pIC₅₀ | < 5.0 |
| ++++: | 5.0 ≤ pIC₅₀ | < 6.0 |
| +++++ | 6.0 ≤ pIC₅₀ | |

## Claims

1. A compound of the formula (I) 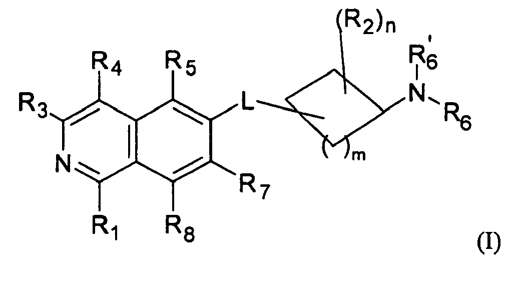 wherein
R₁ is H, OH or NH₂;
R₂ is H, halogen or (C₁-C₆)alkyl;
R₃ is
H,
halogen,
(C₁-C₆)alkyl,
(C₁-C₆)alkylene-R',
OH,
O-R",
NH2,
NHR",
NR"R" or
NH-C(O)-R";
R₄ is
H,
halogen,
hydroxy,
CN,
(C₁-C₆)alkyl,
R',
(C₁-C₆)alkylene-R';
R₅ is
H,
halogen,
CN,
NO₂,
(C₁-C₆)alkyl,
(C₂-C₆)alkenyl,
R',
(C₁-C₆)alkylene-(C₆-C₁₀)aryl,
(C₂-C₆)alkenylene-(C₆-C₁₀)aryl,
(C₁-C₆)alkylene-(C₅-C₁₀)heterocyclyl,
CH(OH)-(C₁-C₆)alkyl,
NH₂,
NH-R',
NH-SO₂H,
NH-SO₂-(C₁-C₆)alkyl,
NH-SO₂-R',
NH-C(O)-(C₁-C₆)alkyl,
NH-C(O)-R',
C(O)N[(C₁-C₆)alkyl]₂,
C(O)OH, or
C(O)O-(C₁-C₆)alkyl;
R₆ and R₆' are independently of each other
H,
R',
(C₁-C₈)alkyl,
(C₁-C₆)alkylene-R',
(C₁-C₆)alkylene-O-(C₁-C₆)alkyl,
(C₁-C₆)alkylene-O-R',
(C₁-C₆)alkylene-CH[R']₂,
(C₁-C₆)alkylene-C(O)-R',
(C₁-C₆)alkylene-C(O)NH₂,
(C₁-C₆)alkylene-C(O)NH-R',
(C₁-C₆)alkylene-C(O)NH-(C₁-C₆)alkyl,
(C₁-C₆)alkylene-C(O)N[(C₁-C₆)alkyl]₂,
(C₁-C₆)alkylene-C(O)N[R']2;
(C₁-C₆)alkylene-C(O)O-(C₁-C₆)alkyl,
C(O)O-(C₁-C₆)alkyl,
C(O)OR'
C(O)(C₁-C₆)alkyl,
C(O)R',
C(O)NH-(C₁-C₆)alkyl,
C(O)NHR',
C(O)N[(C₁-C₆)alkyl]R'
C(O)N[(C₁-C₆)alkyl]₂,
C(O)-(C₁-C₆)alkylene-R',
C(O)O(C₁-C₆)alkylene-R', or
R₆ and R₆', together with the N-atom to which they are attached, form a (C₅-C₁₀) heterocyclyl group;
R₇ is
H,
halogen,
CN,
N02,
(C₁-C₆)alkyl,
O-(C₁-C₆)alkyl.
(C₂-C₆)alkenyl,
R',
(C₂-C₆)alkenylene-(C₆-C₁₀)aryl,
(C₁-C₆)alkylene-R',
CH(OH)-(C₁-C₆)alkyl,
NH2,
NH-R',
NH-SO₂H,
NH-SO₂-(C₁-C₆)alkyl,
NH-SO₂-R',
SO₂-NH₂,
SO₂-NHR',
NH-C(O)-(C₁-C₆)alkyl,
NH-C(O)-R',
C(O)N[(C₁-C₆)alkyl]₂,
C(O)OH, or
C(O)O-(C₁-C₆)alkyl;
R₈ is H, halogen or (C₁-C₆)alkyl;
n is 1, 2, 3 or 4;
m is 1, 2, 3, 4 or 5; and
L is NH(CH₂)p or N(C₁-C₆)alkyl-(CH₂)p;
p is 0, 1, 2, 3 or 4;
wherein
R' is
(C₃-C₈)cycloalkyl,
(C₅-C₁₀)heterocyclyl,
(C₆-C₁₀)aryl; and
R" is
(C₃-C₈)cycloalkyl,
(C₅-C₁₀)heterocyclyl,
(C₆-C₁₀)aryl,
(C₁-C₆)alkyl,
(C₁-C₆)alkylene-R',
(C₁-C₆)alkylene-O-(C₁-C₆)alkyl,
(C₁-C₆)alkylene-O-R', or
(C₁-C₆)alkylene-NRₓR_{y}; and
wherein Rₓ and R_{y} are independently of each other
(C₁-C₆)alkyl,
(C₅-C₁₀)heterocyclyl,
(C₆-C₁₀)aryl,
(C₁-C₄)alkylene-(C₅-C₁₀)heterocyclyl,
(C₁-C₄)alkylene-(C₆-C₁₀)aryl,
(C₁-C₄)alkylene-NH(C₁-C₆)alkyl,
(C₁-C₄)alkylene-N[(C₁-C₆)alkyl]₂,
(C₁-C₄)alkylene-N[(C₆-C₁₀)aryl]₂, or
(C₁-C₄)alkylene-N[(C₅-C₁₀)heterocyclyl]₂;
wherein in residues R₄, R₅, R₆, R₆', R₇ and R₈ alkyl, alkylene or cycloalkyl can optionally be substituted one or more times by OH, OCH₃, COOH, COOCH₃, NH₂, NHCH₃, N(CH₃)₂, CONH₂, CONHCH₃ or CON(CH₃)₂;
wherein in residues R₂ to R₈ alkyl or alkylene can optionally be substituted one or more times by halogen;
wherein in residues R₃ to R₈ (C₆-C₁₀)aryl and (C₅-C₁₀)heterocyclyl are unsubstituted or substituted one or more times by suitable groups independently selected from halogen, OH, NO₂, N₃, CN, C(O)-(C₁-C₆)alkyl, C(O)-(C₆-C₁₀)aryl, COOH, COO(C₁-C₆)alkyl, CONH₂, CONH(C₁-C₆)alkyl, CON[(C₁-C₆)alkyl]₂, (C₃-C₈)cycloalkyl, (C₁-C₆)alkyl, (C₁-C₆)alkylene-OH, (C₁-C₆)alkylene-NH₂, (C₁-C₆)alkylene-NH(C₁-C₆)alkyl, (C₁-C₆)alkylene-N[(C₁-C₆)alkyl]₂, (C₂-C₆)alkenyl, (C₂-C₆)alkynyl, O-(C₁-C₆)alkyl, O-C(O)-(C₁-C₆)alkyl, PO₃H₂, SO₃H, SO₂-NH₂, SO₂NH(C₁-C₆)alkyl, SO₂N[(C₁-C₆)alkyl]₂, S-(C₁-C₆)alkyl; SO-(C₁-C₆)alkyl, SO₂-(C₁-C₆)alkyl, SO₂-N=CH-N[(C₁-C₆)alkyl]₂,
C(NH)(NH₂), NH₂, NH-(C₁-C₆)alkyl, N[(C₁-C₆)alkyl]₂, NH-C(O)-(C₁-C₆)alkyl, NH-C(O)O-(C₁-C₆)alkyl,
NH-SO₂-(C₁-C₆)alkyl, NH-SO₂-(C₆-C₁₀)aryl, NH-SO₂-(C₅-C₁₀)heterocyclyl, N(C₁-C₆)alkyl-C(O)-(C₁-C₆)alkyl, N(C₁-C₆)alkyl-C(O)O-(C₁-C₆)alkyl, N(C₁-C₆)alkyl-C(O)-NH-(C₁-C₆)alkyl],
(C₆-C₁₀)aryl, (C₁-C₆)alkylene-(C₆-C₁₀)aryl, O-(C₆-C₁₀)aryl, O-(C₁-C₆)alkylene-(C₆-C₁₀)aryl, (C₅-C₁₀)heterocyclyl,
(C₁-C₆)alkylene-(C₅-C₁₀)heterocyclyl, O-(C₁-C₆)alkylene-(C₅-C₁₀)heterocyclyl, wherein the (C₆-C₁₀)aryl or (C₅-C₁₀)heterocyclyl may be substituted one to three times by a group independently selected from halogen, OH, NO₂, CN, O-(C₁-C₆)alkyl, (C₁-C₆)alkyl, NH₂, NH(C₁-C₆)alkyl, N[(C₁-C₆)alkyl]₂, SO₂CH₃, COOH, C(O)O-(C₁-C₆)alkyl, CONH₂, (C₁-C₆)alkylene-O-(C₁-C₆)alkyl, (C₁-C₆)alkylene-O-(C₆-C₁₀)aryl, or O-(C₁-C₆)alkylene-(C₆-C₁₀)aryl;
or wherein (C₆-C₁₀)aryl is vicinally substituted by a O-(C₁-C₄)alkylene-O group whereby a 5-8-membered ring is formed together with the carbon atoms the oxygen atoms are attached to;
and wherein aryl or heterocyclyl substituents of (C₆-C₁₀)aryl and (C₅-C₁₀)heterocyclyl groups may not be further substituted by an aryl or heterocyclyl containing group;
or their stereoisomeric and/or tautomeric forms and/or their pharmaceutically acceptable salts.

2. A compound of formula (I) according to claim 1, wherein R₁ is H and is **characterized by** the formula (II)

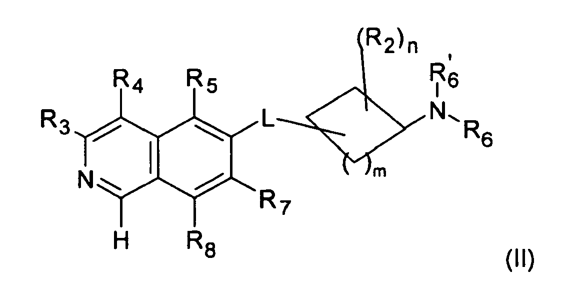

3. A compound of formula (I) according to claim 1, wherein R₁ is OH and is **characterized by** the formula (III)

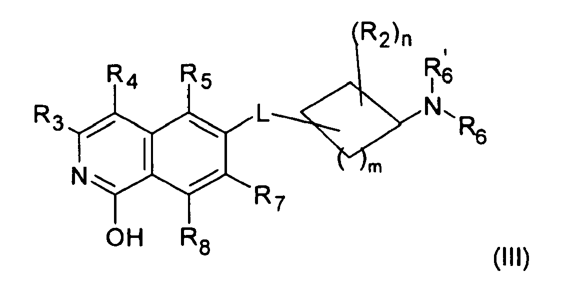

4. A compound of formula (I) according to claim 1 or claim 3, wherein R₁ is OH and is **characterized by** the formula (III')

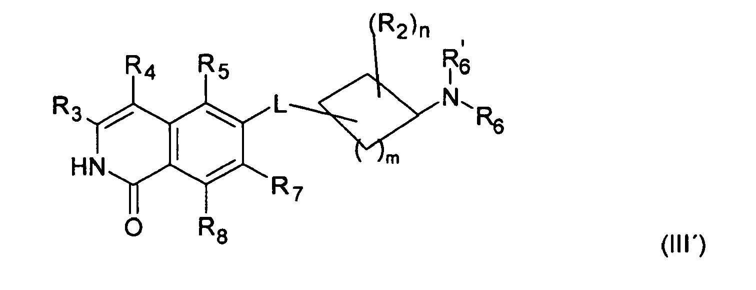

5. A compound according to claim 1, wherein R₁ is NH_{2.}

6. A compound according to one of claims 1 to 5, wherein R₃ is H, halogen, (C₁-C₄)alkylene-R', O-R" or NHR".

7. A compound according to one of claims 1 to 6, wherein R₃ is H or NHR".

8. A compound according to one of claims 1 to 7, wherein R₃ is H; NH-(C₅-C₆)heterocyclyl, or NH-phenyl.

9. A compound according to one of claims 1 to 8, wherein R₃ is H.

10. A compound according to one of claims 1 to 9, wherein R₈ is H, halogen or (C₁-C₄)alkyl.

11. A compound according to one of claims 1 to 10, wherein R₈ is H, Cl, F, methyl or ethyl.

12. A compound according to one of claims 1 to 11, wherein R₈ is H.

13. A compound according to one of claims 1 to 12, wherein R₄ is H, halogen or (C₁-C₆)alkyl.

14. A compound according to one of claims 1 to 13, wherein R₄ is H, halogen or (C₁-C₄)alkyl.

15. A compound according to one of claims 1 to 14, wherein R₄ is H.

16. A compound according to one of claims 1 to 15, wherein R₅ is H, halogen, CN, (C₁-C₆)alkyl, (C₂-C₆)alkenyl, R', NH-(C₆-C₁₀)aryl, (C₁-C₆)alkylene-(C₆-C₁₀)aryl or (C₁-C₆)alkylene-(C₅-C10)heterocyclyl.

17. A compound according to one of claims 1 to 16, wherein R₅ is H, halogen, (C₁-C₆)alkyl, (C₂-C₆)alkenyl, R', NH-(C₆-C₁₀)aryl, (C₁-C₆)alkylene(C₆-C₁₀)aryl or (C₁-C₆)alkylene-(C₅-C₁₀)heterocyclyl.

18. A compound according to one of claims 1 to 17, wherein R₅ is H, halogen, (C₁-C₆)alkyl, (C₂-C₆)alkenyl, (C₆-C₁₀)aryl, NH-(C₆-C₁₀)aryl, (C₁-C₂)alkyl-(C₆-C₁₀)aryl or (C₅-C₁₀)heteroaryl.

19. A compound according to one of claims 1 to 18, wherein R₅ is H, halogen, (C₁-C₆)alkyl, (C₂-C₆)alkenyl, (C₆-C₁₀)aryl, or (C₅-C₁₀)heteroaryl.

20. A compound according to one of claims 1 to 19, wherein R₅ is H, halogen, methyl, ethyl, vinyl, phenyl, thienyl, or pyridyl.

21. A compound according to one of claims 1 to 20, wherein R₅ is H, halogen, methyl, or ethyl.

22. A compound according to one of claims 1 to 21, wherein R₅ is H.

23. A compound according to one of claims 1 to 22, wherein R₇ is H, halogen, CN, (C₁-C₆)alkyl, O-(C₁-C₆)alkyl, (C₂-C₆)alkenyl, R' or (C₁-C₆)alkylene-(C₃-C₈)cycloalkyl.

24. A compound according to one of claims 1 to 23, wherein R₇ is H, halogen, CN, (C₁-C₄)alkyl, O-(C₁-C₄)alkyl, (C₁-C₄)alkenyl, phenyl, cyclopropyl or (C₅-C₆)heteroaryl.

25. A compound according to one of claims 1 to 24, wherein R₇ is H, fluoro, chloro, bromo, methyl, ethyl, methoxy, phenyl, nitrile, cyclopropyl, thienyl or vinyl.

26. A compound according to one of claims 1 to 25, wherein R₇ is H, fluoro, chloro, bromo, methyl or methoxy.

27. A compound according to one of claims 1 to 26, wherein R₇ is H.

28. A compound according to one of claims 1 to 27, wherein m is 2, 3, or 4.

29. A compound according to one of claims 1 to 28, wherein m is 3.

30. A compound according to one of claims 1 to 29, wherein R₂ is H, halogen or (C₁-C₄)alkyl.

31. A compound according to one of claims 1 to 30, wherein R2 is H or (C₁-C₂)alkyl

32. A compound according to one of claims 1 to 31, wherein R₂ is H, methyl or ethyl.

33. A compound according to one of claims 1 to 32, wherein n is 1, 2 or 3.

34. A compound according to one of claims 1 to 33, wherein n is 1 or 2.

35. A compound according to one of claims 1 to 34, wherein n is 1.

36. A compound according to one of claims 1 to 35, wherein
R₆ and R₆' are independently of each other
H,
(C₁-C₆)alkyl,
R',
(C₁-C₄)alkylene-(C₃-C₈)cycloalkyl,
(C₁-C₄)alkylene-(C₅-C₁₀)heterocyclyl,
C₁-C₄)alkylene-(C₆-C₁₀)aryl,
(C₁-C₆)alkylene-O-(C₁-C₆)alkyl,
(C₁-C₄)alkylene-C(O)-(C₅-C₁₀)heterocyclyl,
(C₁-C₄)alkylene-C(O)-(C₆₋C₁₀)aryl,
(C₁-C₆)alkylene-C(O)N[(C₁-C₆)alkyl]₂,
(C₁-C₆)alkylene-C(O)NH-(C₁-C₆)alkyl,
(C₁-C₆)alkylene-C(O)O-(C₁-C₆)alkyl,
C(O)O-(C₁₋C₆₎alkyl,
C(O)(C₁-C₆)alky),
C(O)R'
C(O)NH-(C₁-C₆)alkyl,
C(O)N[(C₁-C₆)alkyl]₂, or
C(O)(C₁-C₆)alkylene-R',
or R₆ and R₆', together with the N-atom to which they are attached, form a (C₅-C₁₀)heterocyclyl group.

37. A compound according to any of claims 1 to 36, wherein R₆ and R₆' are independently of each other
H,
(C₁-C₆)alkyl,
(C₅-C₁₀)heterocyclyl,
(C₃-C₈)cycloalkyl,
(C₆-C₁₀)aryl,
(C₁-C₄)alkylene-(C₃-C₈)cycloalkyl,
(C₁-C₄)alkylene-(C₅-C₁₀)heterocyclyl,
(C₁-C₄)alkylene-(C₆-C₁₀)aryl,
(C₁-C₆)alkylene-O-(C₁-C₆)alkyl,
(C₁-C₆)alkylene-C(O)N[(C₁-C₆)alkyl]₂,
(C₁-C₆)alkylene-C(O)NH-(C₁-C₆)alkyl,
(C₁-C₆)alkylene-C(O)O-(C₁-C₆)alkyl,
C(O)O-(C₁-C₆)alkyl,
C(O)(C₁-C₆)alkyl,
C(O)(C₃-C₈)cycloalkyl,
C(O)NH-(C₁-C₆)alkyl,
C(O)N[(C₁-C₆)alkyl]₂,
C(O)(C₁-C₆)alkylene-(C₃-C₈)cycloalkyl,
C(O)(C₁-C₆)alkylene- (C₅-C₁₀)heterocyclyl,
C(O)(C₁-C₆)alkylene-(C₆-C₁₀)aryl,
or R₆ and R₆', together with the N-atom to which they are attached, form a (C₅-C₁₀)heterocyclyl group.

38. A compound according to any of claims 1 to 37, wherein
R₆ is H, (C₁-C₆)alkyl, (C₃-C₆)cycloalkyl or (C₁-C₄)alkylene-(C₃-C₆)cycloalkyl, and
R₆' is H,
(C₁-C₆)alkyl,
(C₃-C₈)cycloalkyl,
(C₅-C₁₀)heterocyclyl,
(C₆-C₁₀)aryl,
(C₁-C₄)alkylene-(C₃-C₈)cycloalkyl,
(C₁-C₄)alkylene-(C₅-C₁₀)heterocyclyl,
(C₁-C₄)alkylene-(C₆-C₁₀)aryl,
(C₁-C₆)alkylene-O-(C₁-C₆)alkyl,
(C₁-C₆)alkylene-C(O)NH-(C₁-C₆)alkyl,
(C₁-C₆)alkylene-C(O)N[(C₁-C₆)alkyl]₂,
(C₁-C₆)alkylene-C(O)O-(C₁-C₆)alkyl,
C(O)O-(C₁-C₆)alkyl,
C(O)(C₁-C₆)alkyl,
C(O)(C₃-C₈)cycloalkyl,
C(O)NH-(C₁-C₆)alkyl,
C(O)N[(C₁-C₆)alkyl]₂,
C(O)(C₁-C₆)alkylene-(C₃-C₈)cycloalkyl,
C(O)(C₁-C₆)alkylene-(C₅-C₁₀)heterocyclyl,
C(O)(C₁-C₆)alkylene-(C₆-C₁₀)aryl, or
R₆ and R₆', together with the N-atom to which they are attached, form a (C₅-C₁₀)heterocyclyl group.

39. A compound according to any of claims 1 to 38, wherein
R₆ is H, (C₁-C₆)alkyl and
R₆' is H,
(C₁-C₆)alkyl,
(C₃-C₈)cycloalkyl,
(C₆-C₁₀)aryl,
(C₅-C₁₀)heterocyclyl,
(C₁-C₄)alkylene-(C₃-C₈)cycloalkyl,
(C₁-C₄)alkylene-(C₅-C₁₀)heterocyclyl,
(C₁-C₆)alkylene-(C₆-C₁₀)aryl,
(C₁-C₄)alkylene-O-(C₁-C₄)alkyl,
(C₁₋C₄)alkylene-C(O)N[(C₁-C₄)alkyl]₂,
(C₁-C₆)alkylene-C(O)NH-(C₁-C₆)alkyl,
C(O)(C₁-C₆)alkyl,
C(O)(C₁-C₆)alkylene-(C₅-C₁₀)heterocyclyl, or
R₆ and R₆', together with the N-atom to which they are attached, form a (C₅-C₁₀)heterocyclyl group.

40. A compound according to any of claims 1 to 339, wherein
R₆ is H, (C₁-C₆)alkyl and
R₆' is
H,
(C₁-C₆)alkyl;
(C₃-C₈)cycloalkyl;
(C₁-C₄)alkylene-(C₃-C₈)cycloalkyl;
(C₁-C₄)alkylene-O-(C₁-C₄)alkyl;
(C₁-C₄)alkylene-C(O)N[(C₁-C₄)alkyl]₂;
(C₁-C₄)alkylene-(C₅-C₁₀)heterocyclyl, or
(C₁-C₄)alkylene-(C₆-C₁₀)aryl;
C(O)(C₁-C₄)alkyl;
C(O)(C₁-C₄)alkylene-(C₅-C₁₀)heterocyclyl;
or R₆ and R₆', together with the N-atom to which they are attached, form a (C₅-C₆)heterocyclyl group.

41. A compound according to any of claims 1 to 40, wherein R₆ is H, (C₁-C₆)alkyl and R₆'is H, (C₁-C₆)alkyl or (C₃-C₈)cycloalkyl.

42. A compound according to any of claims 1 to 41, wherein R₆ is H and R₆' is H, (C₁-C₆)alkyl or (C₃-C₈)cycloalkyl.

43. A compound according to any of claims 1 to 42, wherein R₆ and R₆' are H.

44. A compound according to one of claims 1 to 43, wherein m is 3 and L is attached to the 3-position or to the 4-position of the amino cyclohexane ring.

45. A compound according to one of claims 1 to 44, wherein m is 3 and L is attached to the 4-position of the amino cyclohexane ring.

46. A compound according to one of claims 1 to 45, wherein p is 0.

47. Use of at least one compounds of the formula (I) and/or their pharmaceutically acceptable salt as claimed in one of claims 1 to 46 for producing a medicament.

48. Use of at least one compounds of the formula (I) and/or their pharmaceutically acceptable salt as claimed in one of claims 1 to 46 for producing a medicament for the treatment and/or prevention of hypertension, pulmonary hypertension, ocular hypertension, retinopathy, glaucoma, peripheral circulatory disorder, peripheral arterial occlusive disease (PAOD), coronary heart disease, angina pectoris, heart hypertrophy, heart failure, ischemic diseases, ischemic organ failure (end organ damage), fibroid lung, fibroid liver, liver failure, nephropathy, renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, AIDS, osteopathy, infection of digestive tracts with bacteria, sepsis or cancer development and progression.

49. A medicament comprising an effective amount of at least one compound as claimed in any of claims 1 to 46 and/or a pharmacologically acceptable salt thereof, pharmaceutically tolerated excipients and carriers and, where appropriate, further additives and/or other active ingredients.

## Patentansprüche

1. Verbindungen der Formel (I) 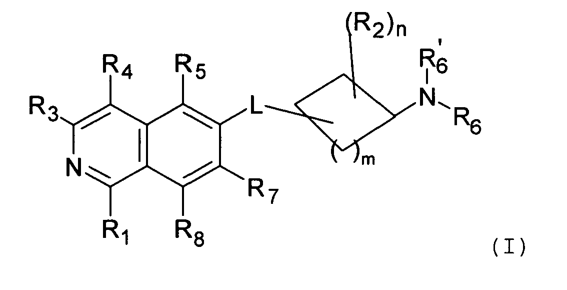 wobei
R₁ für H, OH oder NH₂ steht;
R₂ für H, Halogen oder (C₁-C₆) -Alkyl steht;
R₃ für
H,
Halogen,
(C₁-C₆) -Alkyl,
(C₁-C₆)-Alkylen-R',
OH,
O-R",
NH₂,
NHR",
NR"R" oder
NH-C(O)-R" steht;
R₄ für
H,
Halogen,
Hydroxy,
CN,
(C₁-C₆) -Alkyl,
R',
(C₁-C₆)-Alkylen-R' steht;
R₅ für
H,
Halogen,
CN,
NO₂,
(C₁-C₆) -Alkyl,
(C₂-C₆₎-Alkenyl,
R',
(C₁-C₆)-Alkylen-(C₆-C₁₀)-aryl,
(C₂-C₆)-Alkenylen-(C₆-C₁₀)-aryl,
(C₁-C₆)-Alkylen-(C₅-C₁₀)-heterocyclyl,
CH(OH)-(C₁-C₆)-Alkyl,
NH₂,
NH-R' ,
NH-SO₂H,
NH-SO₂-(C₁-C₆)-Alkyl,
NH-SO₂-R',
NH-C(O)-(C₁-C₆)-Alkyl,
NH-C(O)-R',
C(O)N[(C₁-C₆)-Alkyl]₂,
C(O)OH oder
C(O)O-(C₁-C₆)-Alkyl;
R₆ und R₆' unabhängig voneinander für H,
R',
(C₁-C₈)-Alkyl,
(C₁-C₆)-Alkylen-R',
(C₁-C₆)-Alkylen-O-(C₁-C₆)-alkyl,
(C₁-C₆)-Alkylen-O-R',
(C₁-C₆)-Alkylen-CH [R']₂,
(C₁-C₆)-Alkylen-C(O)-R',
(C₁-C₆)-Alkylen-C(O)NH₂,
(C₁-C₆)-Alkylen-C(O)NH-R',
(C₁-C₆)-Alkylen-C(O)NH-(C₁-C₆)-alkyl,
(C₁-C₆)-Alkylen-C(O)N[(C₁-C₆)-alkyl]₂,
(C₁-C₆)-Alkylen-C(O)N[R']₂;
(C₁-C₆)-Alkylen-C(O)O-(C₁-C₆)-alkyl,
C(O)O-(C₁-C₆)-Alkyl,
C(O)OR'
C(O)(C₁-C₆)-Alkyl,
C(O)R',
C(O)NH-(C₁-C₆)-Alkyl,
C(O)NHR',
C(O)N[(C₁-C₆)-Alkyl]-R'
C(O)N[(C₁-C₆)-Alkyl]₂,
C(O)-(C₁-C₆)-Alkylen-R',
C(O)O(C₁-C₆)-Alkylen-R' stehen, oder
R₆ und R₆' zusammen mit dem Stickstoffatom, an das sie gebunden sind, eine (C₅-C₁₀)-Heterocyclylgruppe bilden;
R₇ für
H,
Halogen,
CN,
NO₂,
(C₁-C₆)-Alkyl,
O-(C₁-C₆)-Alkyl,
(C₂-C₆) -Alkenyl,
R',
(C₂-C₆)-Alkenylen-(C₆-C₁₀)-aryl,
(C₁-C₆)-Alkylen-R',
CH(OH)-(C₁-C6)-Alkyl,
NH₂,
NH-R',
NH-SO₂H,
NH-SO₂-(C₁-C₆)-Alkyl,
NH-SO₂-R',
SO₂-NH₂,
SO₂-NHR',
NH-C(O)-(C₁-C₆)-Alkyl,
NH-C(O)-R',
C(O)N[(C₁-C₆)-Alkyl]₂,
C(O)OH oder
C(O)O-(C₁-₆)-Alkyl steht;
R₈ für H, Halogen oder (C₁-C₆)-Alkyl steht;
n für 1, 2, 3 oder 4 steht;
m für 1, 2, 3, 4 oder 5 steht; und
L für NH(CH₂) p oder N(C₁-C₆)-Alkyl-(CH₂)p steht;
p für 0, 1, 2, 3, oder 4 steht;
wobei
R' für
(C₃-C₈)-Cycloalkyl,
(C₅-C₁₀)-Heterocyclyl,
(C₆-C₁₀)-Aryl steht; und
R'' für
(C₃-C₈)-Cycloalkyl,
(C₅-C₁₀)-Heterocyclyl,
(C₆-C₁₀)-Aryl,
(C₁-C₆)-Alkyl,
(C₁-C₆)-Alkylen-R',
(C₁-C₆)-Alkylen-O-(C₁-C₆)-alkyl,
(C₁-C₆)-Alkylen-O-R'oder
(C₁-C₆)-Alkylen-NRₓR_{y} steht; und
wobei Rₓ und Ry unabhängig voneinander für (C₁-C₆)-Alkyl,
(C₅-C₁₀)-Heterocyclyl,
(C₆-C₁₀)-Aryl,
(C₁-C₄)-Alkylen-(C₅-C₁₀)-heterocyclyl,
(C₁-C₄)-Alkylen-(C₆-C₁₀)-aryl,
(C₁-C₄)-Alkylen-NH(C₁-C₆)-alkyl,
(C₁-C₄)-Alkylen-N[(C₁-C₆)-alkyl]₂,
(C₁-C₄)-Alkylen-N[(C₆-C₁₀)-aryl]₂ oder
(C₁-C₄-Alkylen-N[(C₅-C₁₀)-heterocyclyl]₂ stehen;
wobei in den Resten R₄, R₅, R₆, R₆', R₇ und R₈ Alkyl, Alkylen bzw. Cycloalkyl gegebenenfalls einfach oder mehrfach durch OH, OCH₃, COOH, COOCH₃, NH₂, NHCH₃,
N(CH₃)₂, CONH₂, CONHCH₃ oder CON(CH₃)₂ substituiert sein kann;
wobei in den Resten R₂ bis R₈ Alkyl bzw. Alkylen gegebenenfalls einfach oder mehrfach durch Halogen substituiert sein kann;
wobei in den Resten R₃ bis R₈ (C₆-C₁₀)-Aryl und (C₅-C₁₀)-Heterocyclyl unsubstituiert oder einfach oder mehrfach durch geeignete Gruppen unabhängig voneinander ausgewählt aus Halogen, OH, NO₂, N₃, CN, C(O)-(C₁-C₆)-Alkyl, C(O)-(C₆-C₁₀)-Aryl, COOH, COO(C₁-C₆)-Alkyl, CONH₂, CONH (C₁-C₆) -Alkyl, CON [(C₁-C₆)-Alkyl]₂, (C₃-C₈)-Cycloalkyl, (C₁-C₆)-Alkyl, (C₁-C₆) -Alkylen-OH, (C₁-C₆)-Alkylen-NH₂, (C₁-C₆) -Alkylen-NH (C₁-C₆) -alkyl, (C₁-C₆)-Alkylen-N[(C₁-C₆)-alkyl]₂, (C₂-C₆)-Alkenyl, (C₂-C₆)-Alkinyl, O-(C₁-C₆)-Alkyl, O-C(O)- (C₁-C₆) -Alkyl, PO₃H₂, SO₃H, SO₂-NH₂, SO₂NH(C₁-C₆)-Alkyl, SO₂N[(C₁-C₆)-Alkyl]₂, S-(C₁-C₆)-Alkyl, SO-(C₁-C₆) -Alkyl, SO₂- (C₁-C₆) -Alkyl, SO₂-N=CH-N [(C₁-C₆)-Alkyl]₂,
C(NH)(NH₂), NH₂, NH-(C₁-C₆)-Alkyl, N[(C₁-C₆)-Alkyl]₂, NH-C(O)-(C₁-C₆)-Alkyl, NH-C(O)O-(C₁-C₆)-Alkyl,
NH-SO₂- (C₁-C₆) -Alkyl, NH-SO₂- (C₆-C₁₀) -Aryl, NH-SO₂-(C₅-C₁₀)-Heterocyclyl, N(C₁-C₆)-Alkyl-C(O)-(C₁-C₆)-alkyl, N(C₁-C₆)-Alkyl-C(O)O-(C₁-C₆) -Alkyl,
N(C₁-C₆)-Alkyl-C(O)-NH-(C₁-C₆)-alkyl,
(C₆-C₁₀)-Aryl, (C₁-C₆)-Alkylen-(C₆-C₁₀)-aryl, O-(C₆-C₁₀)-Aryl, O- (C₁-C₆) -Alkylen- (C₆-C₁₀) aryl, (C₅-C₁₀)-Heterocyclyl, (C₁-C₆)-Alkylen-(C₅-C₁₀)-heterocyclyl, O-(C₁-C₆)-Alkylen-(C₅-C₁₀)-heterocyclyl substituiert sind,
wobei das (C₆-C₁₀)-Aryl bzw. (C₅-C₁₀) -Heterocyclyl einfach bis dreifach durch eine Gruppe unabhängig ausgewählt aus Halogen, OH, NO₂, CN, O-(C₁-C₆)-Alkyl, (C₁-C₆)-Alkyl, NH₂, NH (C₁-C₆)-Alkyl, N [(C₁-C₆) -Alkyl]₂, SO₂CH₃, COOH, C(O)O-(C₁-C₆)-Alkyl, CONH₂, (C₁-C₆)-Alkylen-O-(C₁-C₆)-alkyl, (C₁-C₆) -Alkylen-O- (C₆-C₁₀) -aryl oder O-(C₁-C₆)-Alkylen-(C₆-C₁₀)-aryl substituiert sein kann;
oder wobei (C₆-C₁₀)-Aryl vicinal durch eine O-(C₁-C₄)-Alkylen-O-Gruppe substituiert ist, wobei mit den Kohlenstoffatomen, an die die Sauerstoffatome gebunden sind, ein 5- bis 8-gliedriger Ring gebildet wird;
und wobei Aryl- bzw. Heterocyclylsubstituenten von (C₆-C₁₀)-Arylgruppen und (C₅-C₁₀)-Heterocyclylgruppen nicht weiter durch eine aryl- oder heterocyclylhaltige Gruppe substituiert sind;
und deren stereoisomere und/oder tautomere Formen und/oder deren pharmazeutisch unbedenkliche Salze.

2. Verbindungen der Formel (I) nach Anspruch 1, wobei R₁ für H steht und die Verbindungen durch die Formel (II) charakterisiert sind.

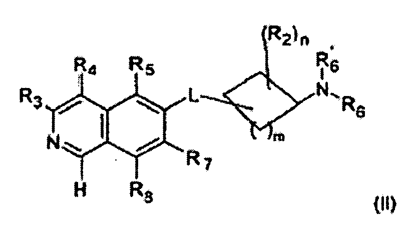

3. Verbindungen der Formel (I) nach Anspruch 1, wobei R₁ für OH steht und die Verbindungen durch die Formel (III) charakterisiert sind.

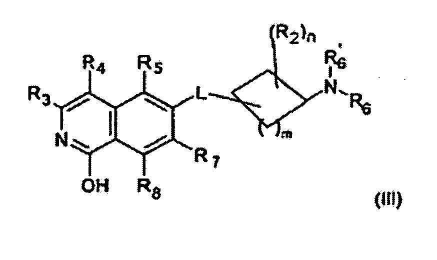

4. Verbindungen der Formel (I) nach Anspruch 1 oder Anspruch 3, wobei R₁ für OH steht und die Verbindungen durch die Formel (III') charakterisiert sind.

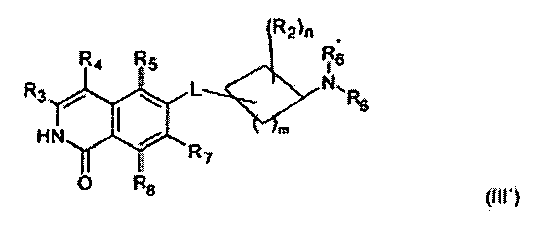

5. Verbindungen nach Anspruch 1, wobei R₁ für NH₂ steht.

6. Verbindungen nach einem der Ansprüche 1 bis 5, wobei R₃ für H, Halogen, (C₁-C₄)-Alkylen-R', O-R" oder NHR" steht.

7. Verbindungen nach einem der Ansprüche 1 bis 6, wobei R₃ für H oder NHR" steht.

8. Verbindungen nach einem der Ansprüche 1 bis 7, wobei R₃ für H, NH-(C₅-C₆)-Heterocyclyl oder NH-Phenyl steht.

9. Verbindungen nach einem der Ansprüche 1 bis 8, wobei R₃ für H steht.

10. Verbindungen nach einem der Ansprüche 1 bis 9, wobei R₈ für H, Halogen oder (C₁-C₄)-Alkyl steht.

11. Verbindungen nach einem der Ansprüche 1 bis 10, wobei R₈ für H, Cl, F, Methyl oder Ethyl steht.

12. Verbindungen nach einem der Ansprüche 1 bis 11, wobei R₈ für H steht.

13. Verbindungen nach einem der Ansprüche 1 bis 12, wobei R₄ für H, Halogen oder (C₁-C₆)-Alkyl steht.

14. Verbindungen nach einem der Ansprüche 1 bis 13, wobei R₄ für H, Halogen oder (C₁-C₄)-Alkyl steht.

15. Verbindungen nach einem der Ansprüche 1 bis 14, wobei R₄ für H steht.

16. Verbindungen nach einem der Ansprüche 1 bis 15, wobei R₅ für H, Halogen, CN, (C₁-C₆)-Alkyl, (C₂-C₆)-Alkenyl, R', NH- (C₆-C₁₀) -Aryl, (C₁-C₆) -Alkylen-C₆-C₁₀)-aryl oder (C₁-C₆)-Alkylen-(C₅-C₁₀)-heterocyclyl steht.

17. Verbindungen nach einem der Ansprüche 1 bis 16, wobei R₅ für H, Halogen, (C₁-C₆)-Alkyl, (C₂-C₆)-Alkenyl, R', NH- (C₆-C₁₀)-Aryl, (C₁-C₆)-Alkylen-(C₆-C₁₀)-aryl oder (C₁-C₆)-Alkylen-(C₅-C10)-heterocyclyl steht.

18. Verbindungen nach einem der Ansprüche 1 bis 17, wobei R₅ für H, Halogen, (C₁-C₆)-Alkyl,(C₂-C₆)-Alkenyl, (C₆-C₁₀)-Aryl, NH-(C₆-C₁₀)-Aryl,(C₁-C₂)-Alkyl,-(C₆-C₁₀)-aryl oder (C₅-C₁₀)-Heteroaryl steht.

19. Verbindungen nach einem der Ansprüche 1 bis 18, wobei R₅ für H, Halogen, (C₁-C₆)-Alkyl, (C₂-C₆)-Alkenyl, (C₆-C₁₀)-Aryl oder (C₅-C₁₀)-Heteroaryl steht.

20. Verbindungen nach einem der Ansprüche 1 bis 19, wobei R₅ für H, Halogen, Methyl, Ethyl, Vinyl, Phenyl, Thienyl oder Pyridyl steht.

21. Verbindungen nach einem der Ansprüche 1 bis 20, wobei R₅ für H, Halogen, Methyl oder Ethyl steht.

22. Verbindungen nach einem der Ansprüche 1 bis 21, wobei R₅ für H steht.

23. Verbindungen nach einem der Ansprüche 1 bis 22, wobei R₇ für H, Halogen, CN, (C₁-C₆)-Alkyl, O-(C₁-C₆)-Alkyl, (C₂-C₆)-Alkenyl, R' oder (C₁-C₆)-Alkylen-(C₃-C₈)-cycloalkyl steht.

24. Verbindungen nach einem der Ansprüche 1 bis 23, wobei R₇ für H, Halogen, CN, (C₁-C₄)-Alkyl, O-(C₁-C₉)-Alkyl, (C₁-C₄)-Alkenyl, Phenyl, Cyclopropyl oder (C₅-C₆)-Heteroaryl steht.

25. Verbindungen nach einem der Ansprüche 1 bis 24, wobei R₇ für H, Fluor, Chlor, Brom, Methyl, Ethyl, Methoxy, Phenyl, Nitril, Cyclopropyl, Thienyl oder Vinyl steht.

26. Verbindungen nach einem der Ansprüche 1 bis 25, wobei R₇ für H, Fluor, Chlor, Brom, Methyl oder Methoxy steht.

27. Verbindungen nach einem der Ansprüche 1 bis 26, wobei R₇ für H steht.

28. Verbindungen nach einem der Ansprüche 1 bis 27, wobei m für 2, 3 oder 4 steht.

29. Verbindungen nach einem der Ansprüche 1 bis 28, wobei m für 3 steht.

30. Verbindungen nach einem der Ansprüche 1 bis 29, wobei R₂ für H, Halogen oder (C₁-C₄)-Alkyl steht.

31. Verbindungen nach einem der Ansprüche 1 bis 30, wobei R₂ für H oder (C₁-C₂)-Alkyl steht.

32. Verbindungen nach einem der Ansprüche 1 bis 31, wobei R₂ für H, Methyl oder Ethyl steht.

33. Verbindungen nach einem der Ansprüche 1 bis 32, wobei n für 1, 2 oder 3 steht.

34. Verbindungen nach einem der Ansprüche 1 bis 33, wobei n für 1 oder 2 steht.

35. Verbindungen nach einem der Ansprüche 1 bis 34, wobei n für 1 steht.

36. Verbindungen nach einem der Ansprüche 1 bis 35, wobei
R₆ und R₆' unabhängig voneinander für
H,
(C₁-C₆)-Alkyl,
R',
(C₁-C₄)-Alkylen-(C₃-C₈)-cycloalkyl,
(C₁-C₄)-Alkylen-(C₅-C₁₀)-heterocyclyl,
(C₁-C₄)-Alkylen-(C₆-C₁₀)-aryl,
(C₁-C₆)-Alkylen-O-(C₁-C₆)-alkyl,
(C₁-C₄-Alkylen-C(O)-(C₅-C₁₀)-heterocyclyl,
(C₁-C₄)-Alkylen-C(O)-(C₆-C₁₀)-aryl,
(C₁-C₆)-Alkylen-C(O)N[(C₁-C₆)-alkyl]₂,
(C₁-C₆)-Alkylen-C(O)NH-(C₁-C₆)-alkyl,
(C₁-C₆)-Alkylen-C(O)O-(C₁-C₆)-alkyl,
C(O)O-(C₁-C₆)-Alkyl,
C(O)(C₁-C₆)-Alkyl,
C(O)R',
C(O)NH-(C₁-C₆)-Alkyl,
C(O)N[(C₁-C₆)-Alkyl]oder
C(O)(C₁-C₆)-Alkylen-R'stehen,
oder R₆ und R₆' zusammen mit dem Stickstoffatom, an das sie gebunden sind, eine (C₅-C₁₀) -Heterocyclylgruppe bilden.

37. Verbindungen nach einem der Ansprüche 1 bis 36, wobei R₆ und R₆' unabhängig voneinander für H,
(C₁-C₆)-Alkyl,
(C₅-C₁₀)-Heterocyclyl,
(C₃-C₈)-Cycloalkyl,
(C₆-C₁₀)-Aryl,
(C₁-C₄)-Alkylen-(C₃-C₈)-cycloalkyl,
(C₁-C₄)-Alkylen-(C₅-C₁₀)-heterocyclyl,
(C₁-C₄)-Alkylen-(C₆-C₁₀)-aryl,
(C₁-C₆)-Alkylen-O-(C₁-C₆)-alkyl,
(C₁-C₆)-Alkylen-C(O)N[(C₁-C₆)-alkyl]₂,
(C₁-C₆)-Alkylen-C(O)NH-(C₁-C₆)-alkyl,
(C₁-C₆)-Alkylen-C(O)O-(C₁-C₆)-alkyl,
C(O)O-(C₁-C₆)-Alkyl,
C(O)(C₁-C₆)-Alkyl,
C(O)(C₃-C₈)-Cycloalkyl,
C(O)NH-(C₁-C₆)-Alkyl,
C(O)N[(C₁-C₆)-Alkyl]₂,
C(O)(C₁-C₆)-Alkylen-(C₃-C₈)-cycloalkyl,
C(O)(C₁-C₆)-Alkylen-(C₅-C₁₀)-heterocyclyl,
C(O)(C₁-C₆)-Alkylen-(C₆-C₁₀)-aryl stehen,
oder R₆ und R₆' zusammen mit dem Stickstoffatom, an das sie gebunden sind, eine (C₅-C₁₀) -Heterocyclylgruppe bilden.

38. Verbindungen nach einem der Ansprüche 1 bis 37, wobei
R₆ für H, (C₁-C₆)-Alkyl, (C₃-C₆)-Cycloalkyl oder (C₁-C₄)-Alkylen-(C₃-C₆)-cycloalkyl steht und
R₆' für H,
(C₁-C₆)-Alkyl,
(C₃-C₈)-Cycloalkyl,
(C₅-C₁₀)-Heterocyclyl,
(C₆-C₁₀)-Aryl,
(C₁-C₄)-Alkylen-(C₃-C₈)-cycloalkyl,
(C₁-C₄)-Alkylen-(C₅-C₁₀)-heterocyclyl,
(C₁-C₄)-Alkylen-(C₆-C₁₀)-aryl,
(C₁-C₆)-Alkylen-O-(C₁-C₆)-alkyl,
(C₁-C₆)-Alkylen-C(O)NH-(C₁-C₆)-alkyl,
(C₁-C₆)-Alkylen-C(O)N[(C₁-C₆)-alkyl]₂,
(C₁-C₆)-Alkylen-C(O)O-(C₁-C₆)-alkyl,
C(O)O-(C₁-Cₛ)-Alkyl,
C(O)(C₁-C₆)-Alkyl,
C(O)(C₃-C₈)-Cycloalkyl,
C(O)NH-(C₁-C₆)-Alkyl,
C(O)N[(C₁-C₆)-Alkyl]₂,
C(O)(C₁-C₆)-Alkylen-(C₃-C₈)-cycloalkyl,
C(O)(C₁-C₆)-Alkylen-(C₅-C₁₀)-heterocyclyl,
C(O)(C₁-C₆)-Alkylen- (C₆-C₁₀)-aryl steht, oder
R₆ und R₆' zusammen mit dem Stickstoffatom, an das sie gebunden sind, eine (C₅-C₁₀)-Heterocyclylgruppe bilden.

39. Verbindungen nach einem der Ansprüche 1 bis 38, wobei
R₆ für H oder (C₁-C₆)-Alkyl steht und
R₆' für H,
(C₁-C₆)-Alkyl,
(C₃-C₈)-Cycloalkyl,
(C₆-C₁₀)-Aryl,
(C₅-C₁₀)-Heterocyclyl,
(C₁-C₄)-Alkylen-(C₃-C₈)-cycloalkyl,
(C₁-C₄-Alkylen-(C₅-C₁₀)-heterocyclyl,
(C₁-C₆)-Alkylen-(C₆-C₁₀)-aryl,
(C₁-C₄)-Alkylen-O-(C₁-C₄)-alkyl,
(C₁-C₄)-Alkylen-C(O)N[(C₁-C₄)-alkyl]₂,
(C₁-C₆)-Alkylen-C(O)NH-(C₁-C₆)-alkyl,
C(O)(C₁-C₆)-Alkyl,
C(O)(C₁-C₆)-Alkylen-(C₅-C₁₀)-heterocyclyl steht, oder
R₆ und R₆' zusammen mit dem Stickstoffatom, an das sie gebunden sind, eine (C₅-C₁₀)-Heterocyclylgruppe bilden.

40. Verbindungen nach einem der Ansprüche 1 bis 39,
wobei
R₆ für H oder (C₁-C₆)-Alkyl steht und
R₆' für H,
(C₁-C₆)-Alkyl,
(C₃-C₈)-Cycloalkyl,
(C₁-C₄)-Alkylen-(C₃-C₈)-cycloalkyl,
(C₁-C₄)-Alkylen-O-(C₁-C₄)-alkyl,
(C₁-C₄)-Alkylen-C(O)N[(C₁-C₄)-alkyl]₂,
(C₁-C₄)-Alkylen-(C₅-C₁₀)-heterocyclyl,
(C₁-C₄)-Alkylen-(C₆-C₁₀)-aryl,
C(O)(C₁-C₄)-Alkyl oder
C(O)(C₁-C₄)-Alkylen-(C₅-C₁₀)-heterocyclylsteht;
oder R₆ und R₆' zusammen mit dem Stickstoffatom, an das sie gebunden sind, eine (C₅-C₆)-Heterocyclylgruppe bilden.

41. Verbindungen nach einem der Ansprüche 1 bis 40, wobei R₆ für H oder (C₁-C₆)-Alkyl steht und R₆' für H, (C₁-C₆)-Alkyl oder (C₃-C₈)-Cycloalkyl steht.

42. Verbindungen nach einem der Ansprüche 1 bis 41, wobei R₆ für H steht und R₆' für H, (C₁-C₆)-Alkyl oder (C₃-C₈)-Cycloalkyl steht.

43. Verbindungen nach einem der Ansprüche 1 bis 42, wobei R₆ und R₆' für H stehen.

44. Verbindungen nach einem der Ansprüche 1 bis 43, wobei m für 3 steht und L an die 3-Stellung oder die 4-Stellung des Aminocyclohexanrings gebunden ist.

45. Verbindungen nach einem der Ansprüche 1 bis 44, wobei m für 3 steht und L an die 4-Stellung des Aminocyclohexanrings gebunden ist.

46. Verbindungen nach einem der Ansprüche 1 bis 45, wobei p für 0 steht.

47. Verwendung wenigstens einer Verbindung der Formel (I) und/oder ihres pharmazeutisch unbedenklichen Salzes nach einem der Ansprüche 1 bis 46 zur Herstellung eines Medikaments.

48. Verwendung wenigstens einer Verbindung der Formel (I) und/oder ihres pharmazeutisch unbedenklichen Salzes nach einem der Ansprüche 1 bis 46 zur Herstellung eines Medikaments zur Behandlung und/oder Prävention von Hypertonie, pulmonaler Hypertonie, okularer Hypertonie, Retinopathie, Glaukom, peripherer Kreislaufstörung, peripherer arterieller Verschlusskrankheit (Peripheral Arterial Occlusive Disease, PAOD), koronarer Herzkrankheit, Angina pectoris, Herzhypertrophie, Herzinsuffizienz, ischämischen Krankheiten, ischämischem Organversagen (Organ-Endschädigung), Lungenfibrose, Leberfibrose, Leberversagen, Nephropathie, Nierenversagen, Nierenfibrose, Nierenglomerulosklerose, Organhypertrophie, Asthma, chronischer obstruktiver Atemwegserkrankung (Chronic Obstructive Pulmonary Disease, COPD), Atemnotsyndrom bei Erwachsenen, thrombotischen Erkrankungen, Schlaganfall, zerebralen Vasospasmen, zerebraler Ischämie, Schmerzen, neuronaler Degeneration,
Rückenmarksverletzung, Alzheimer-Krankheit, Frühgeburt, erektiler Dysfunktion, endokrinen Dysfunktionen, Arteriosklerose, Prostatahypertrophie, Diabetes und diabetischen Komplikationen, metabolischem Syndrom, Blutgefäßrestenose, Atherosklerose, Entzündungen, Autoimmunerkrankungen, AIDS, Osteopathie, bakterieller Infektion des Verdauungstraktes, Sepsis und Krebsentwicklung und -progression.

49. Medikament, enthaltend eine wirksame Menge wenigstens einer Verbindung nach einem der Ansprüche 1 bis 46 und/oder eines pharmazeutisch unbedenklichen Salzes davon, pharmazeutisch verträgliche Exzipienten und Träger und gegebenenfalls weitere Zusatzstoffe und/oder andere Wirkstoffe.

## Revendications

1. Composé de formule (I) 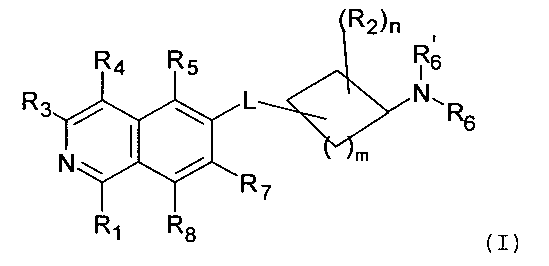 dans laquelle
R₁ est H, OH ou NH₂ ;
R₂ est H, halogène ou (C₁-C₆)alkyle ;
R₃ est
H,
halogène,
(C₁-C₆)alkyle,
(C₁-C₆)alkylène-R',
OH,
O-R",
NH₂,
NHR",
NR"R" ou
NH-C(O)-R'';
R₄ est
H,
halogène,
hydroxy,
CN,
(C₁-C₆)alkyle,
R',
(C₁-C₆)alkylène-R' ;
R₅ est
H,
halogène,
CN,
NO₂,
(C₁-C₆)alkyle,
(C₂-C₆)alcényle,
R',
(C₁-C₆)alkylène-(C₆-C₁₀)aryle,
(C₂-C₆)alcénylène-(C₆-C₁₀)aryle,
(C₁-C₆)alkylène-(C₅-C₁₀)hétérocyclyle,
CH(OH)-(C₁-C₆)alkyle,
NH₂,
NH-R',
NH-SO₂H,
NH-SO₂-(C₁-C₆)alkyle,
NH-SO₂-R',
NH-C(O)-(C₁-C₆)alkyle,
NH-C(O)-R',
C(O)N[(C₁-C₆)alkyle]₂,
C(O)OH, ou
C(O)O-(C₁-C₆)alkyle ;
R₆ et R₆' sont indépendamment l'un de l'autre
H,
R',
(C₁-C₈)alkyle,
(C₁-C₆)alkylène-R',
(C₁-C₆)alkylène-O-(C₁-C₆)alkyle,
(C₁-C₆)alkylène-O-R',
(C₁-C₆)alkylène-CH[R']₂,
(C₁-C₆)alkylène-C(O)-R',
(C₁-C₆)alkylène-C(O)NH_{2'}
(C₁-C₆)alkylène-C(O)NH-R',
(C₁-C₆)alkylène-C(O)NH-(C₁-C₆)alkyle,
(C₁-C₆)alkylène-C(O)N[(C₁-C₆)alkyle]₂,
(C₁-C₆)alkylène-C(O)N[R']₂ ;
(C₁-C₆)alkylène-C(O)O-(C₁-C₆)alkyle,
C(O)O-(C₁-C₆)alkyle,
C(O)OR'
C(O)(C₁-C₆)alkyle,
C(O)R',
C(O)NH-(C₁-C₆)alkyle,
C(O)NHR',
C(O)N[(C₁-C₆)alkyle]R'
C(O)N[(C₁-C₆)alkyle]₂,
C(O)-(C₁-C₆)alkylène-R',
C(O)O(C₁-C₆)alkylène-R', ou
R₆ et R₆', conjointement avec l'atome de N auquel ils sont liés, forment un groupement (C₅-C₁₀)hétérocyclyle ;
R₇ est
H,
halogène,
CN,
NO₂,
(C₁-C₆)alkyle,
O-(C₁-C₆)alkyle,
(C₂-C₆)alcényle,
R',
(C₂-C₆)alcénylène-(C₆-C₁₀)aryle,
(C₁-C₆)alkylène-R',
CH(OH)-(C₁-C₆)alkyle,
NH₂,
NH-R',
NH-SO₂H,
NH-SO₂-(C₁-C₆)alkyle,
NH-SO₂-R',
SO₂-NH₂,
SO₂-NHR',
NH-C(O)-(C₁-C₆)alkyle,
NH-C(O)-R',
C(O)N[(C₁-C₆)alkyle]₂,
C(O)OH, ou
C(O)O-(C₁-C₆)alkyle ;
R₈ est H, halogène ou (C₁-C₆)alkyle ;
n est 1, 2, 3 ou 4 ;
m est 1, 2, 3, 4 ou 5 ; et
L est NH(CH₂)p ou N(C₁-C₆)alkyl-(CH₂)p ;
p est 0, 1, 2, 3, ou 4 ;
où
R' est
(C₃-C₈)cycloalkyle,
(C₅-C₁₀)hétérocyclyle,
(C₆-C₁₀)aryle ; et
R" est
(C₃-C₈)cycloalkyle,
(C₅-C₁₀)hétérocyclyle,
(C₆-C₁₀)aryle,
(C₁-C₆)alkyle,
(C₁-C₆)alkylène-R',
(C₁-C₆)alkylène-O-(C₁-C₆)alkyle,
(C₁-C₆)alkylène-O-R', or
(C₁-C₆)alkylène-NRₓR_{y} ; et
où Rₓ et R_{y} sont indépendamment l'un de l'autre
(C₁-C₆)alkyle,
(C₅-C₁₀)hétérocyclyle,
(C₆-C₁₀)aryle,
(C₁-C₄)alkylène-(C₅-C₁₀)hétérocyclyle,
(C₁-C₄)alkylène-(C₆-C₁₀)aryle,
(C₁-C₄)alkylène-NH(C₁-C₆)alkyle,
(C₁-C₄)alkylène-N[(C₁-C₆)alkyle]₂,
(C₁-C₄)alkylène-N[(C₆-C₁₀)aryle]₂, ou
(C₁-C₄)alkylène-N[(C₅-C₁₀)hétérocyclyle]₂ ;
où dans les résidus R₄, R₅, R₆, R₆', R₇ et R₈ alkyle, alkylène ou cycloalkyle peut être éventuellement substitué une ou plusieurs fois par OH, OCH₃, COOH, COOCH₃, NH₂, NHCH₃, N(CH₃)₂, CONH₂, CONHCH₃ ou CON(CH3)2 ;
où dans les résidus R₂ à R₈ alkyle ou alkylène peuvent être éventuellement substitués une ou plusieurs fois par halogène ;
où dans les résidus R₃ à R₈ (C₆-C₁₀)aryle et (C₅-C₁₀)hétérocyclyle sont non substitués ou substitués une ou plusieurs fois par des groupements convenables choisis indépendamment parmi halogène, OH, NO₂, N₃, CN, C(O)-(C₁-C₆)alkyle, C(O)-(C₆-C₁₀)aryle, COOH, COO(C₁-C₆)alkyle, CONH₂, CONH(C₁-C₆)alkyle, CON[(C₁-C₆)alkyle]₂, (C₃-C₈)cycloalkyle, (C₁-C₆)alkyle, (C₁-C₆)alkylène-OH, (C₁-C₆)alkylène-NH₂, (C₁-C₆)alkylène-NH(C₁-C₆)alkyle, (C₁-C₆)alkylène-N[(C₁-C₆)alkyle]₂, (C₂-C₆)alcényle, (C₂-C₆)alcynyle, O-(C₁-C₆)alkyle, O-C(O)-(C₁-C₆)alkyle, PO₃H₂, SO₃H, SO₂-NH₂, SO₂NH(C₁-C₆)alkyle, SO₂N[(C₁-C₆)alkyle]₂, S-(C₁-C₆)alkyle, SO-(C₁-C₆)alkyle, SO₂-(C₁-C₆)alkyle, SO₂-N=CH-N[(C₁-C₆)alkyle]₂,
C(NH)(NH₂), NH₂, NH-(C₁-C₆)alkyle, N[(C₁-C₆)alkyle]₂, NH-C(O)-(C₁-C₆)alkyle, NH-C(O)O-(C₁-C₆)alkyle, NH-SO₂-(C₁-C₆)alkyle, NH-SO₂-(C₆-C₁₀)aryle, NH-SO₂-(C₅-C₁₀)hétérocyclyle, N(C₁-C₆)alkyl-C(O)-(C₁-C₆)alkyle, N(C₁-C₆)alkyl-C(O)O-(C₁-C₆)alkyle, N(C₁-C₆)alkyl-C(O)-NH-(C₁-C₆)alkyle],
(C₆-C₁₀)aryle, (C₁-C₆) alkylène-(C₆-C₁₀)aryle, O-(C₆-C₁₀)aryle, O-(C₁-C₆)alkylène-(C₆-C₁₀)aryle, (C₅-C₁₀)hétérocyclyle, (C₁-C₆)alkylène-(C₅-C₁₀)hétérocyclyle, O-(C₁-C₆)alkylène-(C₅-C₁₀)hétérocyclyle, où (C₆-C₁₀)aryle ou (C₅-C₁₀)hétérocyclyle peut être substitué une à trois fois par un groupement choisi indépendamment parmi halogène, OH, NO₂, CN, O-(C₁-C₆)alkyle, (C₁-C₆)alkyle, NH₂, NH(C₁-C₆)alkyle, N[(C₁-C₆)alkyle]₂, SO₂CH₃, COOH, C(O)O-(C₁-C₆)alkyle, CONH₂, (C₁-C₆)alkylène-O-(C₁-C₆)alkyle, (C₁-C₆)alkylène-O-(C₆-C₁₀)aryle, ou O-(C₁-C₆)alkylène-(C₆-C₁₀)aryle ;
ou où (C₆-C₁₀)aryle est substitué vicinalement par un groupement O-(C₁-C₄)alkylène-O, un cycle à 5-8 chaînons étant formé avec les atomes de carbone auxquels les atomes d'oxygène sont liés ;
et où les substituants aryle ou hétérocyclyle des groupements (C₆-C₁₀)aryle et (C₅-C₁₀)hétérocyclyle peuvent ne pas être substitués en outre par un groupement contenant un aryle ou un hétérocyclyle ;
ou ses formes stéréoisomères et/ou ses formes tautomères et/ou ses sels pharmaceutiquement acceptables.

2. Composé de formule (I) selon la revendication 1, dans lequel R₁ est H et est **caractérisé par** la formule (II)

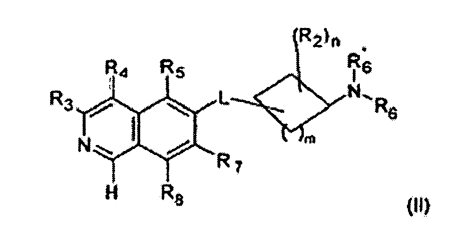

3. Composé de formule (I) selon la revendication 1, dans lequel R₁ est OH, et est **caractérisé par** la formule (III)

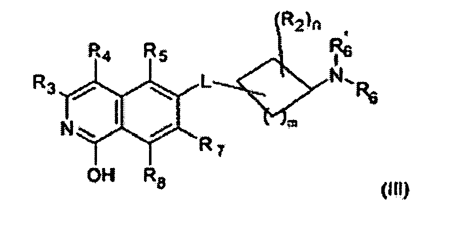

4. Composé de formule (I) selon la revendication 1 ou la revendication 3, dans lequel R₁ est OH et est **caractérisé par** la formule (III')

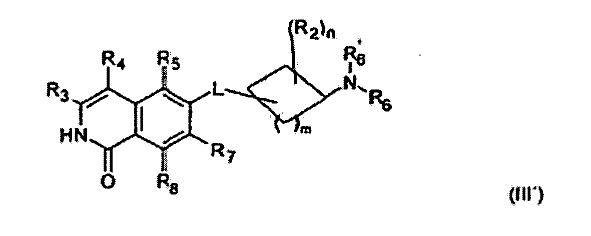

5. Composé selon la revendication 1, **caractérisé en ce que** R₁ est NH₂.

6. Composé selon l'une des revendications 1 à 5, **caractérisé en ce que** R₃ est H, halogène, (C₁-C₄)alkylène-R', O-R" ou NHR".

7. Composé selon l'une des revendications 1 à 6, **caractérisé en ce que** R₃ est H ou NHR".

8. Composé selon l'une des revendications 1 à 7, **caractérisé en ce que** R₃ est H ; NH-(C₅-C₆)hétérocyclyle, ou NH-phényle.

9. Composé selon l'une des revendications 1 à 8, **caractérisé en ce que** R₃ est H.

10. Composé selon l'une des revendications 1 à 9, **caractérisé en ce que** R₈ est H, halogène ou (C₁-C₄)alkyle.

11. Composé selon l'une des revendications 1 à 10, **caractérisé en ce que** R₈ est H, Cl, F, méthyle ou éthyle.

12. Composé selon l'une des revendications 1 à 11, **caractérisé en ce que** R₈ est H.

13. Composé selon l'une des revendications 1 à 12, **caractérisé en ce que** R₄ est H, halogène ou (C₁-C₆)alkyle.

14. Composé selon l'une des revendications 1 à 13, **caractérisé en ce que** R₄ est H, halogène ou (C₁-C₄)alkyle.

15. Composé selon l'une des revendications 1 à 14, **caractérisé en ce que** R₄ est H.

16. Composé selon l'une des revendications 1 à 15, **caractérisé en ce que** R₅ est H, halogène, CN, (C₁-C₆)alkyle, (C₂-C₆)alcényle, R', NH-(C₆-C₁₀)aryle, (C₁-C₆)alkylène-(C₆-C₁₀)aryle ou (C₁-C₆)alkylène-(C₅-C₁₀)hétérocyclyle.

17. Composé selon l'une des revendications 1 à 16, **caractérisé en ce que** R₅ est H, halogène, (C₁-C₆)alkyle, (C₂-C₆)alcényle, R', NH-(C₆-C₁₀)aryle, (C₁-C₆)alkylène-(C₆-C₁₀)aryle ou (C₁-C₆)alkylène-(C₅-C₁₀)hétérocyclyle.

18. Composé selon l'une des revendications 1 à 17, **caractérisé en ce que** R₅ est H, halogène, (C₁-C₆)alkyle, (C₂-C₆)alcényle, (C₆-C₁₀)aryle, NH-(C6-C₁₀)aryle, (C₁-C₂)alkyl-(C₆-C₁₀)aryle ou (C₅-C₁₀)hétéroaryle.

19. Composé selon l'une des revendications 1 à 18, **caractérisé en ce que** R₅ est H, halogène, (C₁-C₆)alkyle, (C₂-C₆)alcényle, (C₆-C₁₀)aryle, ou (C₅-C₁₀)hétéroaryle.

20. Composé selon l'une des revendications 1 à 19, **caractérisé en ce que** R₅ est H, halogène, méthyle, éthyle, vinyle, phényle, thiényle, ou pyridyle.

21. Composé selon l'une des revendications 1 à 20, **caractérisé en ce que** R₅ est H, halogène, méthyle, ou éthyle.

22. Composé selon l'une des revendications 1 à 21, **caractérisé en ce que** R₅ est H.

23. Composé selon l'une des revendications 1 à 22, **caractérisé en ce que** R₇ est H, halogène, CN, (C₁-C₆)alkyle, O-(C₁-C₆)alkyle, (C₂-C₆)alcényle, R' ou (C₁-C₆)alkylène-(C₃-C₈)cycloalkyle.

24. Composé selon l'une des revendications 1 à 23, **caractérisé en ce que** R₇ est H, halogène, CN, (C₁-C₄)alkyle, O-(C₁-C₄)alkyle, (C₁-C₄)alcényle, phényle, cyclopropyle ou (C₅-C₆)hétéroaryle.

25. Composé selon l'une des revendications 1 à 24, **caractérisé en ce que** R₇ est h, fluoro, chloro, bromo, méthyle, éthyle, méthoxy, phényle, nitrile, cyclopropyle, thiényle ou vinyle.

26. Composé selon l'une des revendications 1 à 25, **caractérisé en ce que** R₇ est H, fluoro, chloro, bromo, méthyle ou méthoxy.

27. Composé selon l'une des revendications 1 à 26, **caractérisé en ce que** R₇ est H.

28. Composé selon l'une des revendications 1 à 27, **caractérisé en ce que** m est 2, 3, ou 4.

29. Composé selon l'une des revendications 1 à 28, **caractérisé en ce que** m est 3.

30. Composé selon l'une des revendications 1 à 29, **caractérisé en ce que** R₂ est H, halogène ou (C₁-C₄) alkyle.

31. Composé selon l'une des revendications 1 à 30, **caractérisé en ce que** R₂ est H ou (C₁-C₂) alkyle.

32. Composé selon l'une des revendications 1 à 31, **caractérisé en ce que** R₂ est H, méthyle ou éthyle.

33. Composé selon l'une des revendications 1 à 32, **caractérisé en ce que** n est 1, 2 ou 3.

34. Composé selon l'une des revendications 1 à 33, **caractérisé en ce que** n est 1 ou 2.

35. Composé selon l'une des revendications 1 à 34, **caractérisé en ce que** n est 1.

36. Composé selon l'une des revendications 1 à 35,
**caractérisé en ce que**
R₆ et R₆' sont indépendamment l'un de l'autre
H,
(C₁-C₆)alkyle,
R',
(C₁-C₄)alkylène-(C₃-C₈)cycloalkyle,
(C₁-C₄)alkylène-(C₅-C₁₀)hétérocyclyle,
(C₁-C₄)alkylène-(C₆-C₁₀)aryle,
(C₁-C₆)alkylène-O-(C₁-C₆)alkyle,
(C₁-C₄)alkylène-C(O)-(C₅-C₁₀)hétérocyclyle,
(C₁-C₄)alkylène-C(O)-(C₆-C₁₀)aryle,
(C₁-C₆)alkylène-C(O)N[(C₁-C₆)alkyle]₂,
(C₁-C₆)alkylène-C(O)NH-(C₁-C₆)alkyle,
(C₁-C₆)alkylène-C(O)O-(C₁-C₆)alkyle,
C(O)O-(C₁-C₆)alkyle,
C(O)(C₁-C₆)alkyle,
C(O)R',
C(O)NH-(C₁-C₆)alkyle,
C(O)N[(C₁-C₆)alkyle]₂, ou
C(O)(C₁-C₆)alkylène-R',
ou R₆ et R₆', conjointement avec l'atome de N auquel ils sont liés, forment un groupement (C₅-C₁₀)hétérocyclyle.

37. Composé selon l'une des revendications 1 à 36,
**caractérisé en ce que** R₆ et R₆' sont indépendamment l'un de l'autre
H,
(C₁-C₆)alkyle,
(C₅-C₁₀)hétérocyclyle,
(C₃-C₈)cycloalkyle,
(C₆-C₁₀)aryle,
(C₁-C₄)alkylène-(C₃-C₈)cycloalkyle,
(C₁-C₄)alkylène-(C₅-C₁₀)hétérocyclyle,
(C₁-C₄)alkylène-(C₆-C₁₀)aryle,
(C₁-C₆)alkylène-O-(C₁-C₆)alkyle,
(C₁-C₆)alkylène-C(O)N[(C₁-C₆)alkyle]₂,
(C₁-C₆)alkylène-C(O)NH-(C₁-C₆)alkyle,
(C₁-C₆)alkylène-C(O)O-(C₁-C₆)alkyle,
C(O)O-(C₁-C₆)alkyle,
C(O)(C₁-C₆)alkyle,
C(O)(C₃-C₈)cycloalkyle
C(O)NH-(C₁-C₆)alkyle,
C(O)N[(C₁-C₆)alkyle]₂,
C(O)(C₁-C₆)alkylène-(C₃-C₈)cycloalkyle,
C(O)(C₁-C₆)alkylène-C₅-C₁₀)hétérocyclyle,
C(O)(C₁-C₆)alkylène-(C₆-C₁₀)aryle,
ou R₆ et R₆', conjointement avec l'atome de N auquel ils sont liés, forment un groupement (C₅-C₁₀)hétérocyclyle.

38. Composé selon l'une des revendications 1 à 37,
**caractérisé en ce que**
R₆ est H, (C₁-C₆)alkyle, (C₃-C₆)cycloalkyle ou (C₁-C₄)alkylène-(C₃-C₆)cycloalkyle, et R₆' est H,
(C₁-C₆)alkyle,
(C₃-C₈)cycloalkyle,
(C₅-C₁₀)hétérocyclyle,
(C₆-C₁₀)aryle,
(C₁-C₄)alkylène-(C₃-C₈)cycloalkyle,
(C₁-C₄)alkylène-(C₅-C₁₀)hétérocyclyle,
(C₁-C₄)alkylène-(C₆-C₁₀)aryle,
(C₁-C₆)alkylène-O-(C₁-C₆)alkyle,
(C₁-C₆)alkylène-C(O)NH-(C₁-C₆)alkyle,
(C₁-C₆)alkylène-C(O)N[(C₁-C₆)alkyle]₂,
(C₁-C₆)alkylène-C(O)O-(C₁-C₆)alkyle,
C(O)O-(C₁-C₆)alkyle,
C(O)(C₁-C₆)alkyle,
C(O)(C₃-C₈)cycloalkyle,
C(O)NH-(C₁-C₆)alkyle,
C(O)N[(C₁-C₆)alkyle]₂,
C(O)(C₁-C₆)alkylène-(C₃-C₈)cycloalkyle,
C(O)(C₁-C₆)alkylène-(C₅-C₁₀)hétérocyclyle, ou
C(O)(C₁-C₆)alkylène-(C₆-C₁₀)aryle, ou
R₆ et R₆', conjointement avec l'atome de N auquel ils sont liés, forment un groupement (C₅-C₁₀)hétérocyclyle.

39. Composé selon l'une quelconque des revendications 1 à 38, **caractérisé en ce que**
R₆ est H, (C₁-C₆)alkyle et
R₆' est H,
(C₁-C₆)alkyle,
(C₃-C₈)cycloalkyle,
(C₆-C₁₀)aryle,
(C₅-C₁₀)hétérocyclyle,
(C₁-C₄)alkylène-(C₃-C₈)cycloalkyle,
(C₁-C₄)alkylène-(C₅-C₁₀)hétérocyclyle,
(C₁-C₆)alkylène-(C₆-C₁₀)aryle,
(C₁-C₄)alkylène-O-(C₁-C₄)alkyle,
(C₁-C₄)alkylène-C(O)N[(C₁-C4)alkyle]₂,
(C₁-C₆)alkylène-C(O)NH-(C₁-C₆)alkyle,
C(O)(C₁-C₆)alkyle,
C(O)(C₁-C₆)alkylène-(C₅-C₁₀)hétérocyclyle, ou
R₆ et R₆', conjointement avec l'atome de N auquel ils sont liés, forment un groupement (C₅-C₁₀)hétérocyclyle.

40. Composé selon l'une quelconque des revendications 1 à 39, **caractérisé en ce que**
R₆ est H, (C₁-C₆)alkyle et
R₆' est H,
(C₁-C₆)alkyle ;
(C₃-C₈)cycloalkyle ;
(C₁-C₄)alkylène-(C₃-C₈)cycloalkyle ;
(C₁-C₄)alkylène-O-(C₁-C₄)alkyle ;
(C₁-C₄)alkylène-C(O)N[(C₁-C₄)alkyle]₂
(C₁-C₄)alkylène-(C₅-C₁₀)hétérocyclyle, or
(C₁-C₄)alkylène-(C₆-C₁₀)aryle ;
C(O)(C₁-C₄)alkyle ;
C(O)(C₁-C₄)alkylène-(C₅-C₁₀)hétérocyclyle ;
ou R₆ et R₆', conjointement avec l'atome de N auquel ils sont liés, forment un groupement (C₅-C₆)hétérocyclyle.

41. Composé selon l'une quelconque des revendications 1 à 40, **caractérisé en ce que** R₆ est H, (C₁-C₆)alkyle et R₆' est H, (C₁-C₆)alkyle ou (C₃-C₈)cycloalkyle.

42. Composé selon l'une quelconque des revendications 1 à 41, **caractérisé en ce que** R₆ est H et R₆' est H, (C₁-C₆)alkyle ou (C₃-C₈)cycloalkyle.

43. Composé selon l'une quelconque des revendications 1 à 42, **caractérisé en ce que** R₆ et R₆' sont H.

44. Composé selon l'une des revendications 1 à 43, **caractérisé en ce que** m est 3 et L est lié en position 3 ou en position 4 du cycle aminocyclohexane.

45. Composé selon l'une des revendications 1 à 44, **caractérisé en ce que** m est 3 et L est lié en position 4 du cycle aminocyclohexane.

46. Composé selon l'une des revendications 1 à 45, **caractérisé en ce que** p est 0.

47. Utilisation d'au moins un composé de formule (I) et/ou de son sel pharmaceutiquement acceptable selon l'une des revendications 1 à 46, pour la production d'un médicament.

48. Utilisation d'au moins un composé de formule (I) et/ou de son sel pharmaceutiquement acceptable selon l'une des revendications 1 à 46, pour la produition d'un médicament destiné au traitement et/ou à la prévention de l'hypertension, de l'hypertension pulmonaire, de l'hypertension oculaire, de la rétinopathie, du glaucome, du trouble circulatoire périphérique, de la maladie artérielle occlusive périphérique (MAOP), de la maladie cardiaque coronarienne, de l'angine de poitrine, de l'hypertrophie cardiaque, de l'insuffisance cardiaque, des maladies ischémiques, de l'insuffisance ischémique des organes (l'atteinte des plaques terminales), de la fibrose pulmonaire, de la fibrose hépatique, de l'insuffisance hépatique, de la néphropathie, de l'insuffisance rénale, de la fibrose rénale, de la glomérulosclérose rénale, de l'hypertrophie des organes, de l'asthme, de la bronchopneumopathie chronique obstructive (BPCO), du syndrome de détresse respiratoire chez l'adulte, des troubles thrombotiques, de l'accident vasculaire cérébral, du vasospasme cérébral, de l'ischémie cérébrale, de la douleur, de la dégénérescence neuronale, de la lésion médullaire, de la maladie d'Alzheimer, de l'accouchement prématuré, du dysfonctionnement érectile, des dysfonctionnements endocriniens, de l'artériosclérose, de l'hypertrophie prostatique, du diabète et des complications du diabète, du syndrome métabolique, de la resténose des vaisseaux sanguins, de l'athérosclérose, des inflammations, des maladies auto-immunes, du SIDA, de l'ostéopathie, de l'infection des voies digestives par des bactéries, de l'état septique, du développement et de l'évolution du cancer.

49. Médicament comprenant une quantité efficace d'au moins un composé selon l'une quelconque des revendications 1 à 46 et/ou un sel pharmacologiquement acceptable de celui-ci, des excipients et des supports pharmaceutiquement tolérés et, le cas échéant, d'autres additifs et/ou d'autres principes actifs.
